(19) Europäisches Patentamt
European Patent Office
Office européen des brevets

(11) Veröffentlichungsnummer: **0 247 431**
**B1**

(12) # EUROPÄISCHE PATENTSCHRIFT

(45) Veröffentlichungstag der Patentschrift: **01.08.90**

(21) Anmeldenummer: **87106855.7**

(22) Anmeldetag: **12.05.87**

(51) Int. Cl.⁵: **C07D 487/04,** A61K 31/40
// C07D205/08, C07D303/48,
C07F7/18 ,(C07D487/04, 209:00,
205:00)

(54) **Substituierte 6-Hydroxymethylcarbapenem-Antibiotika, Verfahren zu ihrer Herstellung und ihre Verwendung.**

(30) Priorität: **26.05.86 DE 3617626**

(43) Veröffentlichungstag der Anmeldung:
**02.12.87 Patentblatt 87/49**

(45) Bekanntmachung des Hinweises auf die Patenterteilung:
**01.08.90 Patentblatt 90/31**

(84) Benannte Vertragsstaaten:
**AT BE CH DE ES FR GB GR IT LI NL SE**

(56) Entgegenhaltungen:
**EP-A- 0 017 992**
**EP-A- 0 181 702**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk(DE)**

(72) Erfinder: **Häbich, Dieter, Dr., Krummacherstrasse 82, D-5600 Wuppertal 1(DE)**
Erfinder: **Hartwig, Wolfgang, Dr., Regents Parc 5020 South Lake Shore Drive App. 1604, Chicago Illinois 60615(US)**
Erfinder: **Metzger, Karl Georg, Dr., Pahlkestrasse 75, D-5600 Wuppertal 1(DE)**
Erfinder: **Zeller, Hans-Joachim, Dr., Elsbeeker Strasse 46, D-5620 Velbert 15(DE)**

EP 0 247 431 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft substituierte 6-Hydroxymethyl-carbapenem-antibiotika, Verfahren zu ihrer Herstellung und ihre Verwendung als Arzneimittel.

Es ist bekannt, daß sich Carbapeneme vom Thienamycintyp (vgl. T. Kametani, Heterocycles, 17, 463 (1982) durch gute antibakterielle Wirkung auszeichnen, die auch Methicillin- und Oxacillin-resistente Staphylokokken einschließt. Seit der Entdeckung von Thienamycin ist eine Vielzahl von Derivaten synthetisiert worden, jedoch sind die meisten dieser Verbindungen als Monosubstanz instabil gegen den Abbau durch die Peptidasen der Niere, speziell Dehydropeptidase (I) (DHP-I) [vgl. H. Kropp et al., Antimicrob. Agents., Chemother. 22, 62 (1982)]. Dieses Enzym ist verantwortlich für die metabolische Inaktivierung von Carbapenemen und für die Bildung von toxischen Metaboliten.

Außerdem ist bekannt, daß durch Zusatz eines Dipeptidaseinhibitors [vgl. Drugs of the Future 9, 227 (1984)], durch Einbau einer 1-β-Methylgruppe [vgl. D.H. Shih et al., Heterocycles 21, 29 (1984); Drugs of the Future 9, 336 (1984)] sowie durch Variationen der 2-Seitenketten (vgl. B.G. Christensen et al., in A.G. Brown and S.M. Roberts (eds), Recent Advances in the Chemistry of β-Lactam-Antibiotics, Royal Society of Chemistry, Spezial Publication No. 52, page 86 (1985)] die Instabilität dieser Verbindungsklasse gegen Dehydropeptidase (I) zum Teil unterdrückt werden kann.

In der EP-A 0 017 992 werden Verbindungen beschrieben, die den erfindungsgemäßen 6-Hydroxymethylcarbapenem-Antibiotika strukturell ähnlich sind.

Die EP-A 0 181 702 betrifft Penicillin-1,1-dioxid-Verbindungen.

Die vorliegende Erfindung betrifft substituierte 6-Hydroxmethyl-carbapenem-antibiotika der allgemeinen Formel (I)

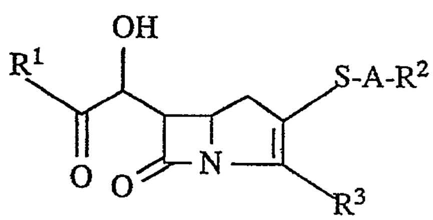

(I)

in welcher

$R^1$ für eine Gruppe der Formel $-OCH_3$, $-OC_2H_5$, $-OC_3H_7$, $-OCH(CH_3)_2$, $-OC_4H_9$, $-OC(CH_3)_3$, $-O-CH_2-CH=CH_2$, $-O-CH_2-C_6H_5$,

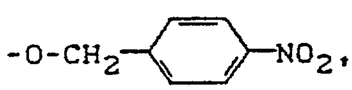 , 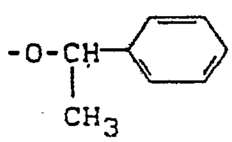 (R-Form, S-Form oder RS-Gemisch)

$-NH_2$, $-NHCH_3$, $-N(CH_3)_2$, $-NHC_2H_5$, $-N(C_2H_5)_2$,

$-NHC_3H_7$, $-N(C_3H_7)_2$, $-NHCH(CH_3)_2$, 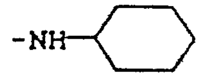,

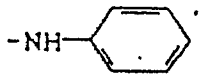, $-NH-CH_2-CH=CH_2$, 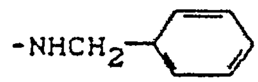,

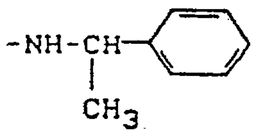 (R-Form, S-Form oder PS-Gemisch)

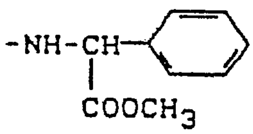 (R-Form, S-Form oder RS-Gemisch)

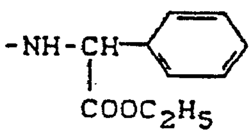 (R-Form, S-Form oder RS-Gemisch)

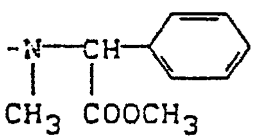 (R-Form, S-Form oder RS-Gemisch)

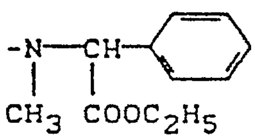 (R-Form, S-Form oder RS-Gemisch)

$-NH-CO-NH_2$, $-NH-CO-NHCH_3$, $-NH-CO-N(CH_3)_2$,

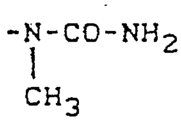, 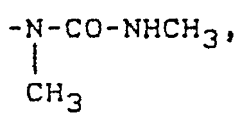, 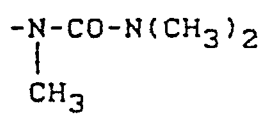,

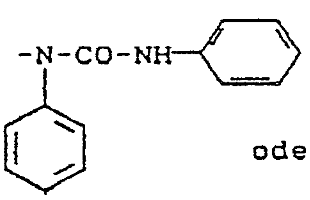 oder 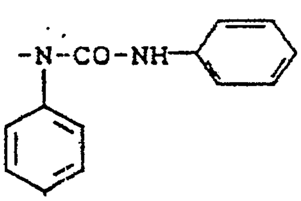 steht,

– die Gruppe AR² für einen Rest der Formel

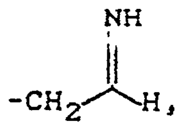 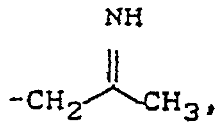 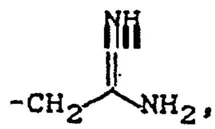 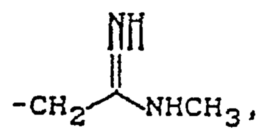

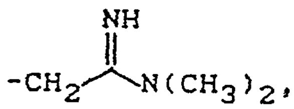 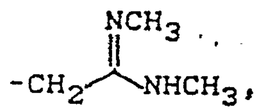 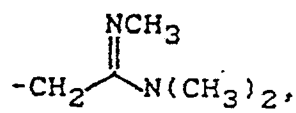

$-CH_2-CH_2-NH_2$, $-CH_2-CH_2-NHCH_3$, $-CH_2-CH_2-N(CH_3)_2$,

$-CH_2-CH_2-NHCOCH_3$, 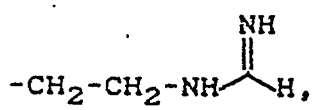

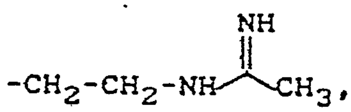 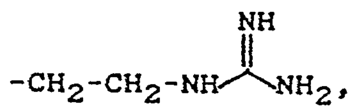 $-CH_2-COOH$,

$-CH_2-COOCH_3$, 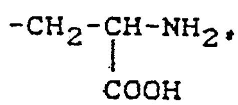 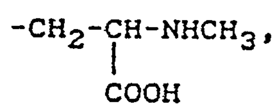

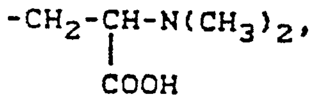, 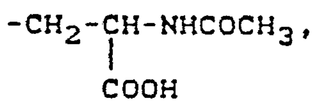, 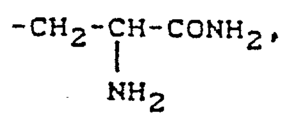,

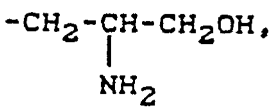, 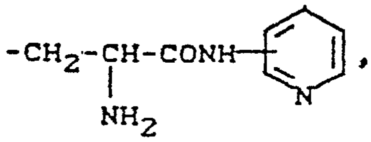, $-CH=CH-NH_2$,

$-CH-CH-NHCH_3$, $-CH=CH-N(CH_3)_2$, $-CH=CH-NHCOCH_3$,

$-CH=CH-CH_2-NH_2$, 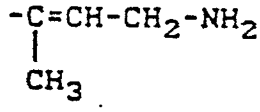

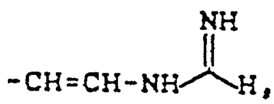, 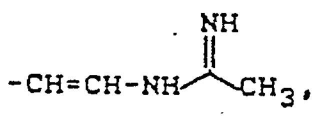, 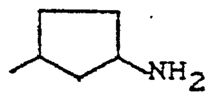,

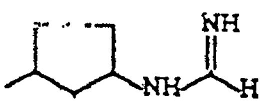, 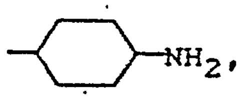, 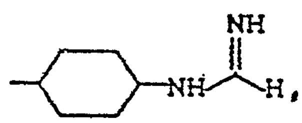,

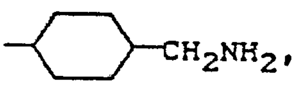, 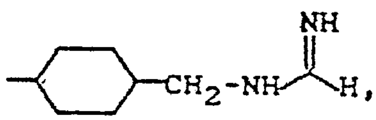, 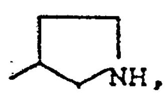,

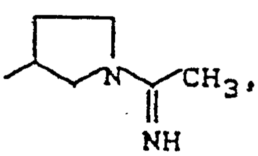, 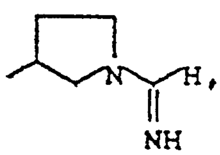, 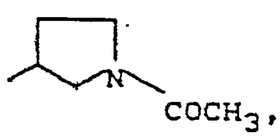, 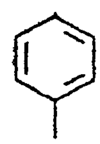,

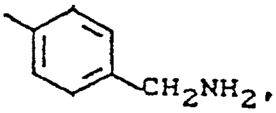, 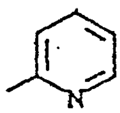, 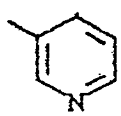, 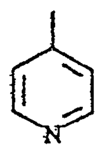, 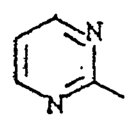

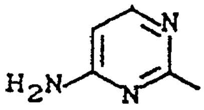, 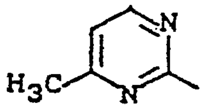, 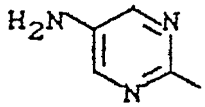, 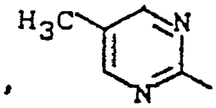,

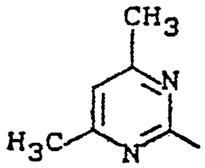, 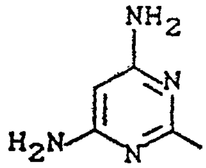, 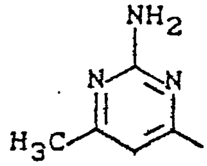,

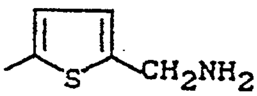, 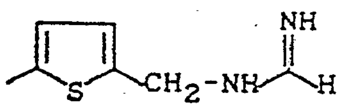, 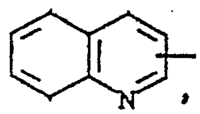,

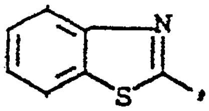, 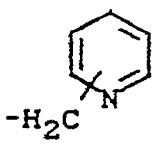, 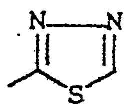, 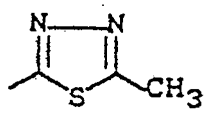,

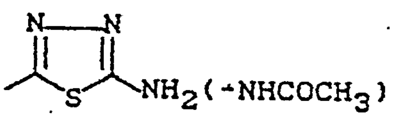, 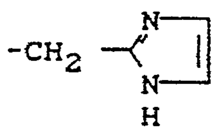, 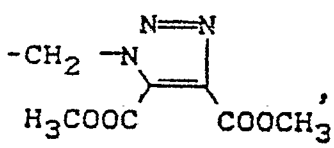,

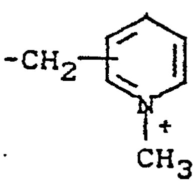, 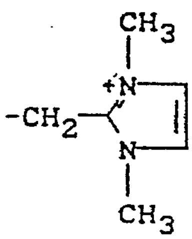, 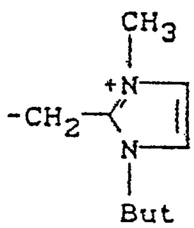,

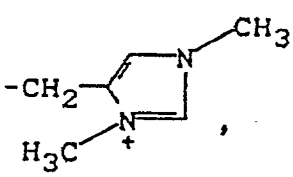, 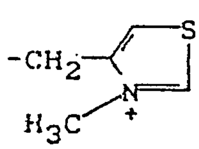, 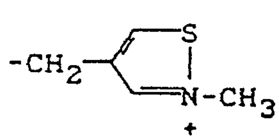

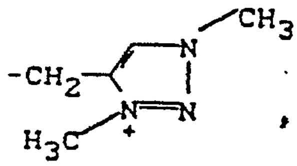 ,
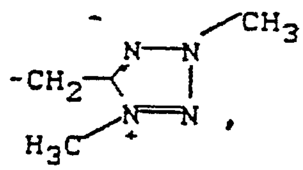 ,

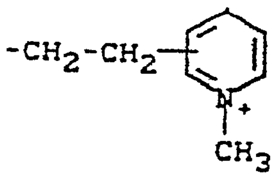 ,
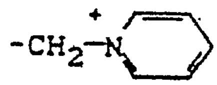 ,
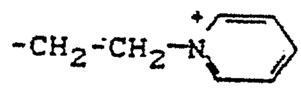 ,

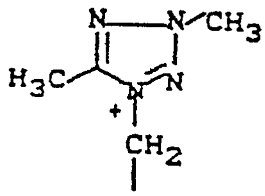 ,
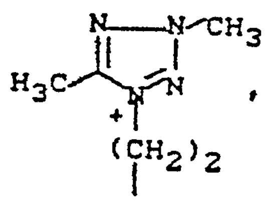 ,
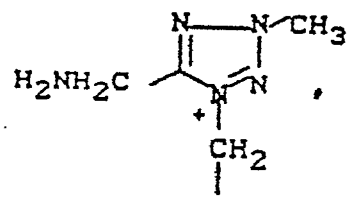 ,

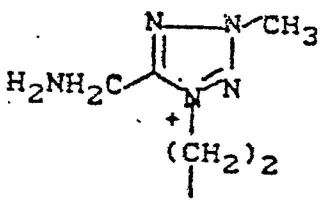 ,
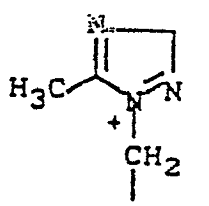 ,
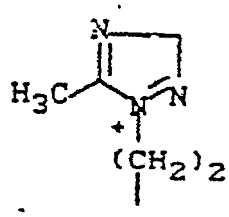

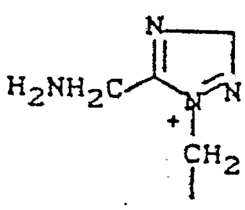 ,
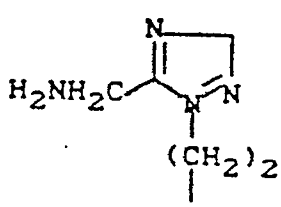 ,
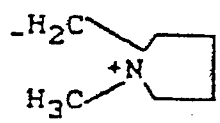 ,

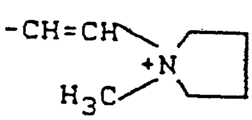 ,
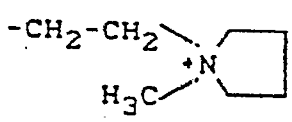 ,
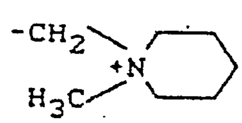 ,

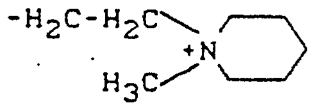 , 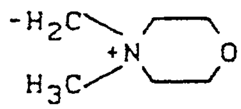 , 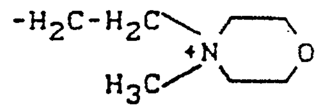 ,

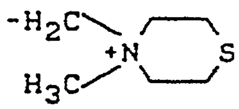 , 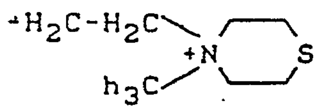 , 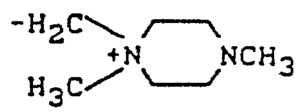 ,

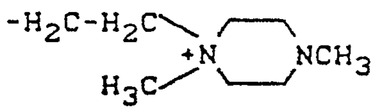 , 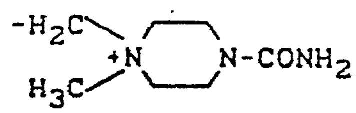 ,

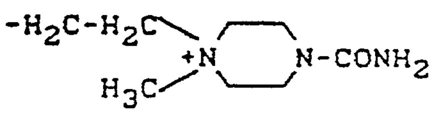 , 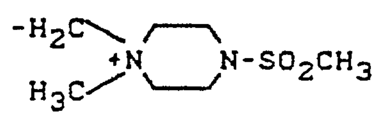 ,

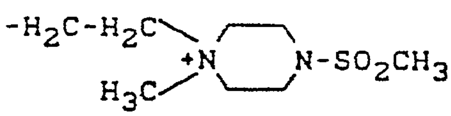 , 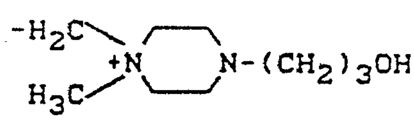 ,

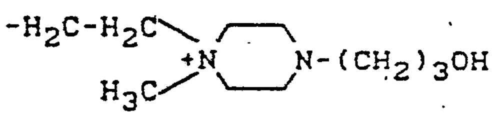 steht,

und

$R^3$ – für eine Carboxygruppe $COOR^{20}$ steht,

worin

$R^{20}$ Wasserstoff, Allyl, 4-Nitrobenzyl oder 4-Methoxybenzyl bedeutet oder

für $COO^-$ steht, falls die Gruppe $AR^2$ einen positif geladenen Rest darstellt, und deren Salze.

Neben den in den Beispielen aufgeführten Produkten sind folgende Verbindungen der allgemeinen Formel (I) und deren Salze insbesondere bevorzugt:

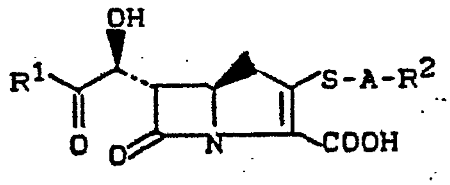

| $R^1$ | A-$R^2$ |
|---|---|
| $CH_3O$ | 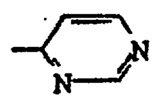 |
| $CH_3O$ | 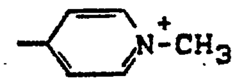 |
| $CH_3O$ | 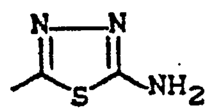 |
| $CH_3O$ | 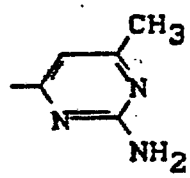 |
| $CH_3O$ | 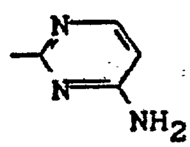 |
| $CH_3O$ | 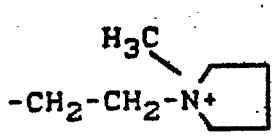 |

| $R^1$ | $A R^2$ |
|---|---|
| $CH_3O$ | 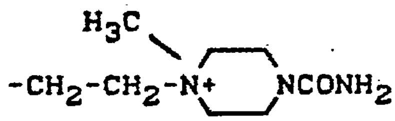 |
| $CH_3O$ | 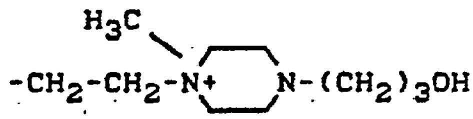 |
| $CH_3O$ | 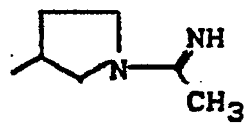 |
| $CH_3O$ | 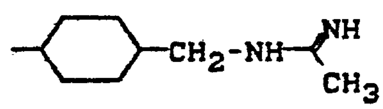 |
| $CH_3O$ | 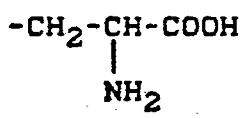 |
| $CH_3O$ | 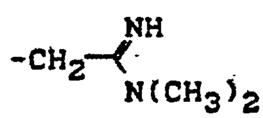 |
| $CH_3O$ | 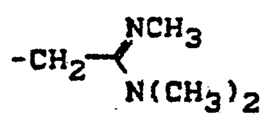 |
| $CH_3O$ | $-CH_2-CH_2-NH_2$ |
| $CH_3O$ | $-CH_2-CH_2-NHCOCH_3$ |
| $CH_3O$ | 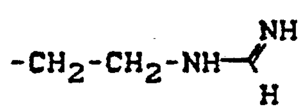 |

| $R^1$ | $-A-R_2$ |
|---|---|
| $CH_3O$ | 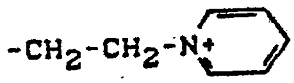 |
| $CH_3O$ | 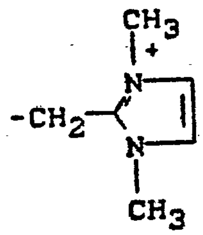 |
| $CH_3O$ | 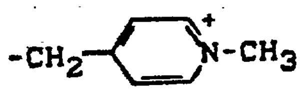 |
| $CH_3O$ | 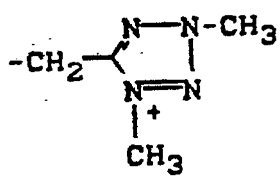 |
| $CH_3O$ | 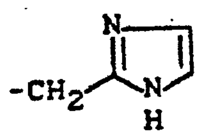 |
| $CH_3O$ | 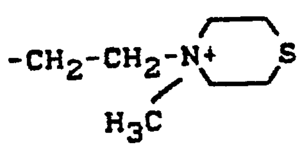 |
| $CH_3O$ | 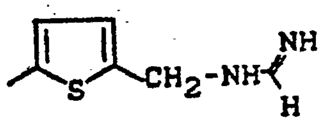 |
| $CH_3O$ | 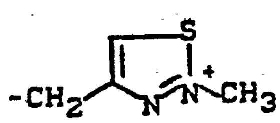 |

| $R^1$ | A $R^2$ |
|---|---|
| OH | 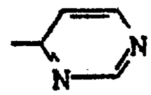 |
| OH | 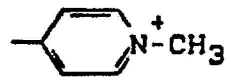 |
| OH | 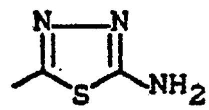 |
| OH | 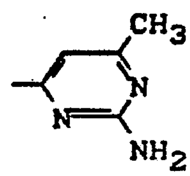 |
| OH | 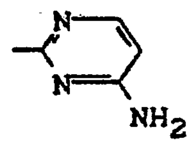 |
| OH | 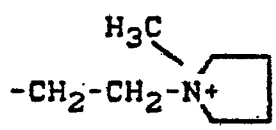 |
| OH | 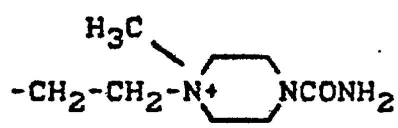 |
| OH | 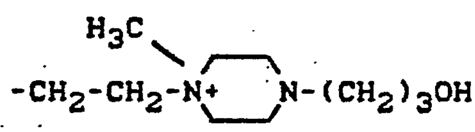 |
| OH | 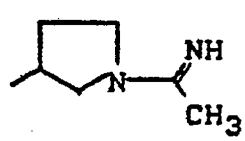 |
| OH | 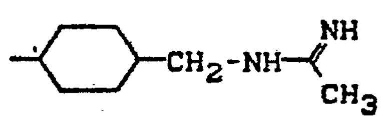 |

| $R^1$ | $-A-R^2$ |
|---|---|
| OH | 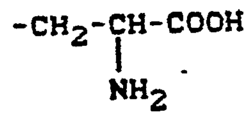 |
| OH | 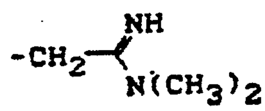 |
| OH | 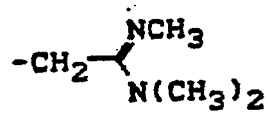 |
| OH | $-CH_2-CH_2-NH_2$ |
| OH | $-CH_2-CH_2-NHCOCH_3$ |
| OH | 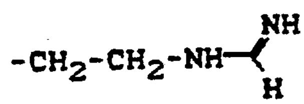 |
| OH | 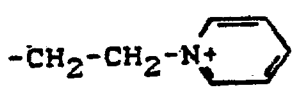 |
| OH | 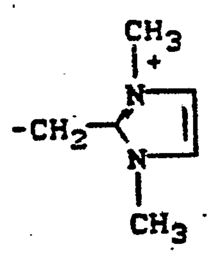 |
| OH | 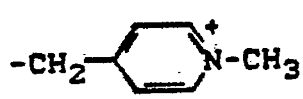 |
| OH | 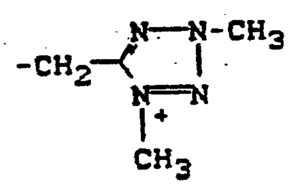 |

| $R^1$ | $-A-R_2$ |
|---|---|
| OH | 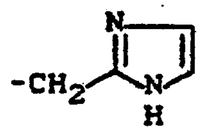 |
| OH | 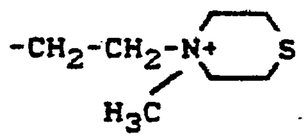 |
| OH | 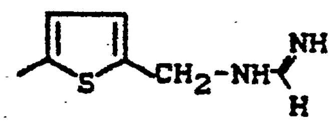 |
| OH | 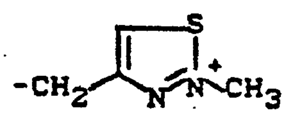 |
| $OC_2H_5$ | 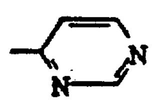 |
| $OC_2H_5$ | 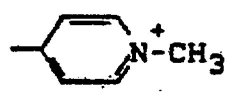 |
| $OC_2H_5$ | 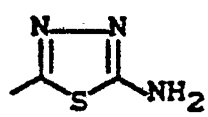 |
| $OC_2H_5$ | 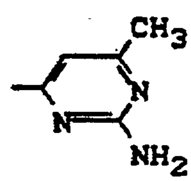 |
| $OC_2H_5$ | 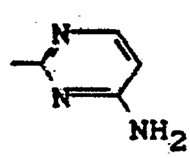 |

| $R^1$ | A-$R^2$ |
|---|---|
| $OC_2H_5$ | 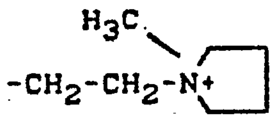 |
| $OC_2H_5$ | 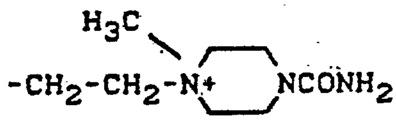 |
| $OC_2H_5$ | 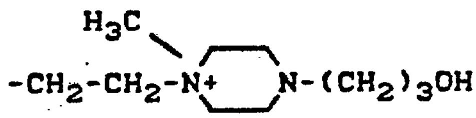 |
| $OC_2H_5$ | 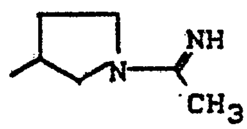 |
| $OC_2H_5$ | 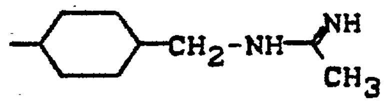 |
| $OC_2H_5$ | 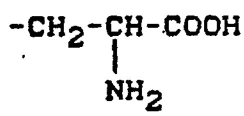 |
| $OC_2H_5$ | 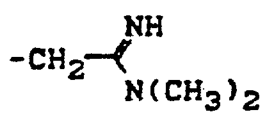 |
| $OC_2H_5$ | 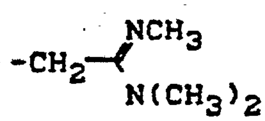 |
| $OC_2H_5$ | $-CH_2-CH_2-NH_2$ |
| $OC_2H_5$ | $-CH_2-CH_2-NHCOCH_3$ |

| $R^1$ | $-A-R^2$ |
|---|---|
| $OC_2H_5$ | 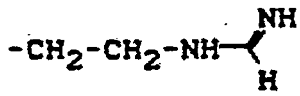 |
| $OC_2H_5$ | 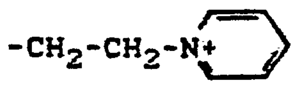 |
| $OC_2H_5$ | 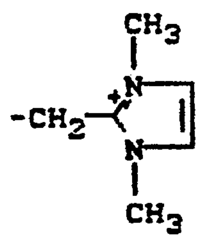 |
| $OC_2H_5$ | 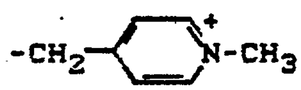 |
| $OC_2H_5$ | 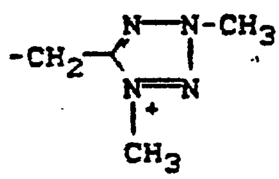 |
| $OC_2H_5$ | 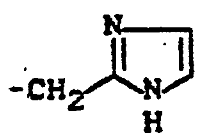 |
| $OC_2H_5$ | 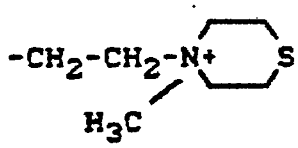 |
| $OC_2H_5$ | 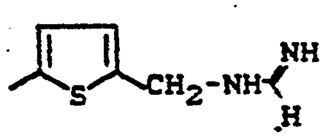 |
| $OC_2H_5$ | 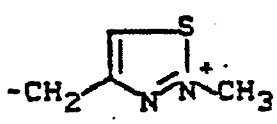 |

| $R^1$ | $A-R^2$ |
|---|---|
| $OCH_2-CH=CH_2$ | 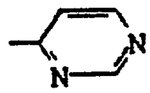 |
| $OCH_2-CH=CH_2$ | 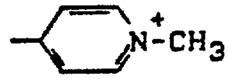 |
| $OCH_2-CH=CH_2$ | 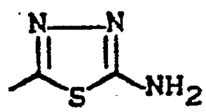 |
| $OCH_2-CH=CH_2$ | 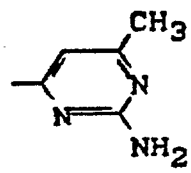 |
| $OCH_2-CH=CH_2$ | 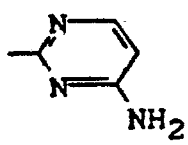 |
| $OCH_2-CH=CH_2$ | 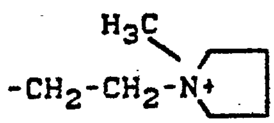 |
| $OCH_2-CH=CH_2$ | 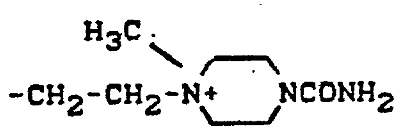 |
| $OCH_2-CH=CH_2$ | 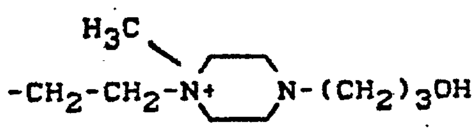 |
| $OCH_2-CH=CH_2$ | 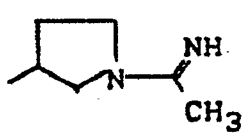 |
| $OCH_2-CH=CH_2$ | 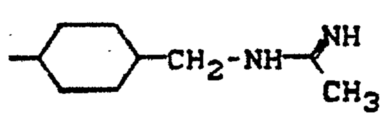 |

| $R^1$ | $-AR^2$ |
|---|---|
| $OCH_2-CH=CH_2$ | 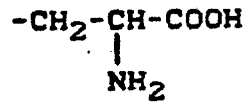 |
| $OCH_2-CH=CH_2$ | 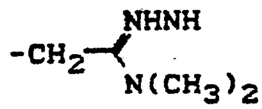 |
| $OCH_2-CH=CH_2$ | 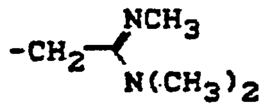 |
| $OCH_2-CH=CH_2$ | $-CH_2-CH_2-NH_2$ |
| $OCH_2-CH=CH_2$ | $-CH_2-CH_2-NHCOCH_3$ |
| $OCH_2-CH=CH_2$ | 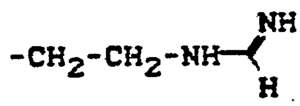 |
| $OCH_2-CH=CH_2$ | 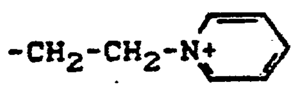 |
| $OCH_2-CH=CH_2$ | 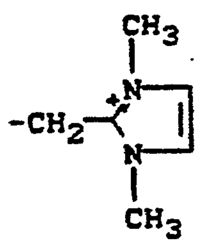 |
| $OCH_2-CH=CH_2$ | 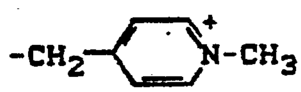 |
| $OCH_2-CH=CH_2$ | 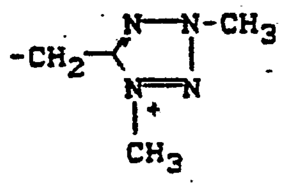 |

| $R^1$ | -A-$R_2$ |
|---|---|
| $OCH_2-CH=CH_2$ | 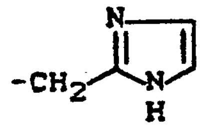 |
| $OCH_2-CH=CH_2$ | 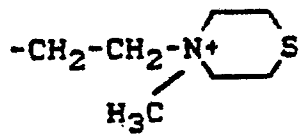 |
| $OCH_2-CH=CH_2$ | 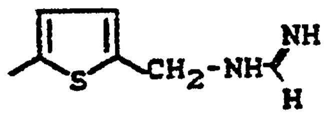 |
| $OCH_2-CH=CH_2$ | 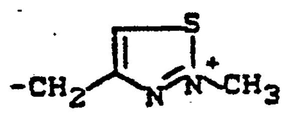 |
| $NH_2$ | 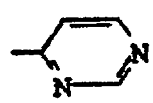 |
| $NH_2$ | 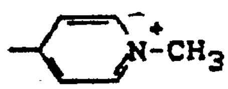 |
| $NH_2$ | 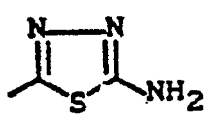 |
| $NH_2$ | 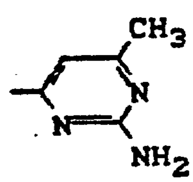 |
| $NH_2$ | 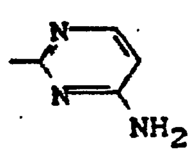 |

| $R^1$ | A-$R^2$ |
| --- | --- |
| $NH_2$ | 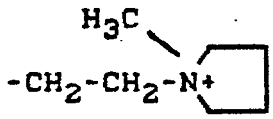 |
| $NH_2$ | 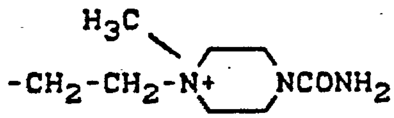 |
| $NH_2$ | 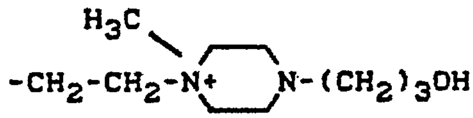 |
| $NH_2$ | 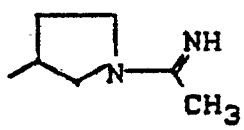 |
| $NH_2$ | 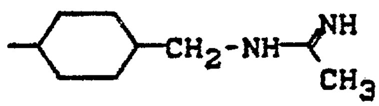 |
| $NH_2$ | 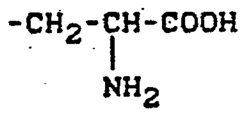 |
| $NH_2$ | 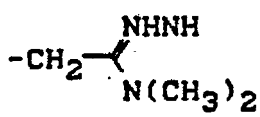 |
| $NH_2$ | 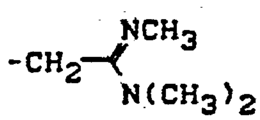 |
| $NH_2$ | $-CH_2-CH_2-NH_2$ |
| $NH_2$ | $-CH_2-CH_2-NHCOCH_3$ |

| $R^1$ | -AXR$^2$ |
|---|---|
| $NH_2$ | 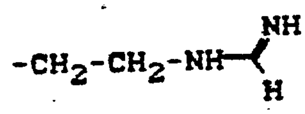 |
| $NH_2$ | 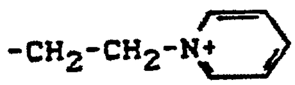 |
| $NH_2$ | 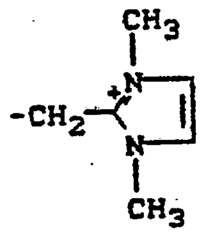 |
| $NH_2$ | 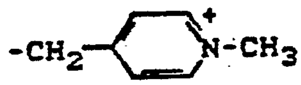 |
| $NH_2$ | 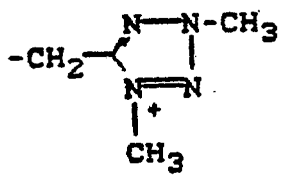 |
| $NH_2$ | 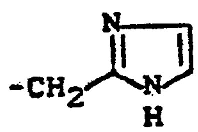 |
| $NH_2$ | 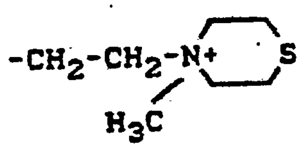 |
| $NH_2$ | 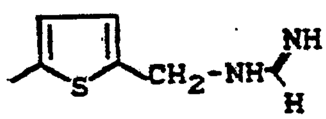 |
| $NH_2$ | 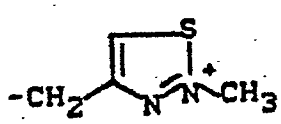 |

| $R^1$ | A–$R^2$ |
|---|---|
| $N(CH_3)_2$ | 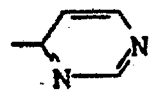 |
| $N(CH_3)_2$ | 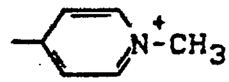 |
| $N(CH_3)_2$ | 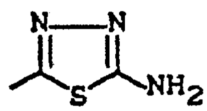 |
| $N(CH_3)_2$ | 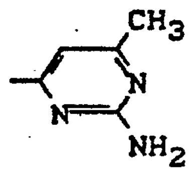 |
| $N(CH_3)_2$ | 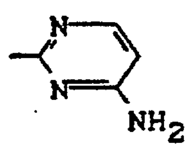 |
| $N(CH_3)_2$ | 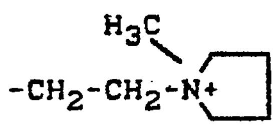 |
| $N(CH_3)_2$ | 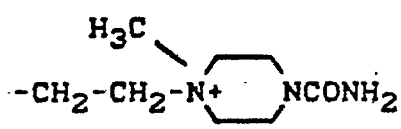 |
| $N(CH_3)_2$ | 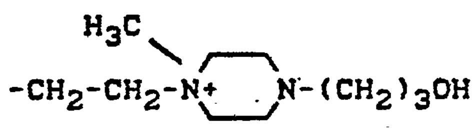 |
| $N(CH_3)_2$ | 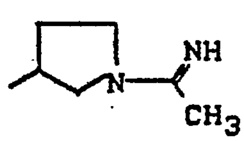 |
| $N(CH_3)_2$ | 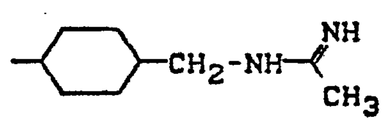 |

| $R^1$ | $-ASR^2$ |
|---|---|
| $N(CH_3)_2$ | 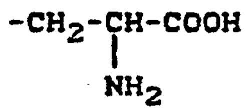 |
| $N(CH_3)_2$ | 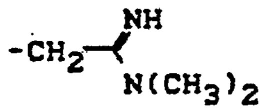 |
| $N(CH_3)_2$ | 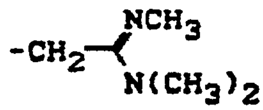 |
| $N(CH_3)_2$ | $-CH_2-CH_2-NH_2$ |
| $N(CH_3)_2$ | $-CH_2-CH_2-NHCOCH_3$ |
| $N(CH_3)_2$ | 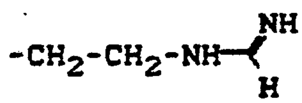 |
| $N(CH_3)_2$ | 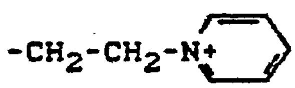 |
| $N(CH_3)_2$ | 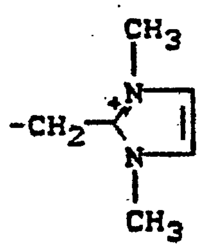 |
| $N(CH_3)_2$ | 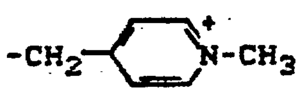 |
| $N(CH_3)_2$ | 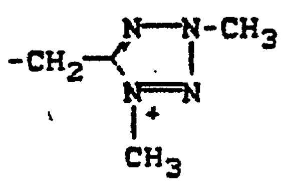 |

| $R^1$ | $-A\$R^2$ |
|---|---|
| $N(CH_3)_2$ | 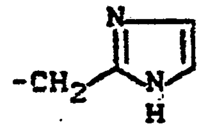 |
| $N(CH_3)_2$ | 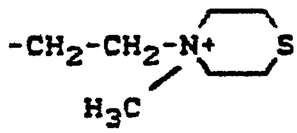 |
| $N(CH_3)_2$ | 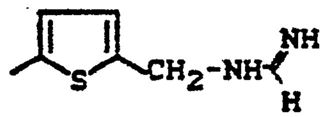 |
| $N(CH_3)_2$ | 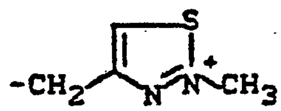 |
| 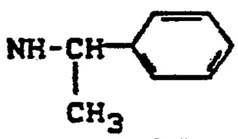 | 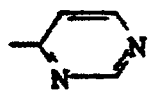 |
| 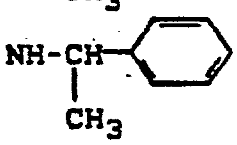 | 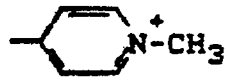 |
| 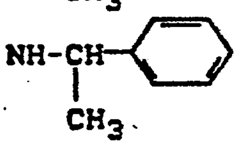 | 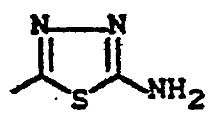 |
| 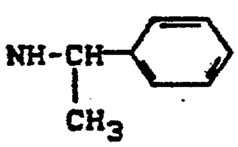 | 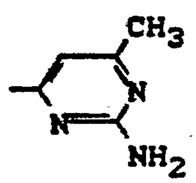 |
| 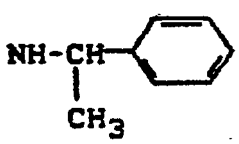 | 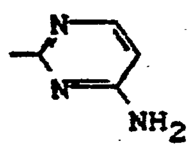 |

| $R^1$ | $A-R^2$ |
|---|---|
| 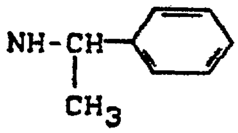 | 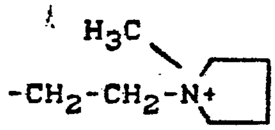 |
| 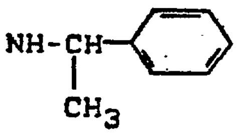 | 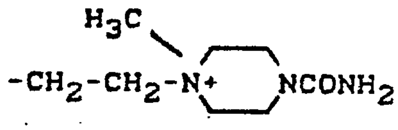 |
| 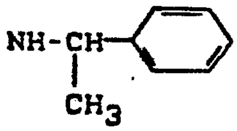 | 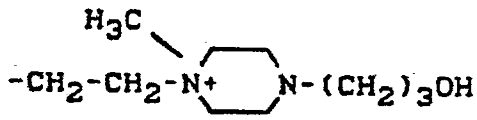 |
| 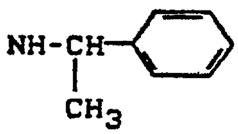 | 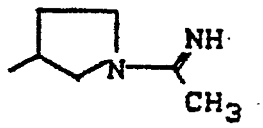 |
| 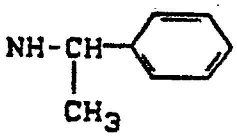 | 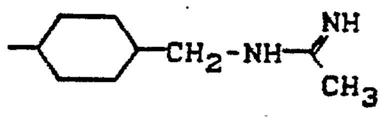 |
| 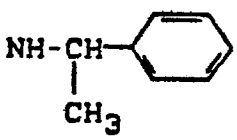 | 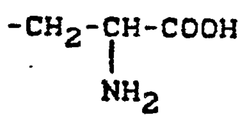 |
| 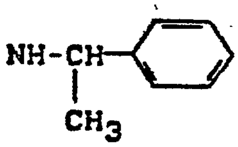 | 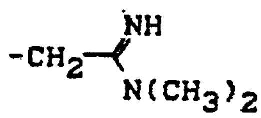 |
| 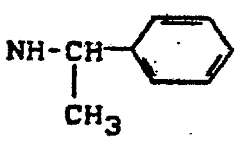 | 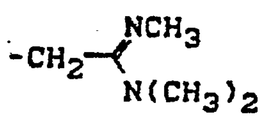 |
| 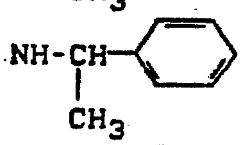 | $-CH_2-CH_2-NH_2$ |
| 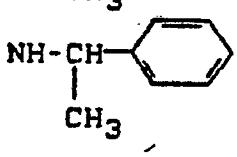 | $-CH_2-CH_2-NHCOCH_3$ |

| $R^1$ | $R^2$ |
| --- | --- |
| 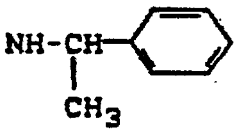 | 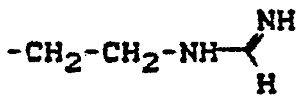 |
| 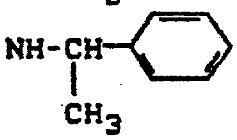 | 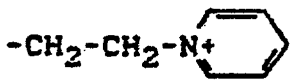 |
| 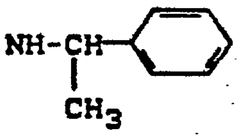 | 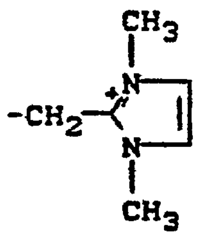 |
| 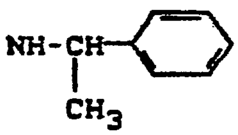 | 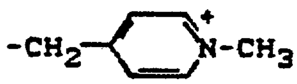 |
| 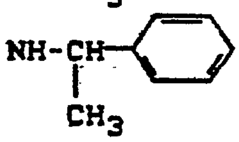 | 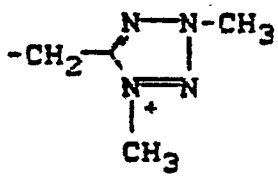 |
| 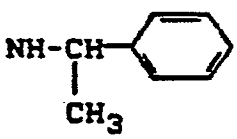 | 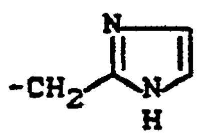 |
| 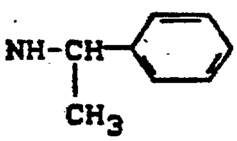 | 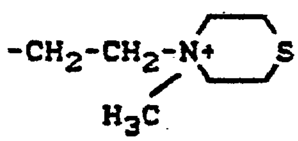 |
| 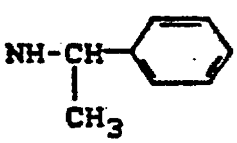 | 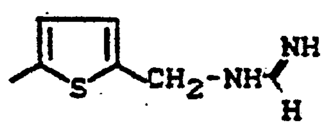 |
| 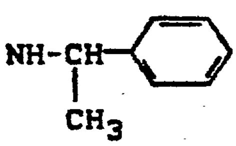 | 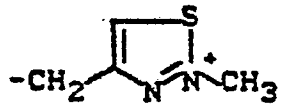 |

| $R^1$ | -ASR$^2$ |
| --- | --- |
| 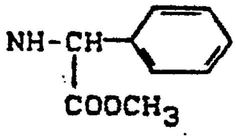 | 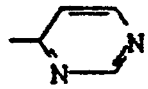 |
| 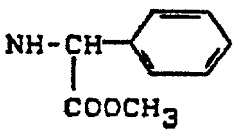 | 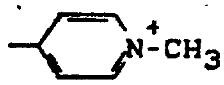 |
| 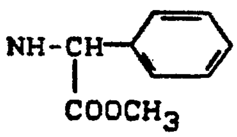 | 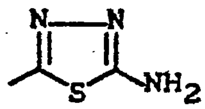 |
| 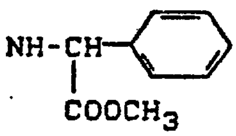 | 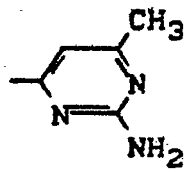 |
| 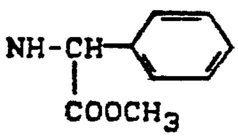 | 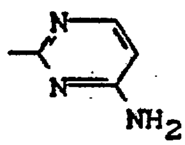 |
| 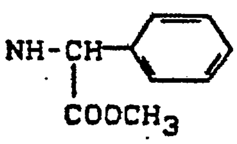 | 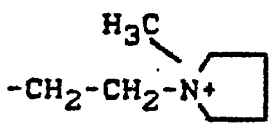 |
| 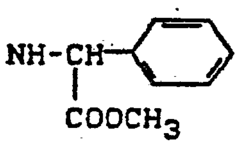 | 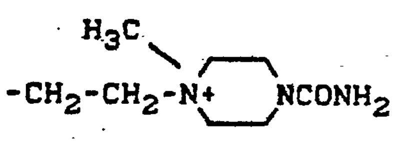 |

| $R^1$ | $-A-R^2$ |
| --- | --- |
| 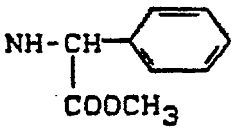 | 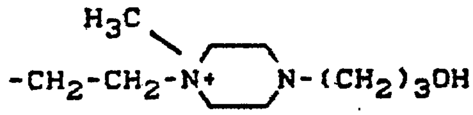 |
| 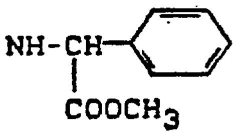 | 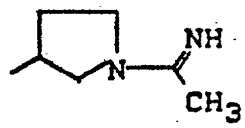 |
| 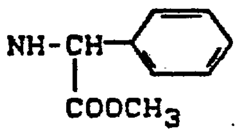 | 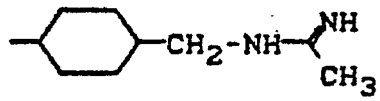 |
| 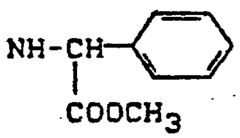 | 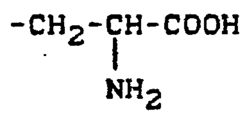 |
| 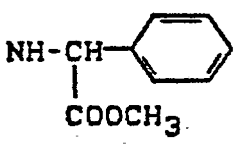 | 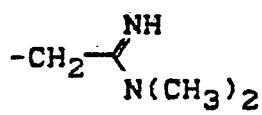 |
| 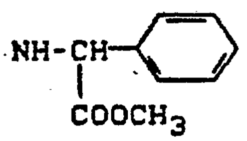 | 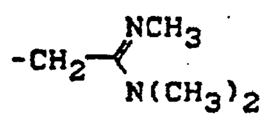 |
| 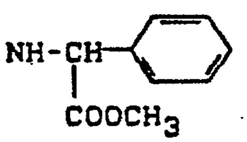 | $-CH_2-CH_2-NH_2$ |
| 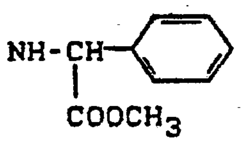 | $-CH_2-CH_2-NHCOCH_3$ |

| $R^1$ | -ASR$^2$ |
|---|---|
| 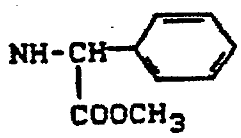 | 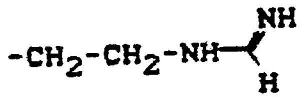 |
| 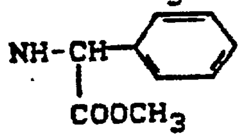 | 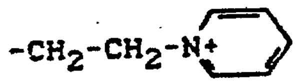 |
| 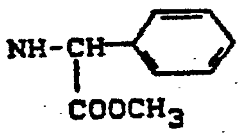 | 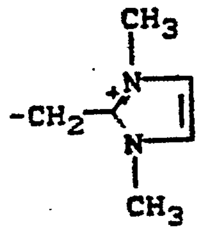 |
| 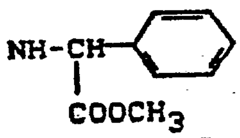 | 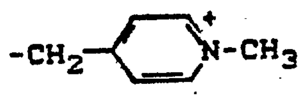 |
| 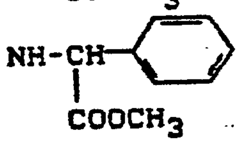 | 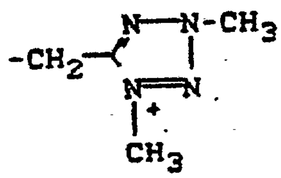 |
| 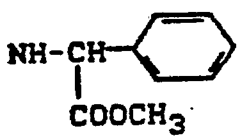 | 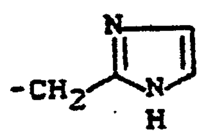 |
| 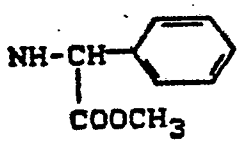 | 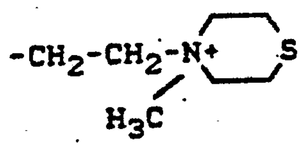 |
| 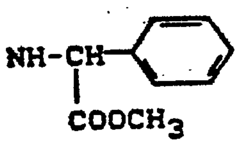 | 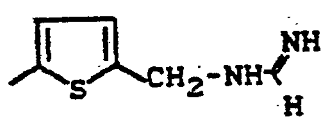 |
| 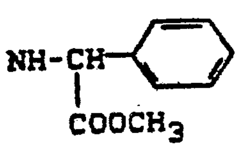 | 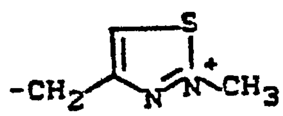 |

Die erfindungsgemäßen Verbindungen der allgemeinen Formel (I) können als freie Säuren, als Ester, als innere Salze oder als nicht toxische, physiologisch verträgliche Salze vorliegen, entweder mit einem externen Gegenanion (wenn $R^2$ einen positiv geladenen Rest darstellt), oder mit einem Gegenkation (wenn $R^3$ für $COO^-$ steht). Als Gegenkationen sind bevorzugt Alkali- oder Erdalkalikationen wie zum Beispiel Natrium-, Kalium-, Magnesium- oder Calciumionen zu nennen, oder Aluminium- oder Ammoniumionen sowie nicht toxische substituierte Ammoniumionen aus Aminen wie Di-, Triniedrigalkylaminen, Procain, Dibenzylamin, N,N'-Dibenzylethylendiamin, N-Benzyl-β-phenyl-ethylamin, N-Methyl- und N-Ethylmorpholin, 1-Ephenamin, Dihydroabiethylamin, N,N'-Bis-dihydroabiethylethylendiamin, N-Niedrigalkylpiperidine und anderen Aminen, die zur Bildung von Salzen von β-Lactam-Verbindungen verwendet werden können. Als Gegenanionen eignen sich insbesondere Chlorid, Bromid, Iodid, Hydroxid, Hydrogencarbonat, Carbonat, Hydrogensulfat, Sulfat oder Anionen von üblichen organischen Säuren wie beispielsweise Acetat, Maleat, Fumarat, Benzoat, Lactat oder Sulfonate.

Die erfindungsgemäßen Verbindungen besitzen mehrere asymmetrische Kohlenstoffatome und können somit in mehreren stereochemischen Formen existieren. Die Erfindung umfaßt die Isomerengemische ebenso wie die einzelnen Stereoisomeren. Bevorzugte Verbindungen der Formel (I) sind solche mit 5R, 6S, 8S-Konfiguration:

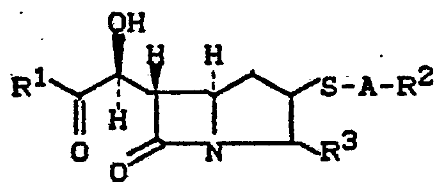

Wenn die Seitenketten, insbesondere $R^1$, weitere Asymmetriezentren enthalten, führt dies zu diastereomeren Produkten, die ebenfalls von der Erfindung umfaßt werden. Die stereochemisch einheitlichen Produkte können entweder aus den Gemischen durch übliche Trennmethoden (z.B. Kristallisation oder Chromatographie), oder durch stereospezifische Synthese ausgehend von optisch reinem Material hergestellt werden.

Die erfindungsgemäßen Verbindungen der allgemeinen Formel (I) und deren Salze erhält man, indem man Ketoester der allgemeinen Formel (II)

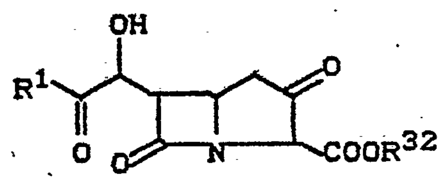

worin
$R^1$ - die oben angegebene Bedeutung hat
und
$R^{32}$- die Bedeutung von $R^{20}$ hat, aber nicht für Wasser stoff stehen darf,
und wobei die Gruppe $COOR^{32}$ nicht für ein Carboxylatanion stehen darf,
und
Thiole der allgemeinen Formel (III)
$H\text{-}S\text{-}A\text{-}R^2$ (III)
in welchen
A und $R^2$ die angegebene Bedeutung haben in inerten Lösungsmitteln, in Gegenwart von Basen mit einem Hilfsstoff umsetzt, gegebenenfalls Schutzgruppen abspaltet und gegebenenfalls die gewünschten Salze herstellt oder die Salze in die freien Verbindungen überführt.

Verwendet man als Ausgangsstoffe (2R, 5R, 6S)-3,7-Dioxo-6-[(1S)-1-hydroxy-1-methoxycarbonyl]methyl-2-p-nitrobenzyloxycarbonyl-1-azabicyclo[3.2.0]heptan und 4-Mercaptopyridin so läßt sich das Verfahren durch folgendes Schema verdeutlichen:

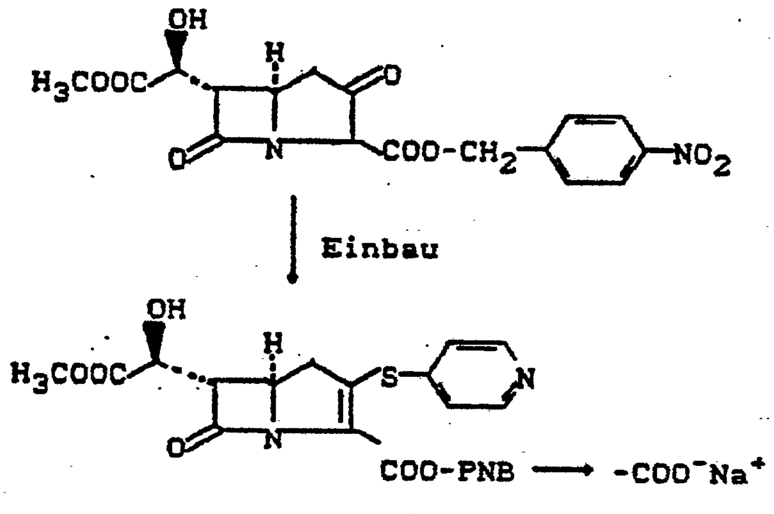


(PNB = 4-Nitrobenzyl)

Als Lösungsmittel eignen sich alle inerten organischen Lösungsmittel, die sich unter den Reaktionsbedingungen nicht verändern. Hierzu gehören bevorzugt Ether wie beispielsweise Diethylether, Dioxan, Tetrahydrofuran, Glykoldimethylether oder Butylmethylether, oder Kohlenwasserstoffe wie Benzol, Toluol oder Xylol, oder Halogenkohlenwasserstoffe wie beispielsweise Dichlormethan, Trichlorethan, Tetrachlormethan, 1,1,2-Trichlormethan, Dichlorethylen oder Trichlorethylen, Chlorbenzol oder Dichlorbenzol, oder Aceton, Essigester, Acetonitril, Dimethylformamid, Hexamethylphosphorsäuretriamid, N,N'-Dimethyl-propylenharnstoff, Acetonitril, oder Gemische der genannten Lösungsmittel.

Als Basen eignen sich die üblichen organischen Basen. Hierzu gehören bevorzugt tertiäre Amine wie beispielsweise Trimethylamin, Triethylamin, Tripropylamin, Tributylamin, Ethyldiisopropylamin, Pyridin, Dimethylaminopyridin, Picolin, Lutidin, N-Methylmorpholin, N-Methylpiperidin, 1,5-Diazabicyclo[5.4.0]undec-5-en (DBU) oder 1,5-Diazabicyclo[4.3.0]non-3-en (DBN).

Als Hilfsstoffe werden im allgemeinen Stoffe eingesetzt, die die Ketoester der allgemeinen Formel (II) in Verbindungen der allgemeinen Formel (IV)

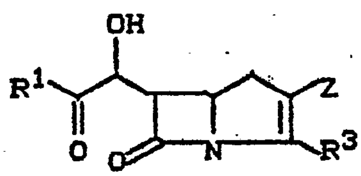

(IV)

in welcher

Z - für eine Abgangsgruppe aus der Reihe

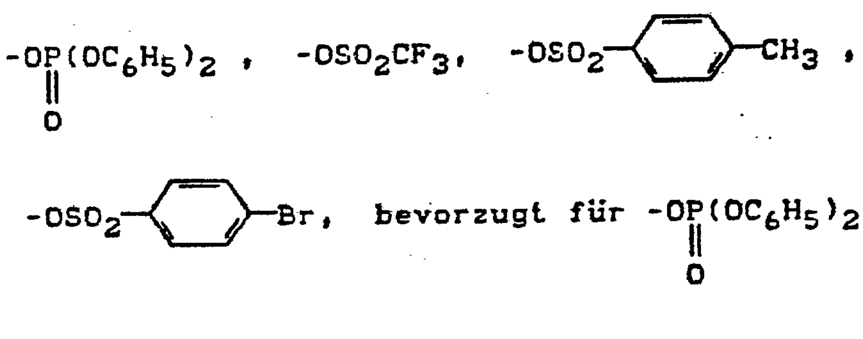


steht,

überführen.

Hierzu gehören bevorzugt Säurehalogenide oder -anhydride von 4-Toluolsulfonsäre, 4-Bromphenylsulfonsäure, Trifluormethansulfonsäure, oder Diphenylchlorphosphat. Besonders bevorzugt wird Diphenylchlorphosphat verwendet.

Die Zwischenprodukte der allgemeinen Formel (IV) können isoliert werden. Es hat sich jedoch als günstig erwiesen, die Reaktion in einem Schritt ohne Isolierung der Zwischenprodukte durchzuführen.

Bevorzugt wird das Verfahren durchgeführt indem man den Ketoester in einem geeigneten Lösungsmittel mit der Base, Diphenylchlorphosphat und danach mit dem entsprechenden Thiol, gegebenenfalls in Anwesenheit weiterer Basen umsetzt.

Ebenso ist es möglich, das Thiol (III) in Form seiner Salze einzusetzen oder das entsprechende Thiolat durch Zugabe von Base in der Reaktionsmischung herzustellen.

Die Base wird im allgemeinen in einer Menge von 1 bis 3 Mol, bevorzugt von 1 bis 1,5 Mol bezogen auf 1 Mol des Ketoesters eingesetzt. Das Thiol wird im allgemeinen in einer Menge von 1 bis 4 Mol, bevorzugt von 1 bis 2 Mol bezogen auf 1 Mol des Ketoesters eingesetzt. Erzeugt man aus dem Thiol in der Reaktionslösung das Thiolat, so wird weitere Base in Mengen von 1 bis 5 Mol, bevorzugt von 1 bis 2 Mol bezogen auf 1 Mol Thiol eingesetzt.

Die Umsetzung wird im allgemeinen in einem Temperaturbereich von -80°C bis +60°C, bevorzugt von -50° C bis +40°C durchgeführt.

Im allgemeinen führt man die Reaktion bei Normaldruck durch. Es ist aber ebenso möglich, bei Unterdruck oder Überdruck zu arbeiten.

Die als Ausgangsstoffe eingesetzten Thiole der allgemeinen Formel (III) sind bekannt oder können nach bekannten Methoden hergestellt werden [Patentanmeldungen: EP 36 650, 48 168,; DE 27 38 711; US-Patent 3 798 229].

Die als Ausgangsstoffe eingesetzten Verbindungen der allgemeinen Formel (II) sind neu. Sie können nach bekannten Verfahrensschritten gemäß dem folgenden Schema hergestellt werden:

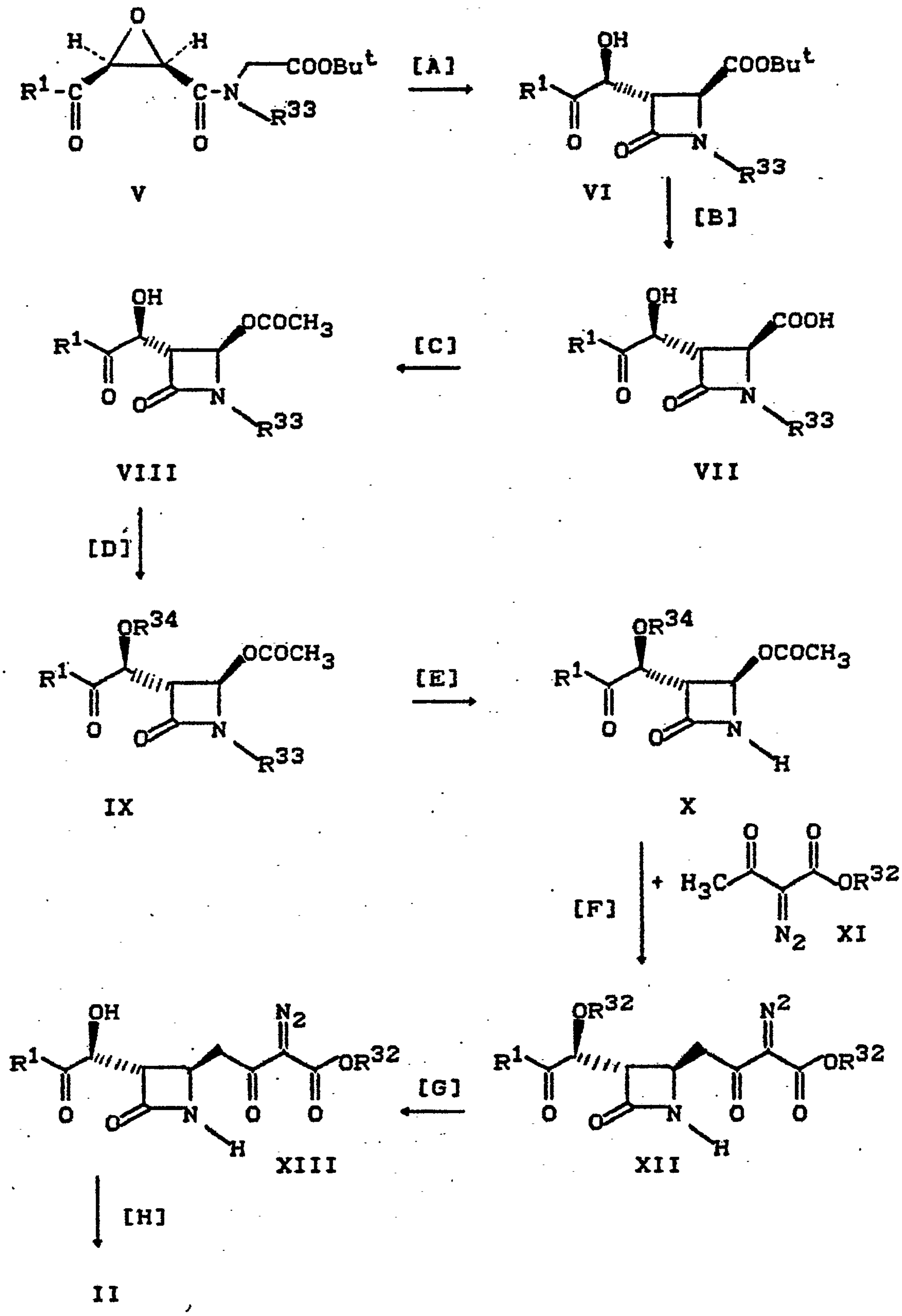


V, VI, VII, VIII, IX, X, XI, XII, XIII, II

in welchem
$R^1$ und $R^{32}$ die oben angegebene Bedeutung haben,
$R^{33}$ für eine Aminoschutzgruppe, bevorzugt für 4-Methoxyphenyl steht
und
$R^{34}$ eine Hydroxyschutzgruppe darstellt.

Danach wird im Schritt [A] das Epoxid (V) worin
$R^1$ die angegebene Bedeutung hat
und
$R^{33}$ für eine Aminoschutzgruppe wie bereits oben definiert, besonders bevorzugt für 4-Methoxyphenyl steht,
mit Tetrabutylammoniumfluorid in inerten organischen Lösungsmitteln wie Ethern, Kohlenwasserstoffen oder Halogenkohlenwasserstoffen, bevorzugt in Ethern wie Diethylether, Dioxan oder besonders bevorzugt Tetrahydrofuran, bei Temperaturen von -80°C bis +40°C, bevorzugt von -30°C bis +10°C zu den Azetidindionen (VI) umgesetzt, wie es von Ernest et al.in EP 126 709 beschrieben wird.

In Schritt [B] wird aus (VI) mit Hilfe von Säuren, bevorzugt starken Carbonsäuren oder Sulfonsäuren, oder besonders bevorzugt Trifluoressigsäure, in inerten organischen Lösungsmitteln wie Ethern, Kohlenwasserstoffen, oder bevorzugt Halogenkohlenwasserstoffen wie Tetrachlormethan, Trichlormethan oder besonders bevorzugt Dichlormethan, bei Temperaturen von -30°C bis +60°C, bevorzugt von 0°C bis +30°C die Säure (VII) hergestellt, wie es von M.Shiozaki et al. in Tetrahedron 40, 1795 (1984) beschrieben wird.

In Schritt [C] wird durch oxidative Decarboxylierung von (VII) mit Hilfe von Säuren, bevorzugt Carbonsäuren wie Essigsäure, Propionsäure, Chloressigsäure oder Trifluoressigsäure, besonders bevorzugt Essigsäure, sowie mit einem Oxidationsmittel bevorzugt Bleitetraacetat, und gleichzeitiger Acetylierung in einem Schritt das Acetylazetidinon (VIII) hergestellt, wie es von P.J.Reider et al. in Tetrahedron Lett. 1982, 2293 beschrieben wird.

In Schritt [D] wird die Verbindung (VIII) mit freier Hydroxygruppe in Verbindungen (IX) mit geschützter Hydroxygruppe überführt. Hierbei steht $R^{34}$ für eine übliche Hydroxyschutzgruppe. Hierzu gehören bevorzugt Silylgruppen wie z.B. Trimethylsilyl, tert-Butyl-dimethylsilyl, oder Oxycarbonylgruppen wie z.B. Benzyloxycarbonyl, 4-Nitrobenzyloxycarbonyl, tert-Butoxycarbonyl, Trichlorethoxycarbonyl oder Allyloxycarbonyl, oder sonstige gebräuchliche Schutzgruppen wie Formyl oder Acetyl. Besonders bevorzugt sind Silylschutzgruppen. Ganz besonders bevorzugt wird die tert-Butyldimethylsilylschutzgruppe eingesetzt. Besonders bevorzugt arbeitet man mit tert-Butyl-dimethylsilylchlorid und Dimethylaminopyridin in Dimethylformamid wie beispielsweise von M.Shiozaki et al. in Tetrahedron 40, 1795 (1984) beschrieben ist.

In Schritt [E] wird nach in der β-Lactam-Chemie üblichen Methoden die Aminoschutzgruppe ($R^{33}$) in (IX) abgespalten wie es beispielsweise von D.R.Kronenthal et al. in J.Org.Chem. 47, 2765 (1982) beschrieben wird. Als bevor zugte Aminoschutzgruppen werden die 4-Methoxyphenyl- und die 2,4-Dimethoxybenzylgruppe verwendet. Ganz besonders bevorzugt ist die 4-Methoxyphenylgruppe.Die Abspaltung erfolgt bevorzugt mit Hilfe von Cer(IV)nitrat in einem Acetonitril/Wasser-Gemisch, wie es beispielsweise von D.R. Kronenthal, C.Y. Han und M.K. Taylor in J. Org. Chem. 47, 2765 (1982) beschrieben wird.

In Schritt [F] wird das Azetidinon (X) mit dem Diazoketoester (XI) in Anwesenheit von Silylierungsmitteln mit Hilfe von Basen in inerten organischen Lösungsmitteln wie Ethern, z.B. Diethylether, Dioxan oder Tetrahydrofuran, oder Kohlenwasserstoffen z.B. Benzol, Toluol, Xylol oder Erdölfraktionen oder Halogenkohlenwasserstoffen z.B. Dichlormethan, Trichlormethan oder Tetrachlormethan, in mehrern Schritten zu den Verbindungen der Formel (XII) umgesetzt wie es beispielsweise von J.B. Buynak et al. in J. Chem. Soc. Chem. Commun. 1984, 948 oder von P.J. Reider et al., in Tetrahedron Letters 1982, 379 oder von M. Shiozaki et al., in J. Org. Chem. 39, 2399 (1983) oder Tetrahedron 40, 1795 (1984) oder von A.G.M. Barret in J. Org. Chem. 49, 1679 (1984) oder von T. Kametani et al., in Heterocycles 14, 1967 (1980) bzw. in J. Chem. Soc. Perkin I 1981, 228 beschrieben wird.

Bevorzugt werden jedoch die Verbindungen (XII) in Verfahrensschritt [F] nach einem Verfahren hergestellt, in dem man die Azetidinone (X) mit Diazoketoestern (XI) in einem inerten Verdünnungsmittel, in Gegenwart einer Base und eines Silylierungsmitteln in einem Eintopfverfahren umsetzt. Das Silylierungsmittel wirkt hierbei gleichzeitig als Katalysator.

Als Verdünnungsmittel kommen alle inerten Lösungsmittel in Frage, die sich unter den Reaktionsbedingungen nicht verändern. Dazu gehören vorzugsweise Ether, wie Dimethoxyethan, Diglyme, Triglyme, Tetrahydrofuran, Dioxan, Diethylether oder tert-Butylmethylether, oder halogenierte Kohlenwasserstoffe wie Dichlormethan, Trichlormethan, Tetrachlormethan, 1,1,2-Trichlorethan, Dichlorethylen oder Trichlorethylen, Chlorbenzol, Dichlorbenzol, Essigsäureethylester, Toluol oder Cyclohexan.

Die Umsetzung erfolgt im allgemeinen bei Temperaturen von -30°C bis +50°C, vorzugsweise bei Raumtemperatur. Als Basen eignen sich alle tertiären Amine. Bevorzugt zu nennen sind Triethyl-, Tripropyl- oder Tributylamin, Ethyldiisopropylamin, Dimethylaminopyridin, Pyridin, Picolin, Lutidin, N-Methylmorpholin, N-Methylpiperidin, 1,5-Diazabicyclo[5.4.0]undec-5-en (DBU) oder 1,5-Diazabicyclo[4.3.0]non-3-en (DBN).

Als Silylierungsmittel bzw. Katalysatoren kommen alle Silylierungsmittel in Betracht, die gleichzeitig als Lewis-Säuren wirken können. Hierzu gehören vorzugsweise Trialkylsilylperfluoralkansulfonate, Trialkylsilylalkansulfonate, Trialkylsilylarylsulfonate, Trialkylsilylperfluoralkanoate, Trialkylsilylalkanoate, Trialkylsilylhalogenide, Trialkylsilylperchlorate, Trialkylsilyldifluorphosphate, Trialkylsilyldichlorphosphate, Trialkylsilylfluorosulfonate, N,O-Bis(trialkylsilyl)acetamide, Trialkylsilylcyanide, Besonders geeignet sind Trimethylsilyltrifluormethansulfonat, Trimethylsilylnonafluorobutansulfonat, Trimethylsilyltrifluoracetat, Trimethylsilylperchlorat, Trimethylsilylfluorsulfonat, N,O-Bis(trimethylsilyl)trifluoracetamid.

Für die Durchführung des Verfahrens ist es notwendig zunächst

a.) pro Äquivalent der Verbindung XI 0,1 - 1,5, bevorzugt 0,5 - 1 Äquivalent der Verbindung X,
b.) pro Äquivalent der Verbindung XI 1 - 2, bevorzugt 1 - 1,5 Äquivalente Silylierungsmittel,
c.) pro Äquivalent Silylierungsmittel 1,01 - 3, bevorzugt 1,05 - 2 Äquivalent Base (Amin)

einzusetzen und nach 0,1 - 24 h, bevorzugt 0,1 - 12 h soviel weiteres Silylierungsmittel zuzugeben, daß dann das Silylierungsmittel in einem geringen Überschuß, bevorzugt in einem katalytischen Überschuß über die Base (Amin) vorhanden ist.

Bei der Durchführung des Verfahrens sind außer den oben angegebenen Mengenverhältnissen an Silylierungsmittel und Base (Amin) für jede freie, d.h. zu schützende Hydroxy-, Mercapto- bzw. Amingruppe in den Verbindungen X und XI jeweils ein zusätzliches Äquivalent Silylierungsmittel und Base (Amin) einzusetzen (z.B. wenn $R^{33} = H$).

Die Umsetzungsgeschwindigkeit ist weitgehend von der Menge des verwendeten Katalysators d.h. des Silylierungsmittelüberschusses abhängig. Die Umsetzung erfolgt innerhalb 0,1 bis 48 h, vorzugsweise von 0,1 bis 24 h.

Dieses neue Eintopfverfahren läßt sich durch folgendes Reaktionsschema verdeutlichen:

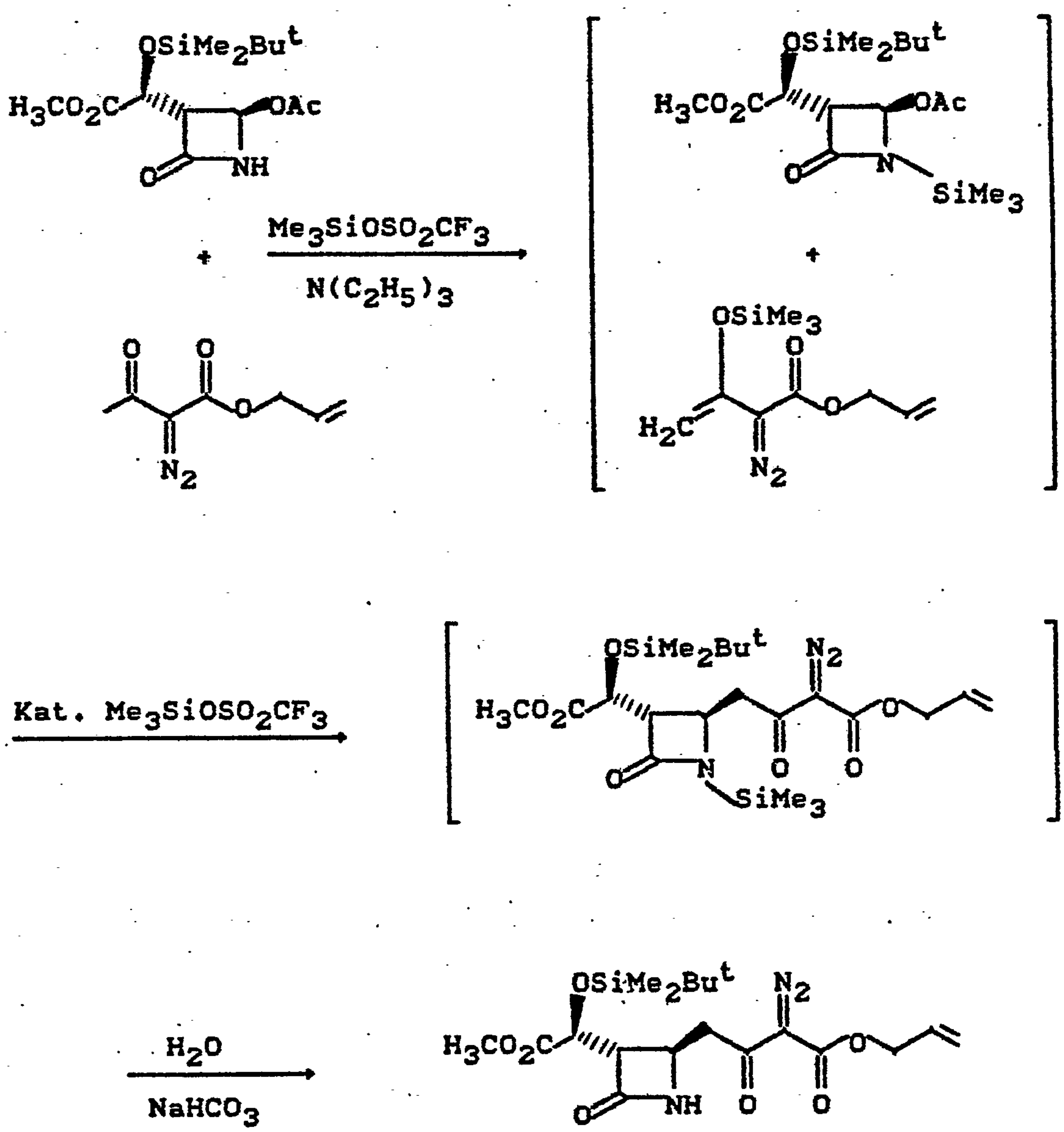


In Schritt [G] werden die Verbindungen der Formel (XIII) aus den Verbindungen (XII) durch Abspalten der Hydroxyschutzgruppe nach üblichen Methoden hergestellt, wie sie beispielsweise von A.Yoshida et al. in Tetrahedron Lett. 1984, 2793 beschrieben wird.

Der Ringschluß von Verbindungen (XIII) zu Verbindungen (II) in Schritt [H] erfolgt nach üblichen Methoden mit Hilfe von Katalysatoren wie Säure, Metallpulver oder Metallsalzen, bevorzugt Edel- bzw. Halbedelmetalle bzw. deren Salze in inerten organischen Lösungsmiteln wie Ethern, z.B. Diethylether, Dioxan oder Tetrahydrofuran, oder Kohlenwasserstoffen z.B. Benzol, Toluol, Xylol oder Erdölfraktionen, oder Halogenkohlenwasserstoffen z.B. Methylenchlorid, Chloroform oder Tetrachlorkohlenstoff, bei Temperaturen von 0°C bis 150°C, bevorzugt von 20°C bis 100°C, wie es beispielsweise von D.H. Shih et al., in Heterocycles 21, 29 (1984) beschrieben wird.

Besonders bevorzugt erfolgt der Ringschluß mit Rhodium-(II)-acetat in siedendem Methylenchlorid.

Die Verbindungen der allgemeinen Formel (XI) sind bekannt oder können nach literaturbekannten Methoden hergestellt werden [vgl. S. Julia et al., Compt. Rend. Acad. Sci., Paris, Section C 246, 1890 (1967), Europäische Patentanmeldungsschrift 78 026, EP 52 299, R.M. Kellogg et al., J.C.S. Chem. Commun. (1977) 932].

Die als Ausgangsstoffe eingesetzten Epoxide der allgemeinen Formel V sind neu und können gemäß folgendem Schema nach bekannten Methoden hergestellt werden:

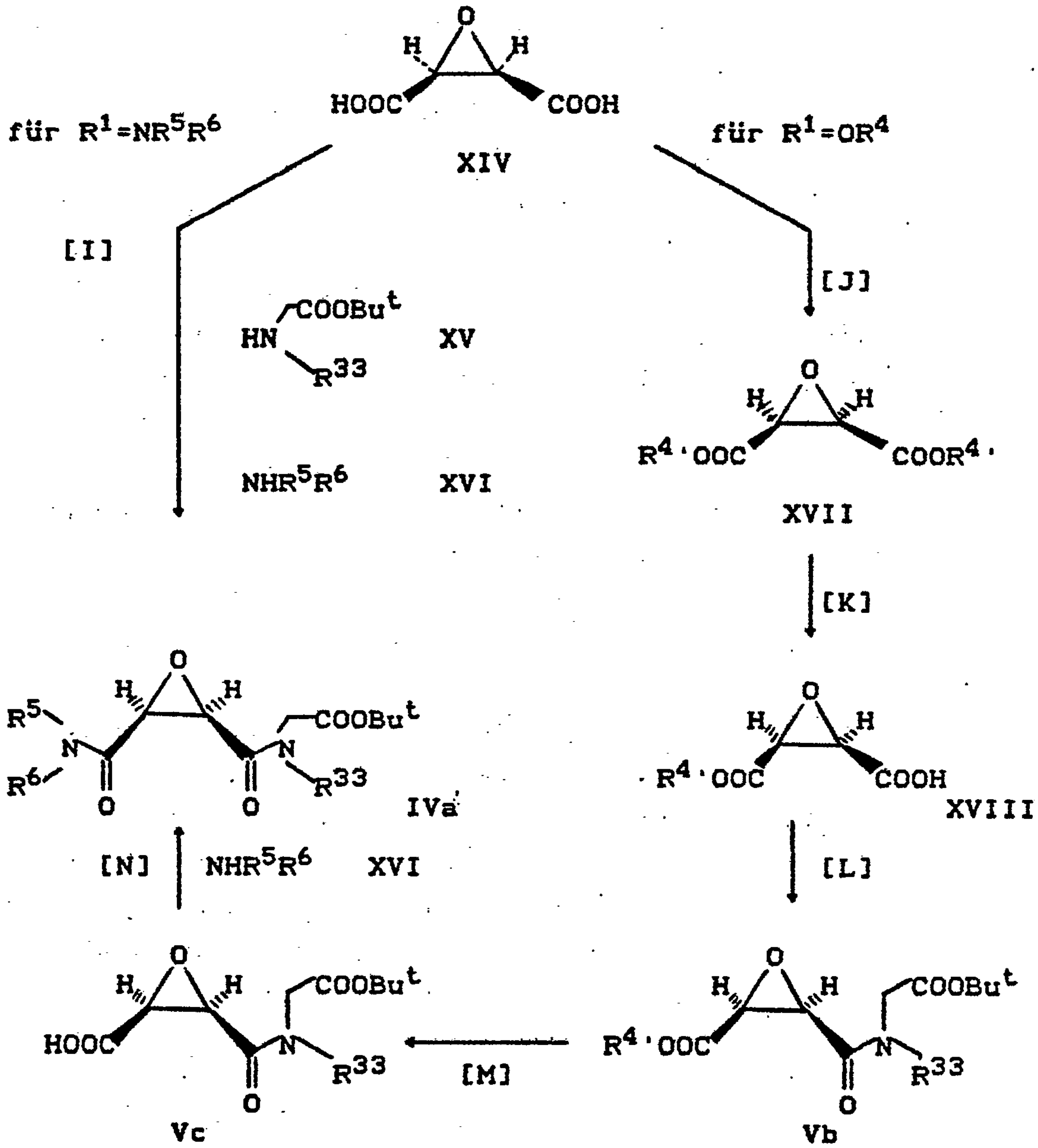


XIV, XV, XVI, XVII, XVIII, IVa', Vb, Vc

in welcher
$R^5$,$R^6$,$R^{33}$ die bereits angegebene Bedeutung haben,
$R^4$ die Bedeutung von $R^4$ hat, aber nicht für H steht.

Danach wird zur Herstellung der Ausgangsstoffe mit $R^1 = NR^5R^6$ (Formel Va) in Schritt [I] 2,3-Oxirandicarbonsäure (XIV), gegebenenfalls nach Aktivierung der Carboxylgruppen durch Überführung in ein gemischtes Anhydrid, z.B. mit Pivaloylchlorid, Chlorameisensäureethyl- oder isobutylester, nach Überführen in das Mesylat mittels Methansulfonsäurechlorid oder nach Überführung in einen aktivierten Ester, z.B. mit 1-Hydroxybenztriazol, Dicyclohexylcarbodiimid, mit einem Amin (XV) und einem Amin (XVI) - in der angegebenen Reihenfolge oder umgekehrt - gegebenenfalls in Anwesenheit eines Kondensationsmittels wie Carbodiimide umgesetzt, wie es beispielsweise von M.Shiozaki et al. in Tetrahedron 40, 1795 (1984) beschrieben wird.

Besonders bevorzugt wird die Reaktion in Anwesenheit von Kondensationsmitteln, wie Carbodiimiden, z.B. Dicyclohexylcarbodiimid, oder durch Aktivierung mit 1-Hydroxybenztriazol oder mit Cyclohexylcarbodiimid, in inerten organischen Lösungsmitteln wie Ethern, z.B. Diethylether, Dioxan oder Tetrahydrofuran, oder Halogenkohlenwasserstoffen z.B. Methylenchlorid, Chloroform oder Tetrachlorkohlenstoff oder Dimethylformamid.

Bei der Umsetzung von (XIV) mit Carbodiimiden erhält man in der Regel auch Verbindungen der Formel (Vd) mit $R^1$ = $-NR^4CONR^5R^6$ unter ansonsten gleichen Reaktionsbedingungen.

Die Herstellung der Verbindungen (Vb) mit $R^1$ = $OR^4$ erfolgt gemäß dem oben aufgeführten Schema über die Verfahrensschritte [J] - [L]. Hierbei wird nach bekannten Methoden in Schritt [J] zunächst die 2,3-Oxirandicarbonsäure (XIV) in den Diester (XVII) überführt, anschließend in Schritt [K] der Diester (XVII) enzymatisch in den Monoester (XVIII) überführt, der in Schritt [L] unter den bereits angegebenen Bedingungen [Schritt I] mit Glycinen (XV) zu den Azetidinonen (Vb) umgesetzt wird.

Alternativ lassen sich die Verbindungen (Va) durch Hydrolyse der Ester (Vb) zu den Monocarbonsäuren (Vc) (Schritt M) und anschließende Umsetzung der Säuren (Vc) mit Aminen (XVI) (Schritt N) unter den Bedingungen, wie sie für Schritt I bereits beschrieben sind, herstellen.

Beispielsweise können die einzelnen Verfahrensschritte durchgeführt werden, wie von M.Schneider et al. in Angew.Chem. 96, 55 (1984), von M.Shiozaki et al. in Tetrahedron 40, 1795 (1985) oder in Jpn.Kokai Tokyo Koho 80 85577 beschrieben.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline. Überraschenderweise zeigen die erfindungsgemäßen Carbapeneme der Formel (I) besonders gute metabolische Stabilität gegenüber dem Enzym Dehydropeptidase I (DHP I) und sind damit anderen Verbindungen dieser Substanzklasse in der Verträglichkeit überlegen.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnlich Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteriodes fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephri tis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intesti naltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enhalten, z.B. tierische und pflanzliche Fette, Wachse, Paraffine, Stärke, Tragant, Cellulose- derivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen im Vergleich zu Ciprofloxacin angegeben.

Antibakterielle Wirkung:

Das Agar-Lösungsverfahren wurde zur Bestimmung der antibakteriellen Wirkung verwendet, wobei ein Iso-Sensitest-Agar verwendet wurde und die minimale Hemmkonzentration (MHK) der Probe wurde durch µg/ml des Nährbodens ausgedrückt (Tabelle).

Tabelle: MHK (µg/ml) für Verbindung aus Beispiel 14

| Keim | | MHK |
|---|---|---|
| Staph. | 133 | 0.5 |
| " | 24455 | 0.5 |
| " | E 25185 | 1.0 |

| Beispiel für eine erfindungsgemäße Tablette | |
|---|---|
| Jede Tablette enthält: | |
| Verbindung des Beispiels 14 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

| | |
|---|---|
| Die Lackhülle enthält: | |
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

Herstellungsbeispiele

Beispiel 1

2,3-Oxirandicarbonsäuredimethylester

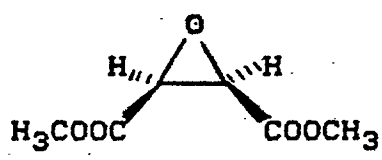

Eine Lösung von 247.0 g (1.87 mol) cis- 2,3-Oxirandicarbonsäure [G.B. Payne et al. J. Org. Chem., 24, 54 (1959)] und 5 ml (94 mmol - 0.05 equiv.) 96%iger Schwefelsäure in 1.5 l Methanol wurde im Kreislauf über einen mit 100 g trockenem Molekularsieb (3Å) gefüllten Soxhlet-Extraktor 22 h im Rückfluß erhitzt. Nach Abkühlung wurde die Reaktionslösung in ein Gemisch aus eiskalter $NaHCO_3$-Lösung und Ether eingerührt, mehrmals mit Ether extrahiert, mit NaCl-Lösung gewaschen und über $MgSO_4$ getrocknet. Nach Abdampfen des Lösungsmittels i.Vak. erhielt man 200.6 g (67% der Theorie) der Titelverbindung als Öl.
Sdp.: 112° C / 1 mm
$^1$H-NMR (300 MHz, $CDCl_3$) $\delta = 3.72$ (s, 2H, CH), 3.81 (s, 6H, $COOCH_3$).
$C_6H_8O_5$ (160.1) Ber.: C 45.01 H 5.04
Gef.: C 44.6 H 5.3

Beispiel 2

(2R,3S)-2,3-Oxirandicarbonsäuremonomethylester

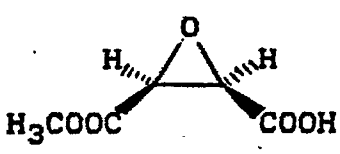

Zu einer Lösung von 16,0 g (0,1 mol) 2,3-Oxirandicarbonsäuredimethylester in 100 ml Phosphatpuffer (0,1 M, pH 7) wurden unter Rühren 1000 Einheiten (ca. 10 mg) Schweineleber-Esterase gegeben. Die Mischung wurde 20 h bei Raumtemperatur gerührt, wobei der pH-Wert durch Zugabe einer 1 N NaOH Lösung aus einem Autotitrator konstant gehalten wurde. Nach Zugabe von 100 ml (0,1 mol) NaOH wurde das Gemisch gekühlt und unter Rühren mit 5 N $H_2SO_4$ auf pH 2 gestellt. Die Lösung wurde mit festem NaCl gesättigt und erschöpfend mit Ethylacetat extrahiert. Nach Trocknen der organischen Phase mit $MgSO_4$ und Abdampfen des Lösungsmittels i.Vak. erhielt man 7,74 g (53% der Theorie) der Titelverbindung als Gemisch der Enantiomere im Verhältnis 3:2 [NMR-spektroskopisch bestimmt durch Zugabe von (R)-(+)-1-Phenethylamin zum Rohprodukt als Verhältnis der $CH_3$-Signale bei δ 3.60 (s) und 3.63 (s)].
Sdp.: ca. 150°C / 1.5 mm (Kugelrohr)
$[\alpha]_D^{20}$ = -3.45° (c = 0.489 in $CHCl_3$)

$C_5H_6O_5$ (146.1) Ber.: C 41.11 H 4.14
Gef.: C 40.8 H 4.4

Beispiel 3

(2R, 3S)-N-(t-Butoxycarbonylmethyl)-N-(4-methoxyphenyl)-3-methoxycarbonyl-2,3-epoxypropionamid

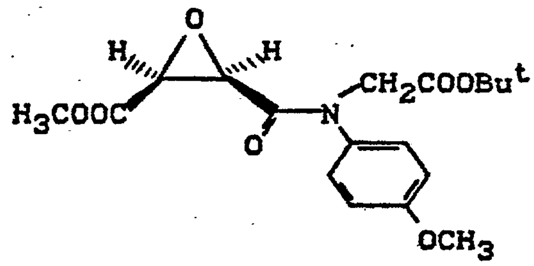

Zu einer Lösung von 129,0 g (0,88 mol) (2R,2S)-2,3-Oxirandicarbonsäuremonomethylester und 196,0 g (0,8 mol) t-Butyl-N-(4-methoxyphenyl)glycinat [M. Shiozaki et al., Tetrahedron 40, 1795, (1984)] in 1100 ml THF wurden innerhalb von 15 min. eine Lösung von 188,0 g (0,88 mol) Dicyclohexylcarbodiimid in 300 ml THF getropft. Anschließend wurde 30 min. bei Raumtemperatur nachgerührt, der entstandene Niederschlag durch Filtration abgetrennt und die Filtratlösung i.Vak. eingedampft. Nach Reinigung des Produktes an 1,8 kg Kieselgel (Toluol : Ethylacetat = 7:3) erhielt man 263,2 g (90% der Theorie) der Titelverbindung als Kristalle, Schmp.: 89°C.
$[\alpha]_D^{20}$ = -21.78° (c = 0,435 in $CHCl_3$).

Das im Herstellungsbeispiel bestimmte Enantiomerenverhältnis von 3:2 wurde durch NMR-shift Versuche mit $Eu(TFC)_3$ bestätigt. IR ($CHCl_3$) 1737 (C=O, Ester), 1681 (C=O, Amid) 1509 $cm^{-1}$.
$^1H$-NMR (250 MHz, $CDCl_3$): δ = 1,44 (s, 9H, $CH_3$-C); 3,46 und 3,51 (AB, J=4,5 Hz, 2H, Oxirn-H); 3,82 (s, 6H, $OCH_3$, $COOCH_3$); 4,08 und 4,36 (AB, J=16 Hz, $CH_2COO$); 6,91 und 7,31 (AB, J=9 Hz, 4H, p-$H_3CO$-$C_6H_4$).

Beispiel 4

(3S,4S)-4-t-Butoxycarbonyl-3-[(1S)-1-hydroxy-1-methoxycarbonyl]methyl-1-(4-methoxyphenyl)azetidin-2.on

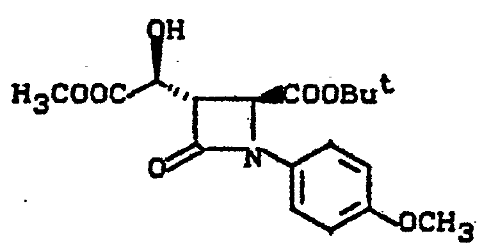

Eine Lösung von 4,19 g (11,5 mol) (2R,3S)-N-(t-Butoxycarbonylmethyl)-N-(4-methoxyphenyl)-3-methoxycarbonyl-2,3-epoxypropionamid in 12 ml THF wurde zu einer auf 0°C gekühlten Mischung von 12,6 mmol (1,1 equiv.) Tetrabutylammoniumfluorid und 6 g Molekularsieb (4Å) in 25 ml THF getropft. Nach 1,2 h bei 0°C wurde filtriert. Die Filtratlösung wurde mit Toluol versetzt und das THF wurde i.Vak. abgetrennt. Die zurückbleibende Toluol-Lösung wurde mehrmals mit Wasser gewaschen und über $MgSO_4$ getrock-

net. Nach Abdampfen des Lösungsmittels i.Vak. und Chromatographie des Rohproduktes an 150 g Kieselgel (Toluol : Ethylacetat = 4:1) erhielt man 2,44 g (58% der Theorie) der Titelverbindung als Kristalle, Schmp.: 82°C, $R_f$ = 0,21 (Toluol : Ethylacetat = 7:3).
IR (KBr) 3425, 1758, 1740, 1516 $cm^{-1}$.
$^1$H-NMR (250 MHz, $CDCl_3$): δ = 1,45 (s, 9H, $CH_3$-C), 3,24 (d, J=4 Hz, 1H, OH); 3,75 (dd, J=3 Hz, 2,5 Hz, H-3); 3,78 (s, 3H, $OCH_3$); 3,86 (s, 3H, $COOCH_3$); 4,46 (d, J=2,5 Hz, 1H, H-4); 4,75 (dd, J=4 Hz, 3Hz, <u>CH</u>-$COOCH_3$); 6,85 und 7,25 (AB, J=9 Hz, 4H, p-$H_3CO$-$C_6H_4$).
MS (70 eV) m/e = 365 ($M^+$); ber. 365,4

<u>Beispiel 5</u>

(3S, 4S)-4-Carboxy-3-[(1S)-1-hydroxy-1-methoxycarbonyl]methyl-1-(4-methoxyphenyl)azetidin-2-on

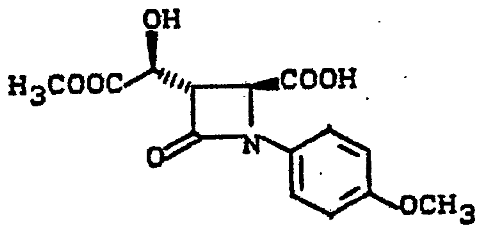

Eine Lösung von 6,49 g (17,8 mmol) (3S, 4S)-4-t-Butoxycarbonyl-3-[(1S)-1-hydroxy-1-methoxycarbonyl]methyl-1-(4-methoxyphenyl)azetidin-2-on in 23 ml Dichlormethan wurde bei Raumtemperatur mit 23 ml Trifluoressigsäure versetzt. Nach 3 h wurde die Lösung i.Vak. eingedampft, mit Toluol versetzt und erneut eingedampft. Diese Procedur wurde noch 2 x wiederholt, dann wurde der kristalline Rückstand mit Ether verrührt, abgesaugt und über $P_4O_{10}$ im Hochvakuum getrocknet. Man erhielt 4,52 g (82% der Theorie) der Titelverbindung als farblose Kristalle, Schmp.: 147°C
IR(KBr) 1755, 1729, 1517, 1298 $cm^{-1}$.
$^1$H-NMR (250 MHz, DMSO): δ = 3,68 (s, $OCH_3$), 3,73 (s, $COOCH_3$); 3,72 (m, H-3) zusammen 7H, 4,51 (d, J=2,5 Hz, 1H, H-4); 4,58 (d, J=3 Hz, 1H, <u>CH</u>-$COOCH_3$); 6,96 und 7,95 (AB, J=9 Hz, 4H, p-$H_3CO$-$C_6H_4$).

<u>Beispiel 6</u>

(3R, 4R)-4-Acetoxy-3-[(1S)-1-hydroxy -1-methoxycarbonyl] methyl-1-(4-methoxyphenyl)azetidin-2-on

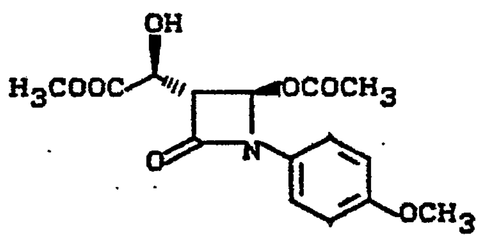

Eine Lösung von 4,38 g (14,2 mmol) (3S, 4S)-4-Carboxy-3-[(1S)-1-hydroxy -1-methoxycarbonyl]methyl-1-(4-methoxyphenyl)azetidin-2-on in 30 ml DMF und 3 ml Eisessig wurde mit 6,27 g (14,2 mmol) Bleitetraacetat versetzt und genau 7 min. in einem auf 60°C vorgeheizten Ölbad erhitzt. Danach wurde abgekühlt und in ein Gemisch aus Wasser, NaCl-Lösung und Ethylacetat gegossen. Es wurde mit Ethylacetat extrahiert (4 x) und die organischen Extrakte wurden mit Wasser (3 x) und $NaHCO_3$-Lösung bis zur Neutralität gewaschen und mit $MgSO_4$ getrocknet. Nach Abdampfen des Lösungsmittels in Vak. erhielt man 2,20 g (47% der Theorie) der Titelverbindung als farblose Kristalle, Schmp.: 138°C, $R_f$ = 0,35 (Toluol : Ethylacetat = 1:1).
IR(KBr) 3404 (OH), 1755 (C=O, β-Lactam), 1737 (C=O,Ester), 1517 $cm^{-1}$.
$^1$H-NMr (200 MHz, $CDCl_3$): δ = 2,13 (s, 3H, $OCOCH_3$); 3,33 (bs, 1H, OH); 3,62 (dd, J=2 Hz, 1 Hz, 1H, H-3); 3,78 (s, 3H, $COOCH_3$); 3,85 (s, 3H, $OCH_3$); 4,65 (d, J=2 Hz, 1H, <u>CH</u>-$COOCH_3$); 6,18 (d, J=1 Hz, 1H, H-4); 6,85 und 7,33 (AB, J=9 Hz, 4H, p-$H_3CO$-$C_6H_4$).

Beispiel 7

(3R, 4R)-4-Acetoxy-3-[(1S)-1-t-butyldimethylsilyloxy-1-methoxycarbonyl]methyl-1-(4-methoxyphenyl) azetidin-2-on

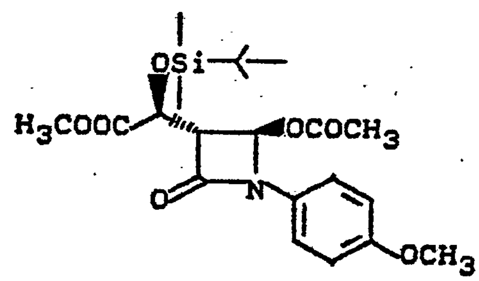

Eine Lösung von 10,29 g (30,8 mmol) (3R, 4R)-4-Acetoxy-3-[(1S)-1-hydroxy-1-methoxycarbonyl]methyl-1-(4-methoxyphenyl)azetidin-2-on und 5,1o g (33,9 mmol - 1.1 equiva.) t-Butyldimethylsilylchlorid in 30 ml DMF wurden mit 4,32 g (35,4 mmol - 1,15 equiv.) Dimethylaminopyridin versetzt und 22 h bei Raumtemperatur gerührt. Zur Aufarbeitung goß man in ein Gemisch aus kalter 1 N HCl und Ethylacetat, extrahierte mit Ethylacetat (2 x), wusch die Extrakte mit Wasser, $NaHCO_3$-Lösung und trocknete über $MgSO_4$. Nach Abdampfen des Lösungmittels i.Vak. erhielt man 11,61 g (86% der Theorie) der Titelverbindung als farblose Kristalle, Schmp.: 102°C, $R_f$ = 0,33 (Toluol : Ethylacetat = 9:1).
IR(KBr) 1759, 1734, 1516, 1246 $cm^{-1}$.
$^1$H-NMR (200 MHz, $CDCl_3$): δ = 0,01 und 0,10 (s, je 3H, $CH_3Si$); 0,75 (s, 9H, $CH_3$-C-Si); 2,08 (s, 3H, $OCOCH_3$); 3,60 (dd, J=2 Hz, 0,5 Hz, 1H, H-3); 3,76 (s, 6H, $OCH_3$, $COOCH_3$); 4,63 (d, J=2 Hz, 1H, CH-$COOCH_3$); 6,35 (d, J=0,5 Hz, 1H, H-4); 6,83 und 7,30 (AB, J=9 Hz, 4H, p-$H_3CO$-$C_6H_4$).
MS (CI, 150 eV, $NH_3$): m/e = 438 (M+H), 455 (M+$NH_4$)

Beispiel 8

(3R, 4R)-4-Acetoxy-3-[(1S)-t-butyldimethylsilyloxy-1-methoxycarbonyl]methyl-azetidin-2-on

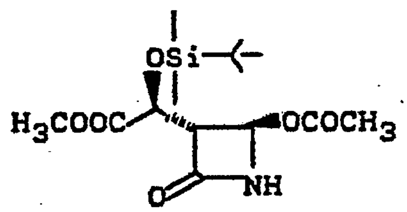

Eine auf 0°C gekühlte Lösung von 1,58 g (3,64 mmol) (3R, 4R)-4-Acetoxy-3-[(1S)-1-t-butyldimethylsilyloxy-1-methoxycarbonyl]methyl-1-(4-methoxyphenyl)azetidin-2-on in 18 ml Acetonitril wurde langsam mit einer Lösung von 5,94 g (10,8 mol - 3 eqiv.) Ammoniumcer(IV)nitrat in 21 ml Wasser versetzt, so daß die Innentemperatur nicht über +5°C stieg. Anschließend rührte man 30 min. bei 0°C und goß in ein Gemisch aus NaCl-, $NaHCO_3$-Lösung und Ethylacetat. Man extrahierte mit Ethylacetat, wusch mit $NaHCO_3$- und NaCl-Lösung und trocknete über $MgSO_4$. Nach Abdampfen des Lösungsmittels i.Vak. und Chromatographie des Rückstandes an 50 g Kieselgel (Toluol : Ethylacetat = 4:1) erhielt man 930 mg (78% der Theorie) der Titelverbindung.
$R_f$ = 0,24 (Toluol : Ethylacetat = 4:1)
IR(KBr) 3344, 1791, 1750, 1731 $cm^{-1}$.
$^1$H-NMR (200 MHz, $CDCl_3$): δ = 0,03 und 0,05 (s, 6H, $CH_3$-Si); 0,88 (s, 9H, $CH_3$-C-Si); 2,04 (s, 3H, $OCOCH_3$); 3,57 (dd, J=2 Hz, 1Hz, H-3); 3,76 (s, 3H, $COOCH_3$); 4,57 (d, J=2 Hz, 1H; CH-$COOCH_3$); 5,83 (d, J=1 Hz, H-4); 6,51 (bs, 1H, NH).

Beispiel 9

4-Nitrobenzyl-2-diazo-3-oxo-butanoat

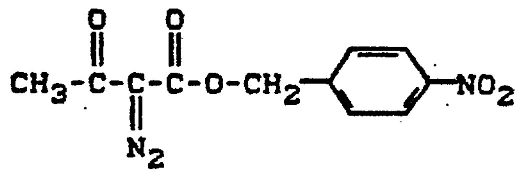

Eine auf 0°C gekühlte Lösung von 33,18 g (0,14 mol) Acetessigsäure-4-nitrobenzylester und 30,40 g (0,15 mol) p-Toluolsulfonylazid in 280 ml wasserfreiem Acetonitril wurde tropfenweise mit 35,0 ml (0,25 mol - 1,8 euiv.) Triethylamin versetzt. Die Mischung wurde 2 h bei 0°C gerührt, dann wurde der entstandene Niederschlag abgesaugt, mit Ether gewaschen und über $P_4O_{10}$ im Hochvakuum getrocknet. Man erhielt 20,3 g (55% der Theorie) der Titelverbindung als farblose Kristalle, Schmp.: 132°C. Durch Chromatographie der Filtratlösung an 400 g Kieselgel (Toluol : Ethylacetat = 9:1) wurden weitere 6,3 g (17% der Theorie) der Titelverbindung erhalten, $R_f$ = 0,35 (Toluol : Ethylacetat = 9:1).
IR(KBr) 2144 ($N_2$), 1709 (C=O), 1662 (C=O), 1514 ($NO_2$ asym.), 1343 $cm^{-1}$ ($NO_2$ sym.).
$^1$H-NMR (200 MHz, DMSO): δ = 2,44 (s, 3H, $CH_3$); 5,44 (s, 2H, $CH_2$); 7,72 (d, J=9 Hz, 2H, $H_{arom}$).; 8,28 (d, J=9 Hz, 2H, $H_{arom}$).
$C_{11}H_9N_3O_5$ (263,2) Ber.: C 50,20 H 3,45 N 15,96
Gef.: C 50,2 H 3,4 N 15,9

Beispiel 10

(3S, 4R)-3-[(1S)-1-t-Butyldimethylsilyloxy-1-methoxycarbonyl]methyl-4-(3-p-nitrobenzyloxycarbonyl-3-diazo-2-oxo-propyl)-azetidin-2-on

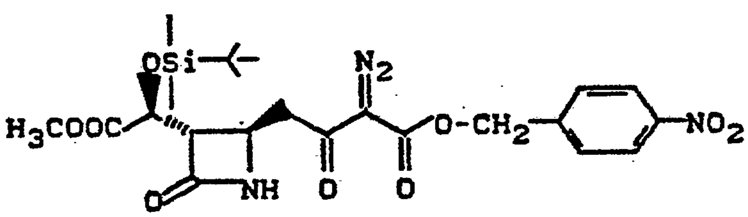

Eine auf 0°C gekühlte Lösung von 4,53 g (13,7 mmol) (3R, 4R)-4-Acetoxy-3-[(1S)-1-t-butyldimethylsilyloxy 1-methoxycarbonyl]methyl-azetidin-2-on und 4.31 g (16.4 mmol) 4-Nitrobenzyl-2-diazo-3-oxo-butanoat in 125 ml wasserfreiem Dichlormethan wurde mit 4,74 ml (34,2 mmol) Triethylamin und dann tropfenweise mit 6,08 ml (31,4 mmol) Trimethylsilyltrifluormethansulfonat versetzt. Das Kühlbad wurde entfernt, man rührte 15 min. bei Raumtemperatur, gab 0,79 ml (4,1 mmol) Trimethylsilyltrifluormethansulfonat zu und rührte weitere 15 min. bei Raumtemperatur. Danach goß man die Reaktionslösung in ein Gemisch aus kalter, gesättigter $NaHCO_3$-Lösung und Ethylacetat, extrahierte mit Ethylacetat, wusch die organischen Extrakte mit gesättigter NaCl-Lösung und trocknete über $MgSO_4$. Das Lösungsmittel wurde i.Vak. abgedampft und der Rückstand an 450 g Kieselgel (Toluol : Ethylacetat = 4:1) chromatographiert. Man erhielt 4,70 g (64% der Theorie) der Titelverbindung, $R_f$ = 0,40 (Toluol : Ethylacetat = 1:1).
IR(KBr) 2138, 1758, 1725, 1656, 1526, 1349 $cm^{-1}$.
$^1$H-NMR (250 MHz, $CDCl_3$): δ = 0,09 und 0,10 (s, 6H, $CH_3$-Si); 0,90 (s, 9H, $CH_3$-C-Si); 3,04 (dd, J=17 Hz, 1H, H-1); 3,26 (m, H-3); 3,27 (dd, J=17 Hz, 4,5 Hz, H-1); zusammen 2H, 3,74 (s, 3H, $COOCH_3$); 4,18 (m, 1H, H-4); 4,59 (d, J=2,5 Hz, 1H, CH-$COOCH_3$); 5,34 (s, 2H, $COOCH_2$); 6,05 (s, 1H, NH); 7,50 und 8,23 (AB, J=9 Hz, 4H, p-$NO_2$-$C_6H_4$).

Beispiel 11

(3S, 4R)-3-[(1S)-1-Hydroxy -1-methoxycarbonyl]methyl-4-[3-diazo-3-p-nitrobenzyloxycarbonyl-2-oxo-propyl]-azetidin-2-on

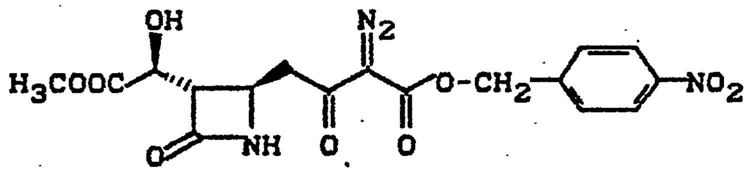

3,92 g (7,33 mmol) (3S, 4R)-3-[1-(1S)-1-Hydroxyy-1-methoxycarbonyl]methyl-4-[3-diazo-3-p-nitrobenzyloxycarbonyl-2-oxopropyl]-azetidin-2-on wurden bei 0°C in 20 ml einer Maßlösung von 1,80 ml (14,7 mmol - 2 equiv.) Bortrifluoridetherat in Acetonitril gelöst und 1 h bei dieser Temperatur gerührt. Danach wurde die Reaktionslösung in ein kaltes Gemisch aus $NaHCO_3$-Lösung und Ethylacetat eingerührt. Man extrahierte mit Ethylacetat, wusch die organischen Extrakte mit gesättigter NaCl-Lösung und trocknete über $MgSO_4$. Nach Abdampfen des Lösungsmittels i.Vak. und Chromatographie des Rohprodukts an 50 g Kieselgel (Toluol : Ethylacetat = 1:4), erhielt man 2,73 g (89% der Theorie) der Titelverbindung als Feststoff, $R_f$ = 0,18 (Toluol : Ethylacetat = 1:4).

IR(KBr) 3326 (OH), 2135 ($N_2$), 1758 (C=O, β-Lactam), 1744, 1725, 1658 $cm^{-1}$.

$^1$H-NMR (200 MHz, $CDCl_3$): δ = 3,1 - 3,3 (m, 4H, H-1, H-3, OH); 3,83 (s, 3H, $COOCH_3$); 4,01 (m, 1H, H-4); 4,62 (m, 1H, <u>CH</u>-$COOCH_3$); 5,34 (s, 2H, $COOCH_2$); 6,09 (bs, 1H, NH); 7,50 und 8,23 (AB, J=9 Hz, 4H, p-$NO_2$-$C_6H_4$).

Beispiel 12

(2S, 5R, 6S)-3,7-Dioxo-6-[(1S)-1-hydroxy-1-methoxycarbonyl]methyl-2-p-nitrobenzyloxycarbonyl-1-aza-bicyclo-[3.2.0]heptan

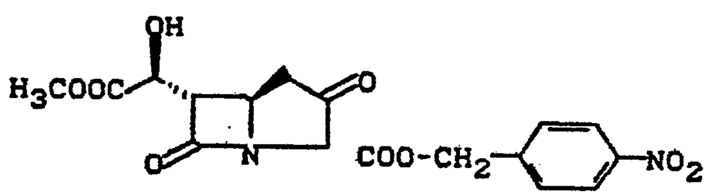

Eine Lösung von 2,73 g (6,5 mmol) (3S, 4R)-3-[(1S)-1-Hydroxy -1-methoxycarbonyl]methyl-4-[3-p-nitrobenzylcarbonyl-3-diazo-2-oxopropyl]-azetidin-2-on in 50 ml wasserfreiem Dichlormethan wurde in Gegenwart von 85 mg (3 mol %) Rhodium(II)acetat 30 Minuten im Rückfluß erhitzt. Die Reaktionslösung wurde durch wenig Kieselgur filtriert und im Vakuum eingedampft. Man erhielt 1,45 g (57% der Theorie) der Titelverbindung als farblose Kristalle, Schmp.: 169°C, $R_f$ = 0,41 (Toluol : Ethylacetat = 3:7).

IR(KBr) 3460 (OH), 1760 (C=O, β-Lactam), 1744 (C=O, Ester), 1519 ($NO_2$, asym.), 1350 $cm^{-1}$ ($NO_2$, sym.).

$^1$H-NMR (200 MHz, $CDCl_3$): δ = 2,46 (dd, J=19 Hz, 7,5 Hz, 1H, H-1); 2,88 (dd, J=19 Hz, 6Hz, 1H, H-1); 3,23 (d, J=5 Hz, 1H, OH), 3,58 (dd, J=5 Hz, 2,5 Hz, 1H, H-6); 3,85 (s, 3H, $COOCH_3$); 4,14 (ddd, J=7,5 Hz, 6 Hz, 2,5 Hz, 1H, H-5); 4,69 (m, 1H, <u>CH</u>-$COOCH_3$); 4,76 (s, 1H, H-3); 5,21 und 5,33 (AB, J=12,5 Hz, 2H, $COOCH_2$); 7,49 und 8,21 (AB, J=8,5 Hz, 4H, p-$NO_2$-$C_6H_4$).

MS: m/e (FAB) 393 (M+H).

$C_{17}H_{16}N_2O_9$ (392,3) Ber.: C 52,05 H 4,11 N 7,14

Gef.: C 52,0 H 4,1 N 7,0

Beispiel 13

(5R,6S)-6-[(1S)-1-Hydroxy-1-methoxycarbonyl]methyl-7-oxo-3-(4-pyridinylthio)-1-azabicyclo[3.2.0]-hept-2-en-2-carbonsäure-p-nitrobenzylester

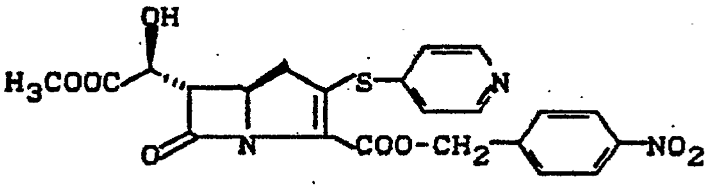

Eine auf 0°C gekühlte Lösung von 645 mg (1,64 mmol) (2S,5R,6S)-3,7-Dioxo-6-[(1S)-1-hydroxy-1-methoxycarbonyl]methyl-2-p-nitrobenzyloxycarbonyl-1-azabicyclo[3.2.0]-heptan in 8 ml wasserfreiem Acetonitril wurde innerhalb von 10 min gleichzeitig mit 0,3 ml (1,73 mmol - 1,05 equiv.) Ethyldiisopropylamin und 0,36 ml (1,73 mmol) Diphenylchlorphosphat versetzt. Danach rührte man 15 min. bei 0°C, kühlte auf -40°C ab und gab nacheinander 0,32 ml (1,81 mmol - 1.1 equiv.) Ethyldiisopropylamin und 201 mg (1,81 mmol) 4-Mercaptopyridin zu. Die Reaktionsmischung durfte sich auf 0°C erwärmen und wurde dann in ein kaltes Gemisch aus $NaHCO_3$-Lösung und Ethylacetat eingerührt. Die Mischung wurde mit Ethylacetat extrahiert, mit NaCl-Lösung und Wasser gewaschen und über $MgSO_4$ getrocknet. Nach Abdampfen des Lösungsmittels i.Vak. und Chromatographie des Rückstandes an 100 g Kieselgel (Ethylacetat : Aceton = 95:5) erhielt man 208 mg (26% der Theorie) der Titelverbindung als farblose Kristalle, Schmp.: 155°C, $R_f$ = 0,29 (Ethylacetat : Aceton = 95:5).
IR(KBr) 3395, 1774, 1740, 1710, 1516, 1349 $cm^{-1}$.
$^1$H-NMR (200 MHz, $CDCl_3$): δ = 2,83 (dd, J=9 Hz, 2,5 Hz, 2H, H-1); 3,63 (dd, J=4 Hz, 3 Hz, 1H, H-6); 3,83 (s, 3H, $COOCH_3$); 4.23 (dt, J=9 Hz, 3 Hz, 1H, H-5); 4,67 (d, J=4 Hz, 1H, <u>CH</u>-$COOCH_3$); 5,33 und 5.56 (AB, J=14 Hz, 2H, $COOCH_2$); 7,44 und 8,67 (AB, J=5,5 Hz, 4H, pyridinyl-H); 7,71 und 8,26 (AB, J=9,5 Hz, 4H, p-$NO_2$-$C_6H_4$).
MS: m/e (FAB) 486 (M+H), 524 (M+K); ber.: 485,46

Beispiel 14

Natrium-(5R,6S)-6-[(1S)-1-hydroxy-1-methoxycarbonyl]-methyl-7-oxo-3-(4-pyridinylthio)-1-azabicyclo[3.2.0]hept-2-en-carboxylat

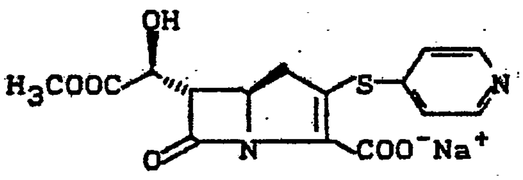

Eine Lösung von 245 mg (0,5 mmol) (5R,6S)-6-[(1S)-1-Hydroxy-1-methoxycarbonyl]methyl-7-oxo-3-(4-pyridinylthio-1-azabicyclo[3.2.0]hept-2-en-2-carbonsäure-p- nitrobenzylester in 15 ml eines Gemisches, bestehend aus THF und 0,1 <u>M</u> Phosphatpuffer pH 7 (1:1) wurde in Gegenwart von 245 mg Palladium auf Kohle (10%) 2 h lang hydrogenolysiert. Danach wurde der Katalysator durch Filtration über Kieselgur abgetrennt, das THF i.Vak. entfernt und die zurückbleibende Lösung auf pH 7 gestellt. Die wässrige Lösung wurde 2 x mit Ethylacetat extrahiert, i.Vak. auf ca. 10ml eingeengt und auf eine Säule mit 120 ml Diaion HP 20 geladen. Es wurde mit Wasser, welches einen steigenden Anteil an Acetonitril enthielt eluiert. Die das Produkt enthaltenden Fraktionen wurden gesammelt, durch einen 0,2µm Filter filtriert und gefriergetrocknet. Man erhielt 93 mg (50% der Theorie) der Titelverbindung als farbloses Lyophilisat, $R_f$ = 0,19 (Acetonitril : Wasser = 9:1), in einer Reinheit von 97% (HPLC).
IR(KBr) 3340, 1752, 1611, 1580 $cm^{-1}$.
$^1$H-NMR (200 MHz, $D_2O$): δ = 2.95 (d, J=9,5 Hz, 2H, H-1); 3,78 (s, 3H, $COOCH_3$); 3,92 (dd, J=3 Hz, 2,5 Hz, 1H, H-6); 4,27 (dt, J=9,5 Hz, 1H, H-5); 4,80 (d, J=3 Hz, 1H, <u>CH</u>-$COOCH_3$); 7,51 und 8,43 (AB, J=5 Hz, 4H, pyridinyl-H).

Beispiel 15 und Beispiel 16

(2R,3S)-1-(t-Butoxycarbonylmethyl)-1-(4-methoxyphenyl)-4-[(1S)-1-phenethyl]-2,3-oxirandicarbonsäurediamid

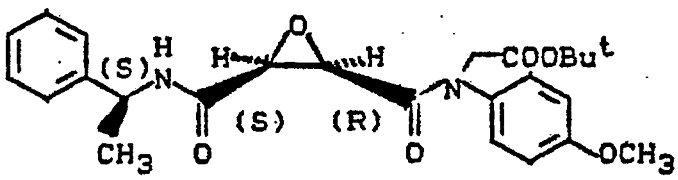

und

(2S,3R)-1-(t-Butoxycarbonylmethyl)-1-(4-methoxyphenyl)-4-[(1S)-1-phenethyl]-2,3-oxirandicarbonsäurediamid

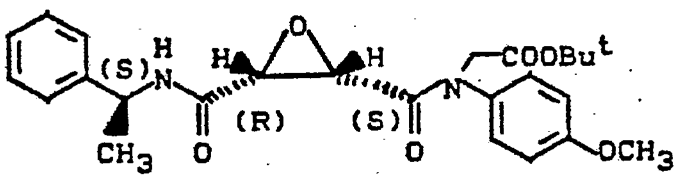

Methode A:

Zu einer gerührten Lösung von 55,8 g (0,42 mol) 2,3-Oxirandicarbonsäure [G.B. Payne et al., J. Org. Chem. 24, 54 (1959)] in 630 ml THF tropfte man innerhalb von 30 min. bei Raumtemperatur gleichzeitig eine Lösung von 100,3 g (4,2 mol) t-Butyl-N-(4-methoxyphenyl)glycinat [M. Shiozaki et al., Tetrahedron, 40, 1795 (1984)] in 100 ml THF und eine Lösung von 87,3 g (0,42 mol) Dicyclohexylcarbodiimid in 100 ml THF. Man rührte 30 min bei Raumtemperatur, gab nacheinander eine Lösung von 54,5 ml (0,42 mol) (S)-(-)-1-Phenethylamin in 100 ml THF und eine Lösung von 87,3 g (0,42 mol) Dicyclohexylcarbodiimid in 100 ml THF zu und rührte 1 h bei Raumtemperatur nach. Der Niederschlag wurde durch Filtration abgetrennt und die Filtratlösung i.Vak. eingedampft und an 2 kg Kieselgel filtriert (Toluol : Ethylacetat = 8:2 → 7:3). Man erhielt so 2 Rohfraktionen die durch erneute Chromatographie an jeweils 1 kg Kieselgel gereinigt wurden und die isomeren Titelverbindungen ergaben:
unpolares Isomer: $R_f$ = 0,38 (Toluol : Ethylacetat = 7:3)
$[\alpha]_D^{20}$ = 164,4° (c = 0,897 in $CHCl_3$).

IR(KBr) 3384, 1746, 1682, 1606, 1444, 1370 $cm^{-1}$.
$^1$H-NMR (250 MHz, $CDCl_3$): δ = 1,48 (s, $C(CH_3)_3$); 1,48 (d, J=7,5 Hz, CH$CH_3$); zusammen 13H, 3,41 und 3,46 (AB, J=5,5 Hz, 2H, Oxiran-H); 3,83 (s, 3H, $OCH_3$); 4,23 und 4,35 (AB, J=16 Hz, 2H, $CH_2COO$); 5,12 (q, J=7,5 Hz, 1H, $CHCH_3$); 6,96 (d, J=9 Hz, 2H, p-$H_3CO$-$C_6H_4$); 7,3 - 7,4 (m, 7H, Ph, p-$H_3CO$-$C_6H_4$).
$C_{25}H_{30}N_2O_6$ (454,5) Ber.: C 66.07 H 6,65 N 6,16
Gef.: C 66,3 H 6,9 N 6,2
polares Isomer: $R_f$ = 0,26 (Toluol : Ethylacetat = 7:3)
$[\alpha]_D^{20}$ = -189,4° (c = 0,405 in $CHCl_3$)

$^1$H-NMR (200 MHz, Aceton-$d_6$): δ = 1,44 (s, 9H, $C(CH_3)_3$); 1,49 (d, J=7 Hz, 3H, CH$CH_3$); 3,38 und 3,54 (AB, J=6 Hz, 2H, Oxiran-H); 3,83 (s, 3H, $OCH_3$); 3,92 und 4,36 (AB, J=17 Hz, 2H, $CH_2COO$); 5,08 (m, 1H, $CHCH_3$); 6,95 und 7,23 (d, J=9 Hz, 4H, p-$H_3CO$-$C_6H_4$); 7,38 (m, 5H, Ph).

Als Nebenprodukt dieser Reduktion erhielt man Beispiel 17:

(±)-Cis-N-(t-Butoxycarbonylmethyl)-N-(4-methoxyphenyl)-N′-cyclohexyl-N′-cyclohexylaminocarbonyl-2,3-oxirandicarbonsäurediamid

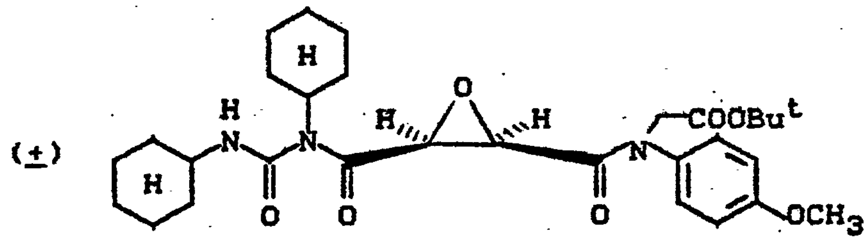

als farblose Kristalle, Schmp.: 150°C, $R_f$ = 0,40 (Toluol : Ethylacetat = 4:1).
IR(KBr) 3397, 1739, 1705, 1673, 1513 $cm^{-1}$.
$^1$H-NMR (200 MHz, DMSO): δ = 1,0 - 1,8 (m, $-CH_2-$); 1,42 (s, $C(CH_3)_3$) zusammen 29H, 2,04 (m, 2H, N-<u>CH</u>-$CH_2$); 3,50 und 3,74 (m, 2H, Oxiran-H); 3,76 (s, 3H, $OCH_3$); 4,16 (bs 2H, $CH_2COO$); 5,32 (bs, 1H, CONH); 7,00 und 7,36 (AB, J=9 Hz, 4H, p-$H_3CO-C_6H_4$).
MS (Cl, 150 eV, $NH_3$); m/e = 558 (M+H); ber. 557,70.

Methode B:

Eine Lösung von 3,34 g (9,5 mmol) (2R,3SR)-N-(t-Butoxycarbonylmethyl)-N-(4-methoxyphenyl)-3-carboxy-2,3-epoxypropionamid in 15 ml wasserfreiem THF wurde nacheinander mit 1,24 ml (9,5 mmol) (S)-(-)-Phenethylamin, 2,0 g (9,5 mmol) Dicyclohexylcarbodiimid in 3 ml THF und 1,28 g (9,5 mmol) 1-Hydroxy-1H-benztriazol versetzt. Die zunächst klare Lösung wurde 2 h bei Raumtemperatur gerührt. Der entstandene Niederschlag wurde abgesaugt und die Filtratlösung wurde i.Vak. eingedampft. Das so erhaltene Gemisch der Titelverbindungen wurde durch Chromatographie an 640 g Kieselgel (Toluol : Ethylacetat = 4:1) getrennt und ergab 646 mg (15% der Theorie) des reinen unpolaren Diastereomers, $R_f$ = 0,38 (Toluol : Ethylacetat = 7:3), und 983 mg (21% der Theorie) des reinen polaren Diastereomers, $R_f$ = 0,26 (Toluol : Ethylacetat = 7:3)
Die physikalischen Daten waren mit den nach Methode A hergestellten Diastereomeren identisch.

Beispiel 18

(3S,4S)-4-t-Butoxycarbonyl-3-[(1S)-1-hydroxy-1-{(1S)-1-phenethylaminocarbonyl}]methyl-1-(4-methoxyphenyl)-azetidin-2-on

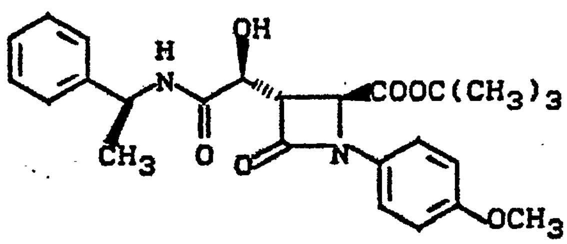

Zu einer auf 0°C gekühlten Mischung von 2,3 mmol Tetrabutylammoniumfluorid und 1 g Molekularsieb 4Å in 2 ml THF tropfte man eine Lösung von 880 mg (1,94 mmol) (2R,3S)-1-(t-Butoxycarbonylmethyl)-1-(4-methoxyphenyl)-4-[(1S)-1-phenethyl]-2,3-oxirandicarbonsäurediamid. Nach 15 min. wurde filtriert, die Filtratlösung mit Toluol versetzt und das THF i.Vak. abgedampft. Die zurückbleibende Toluol-Lösung wurde mehrmals mit Wasser gewaschen und über $MgSO_4$ getrocknet. Nach Abdampfen des Lösungsmittels i.Vak. und Chromatographie des Rückstandes an 40 g Kieselgel (Toluol : Ethylacetat = 7:3) erhielt man 285 mg (32% der Theorie) der Titelverbindung als farblose Kristalle, Schmp.: 184°C, $R_f$ = 0,19 (Toluol : Ethylacetat = 7:3),
$[\alpha]_D^{20}$ = -47,3° (c=0,8085 in $CHCl_3$).

IR(KBr) 3348 (OH), 1769 (C=O, β-Lactam), 1748 (C=O, Ester), 1658 (C=O, Amid), 1515 $cm^{-1}$.
$^1$H-NMR (250 MHz, $CDCl_3$): δ = 1,45 (s, 9H, $C(CH_3)_3$); 1,54 (d, J=7 Hz, 3H, CH<u>$CH_3$</u>); 3,60 (dd, J=7,5 Hz, 2 Hz, 1H, H-3); 3,79 (s, 3H, $OCH_3$); 4,01 (d, J=4,5 Hz, 1H, OH); 4,42 (dd, J=7,5 Hz, 4,5 Hz, 1H, HO-<u>CH</u>- CON); 4,48 (d, J=2 Hz, 1H, H-4); 5,14 (dq, J=8 Hz, 7 Hz, 1H, <u>CH</u>$CH_3$); 6,88 (d, J=9 Hz, 2H, p-$C_6H_4$); 7,2 - 7,4 (m, 7H, Ph, p-$H_3CO-C_6H_4$); 7,83 (d, J=8 Hz, 1H, CONH).

Beispiel 19

(3S,4S)-4-Carboxy-3-[(1S)-1-hydroxy-1-{(1S)-1-phenylethylaminocarbonyl}]methyl-1-(4-methoxyphenyl)azetidin-2-on

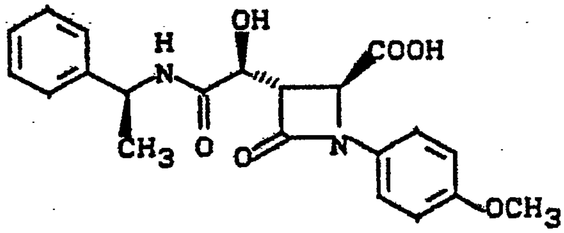

Wie für Herstellungsbeispiel 5 beschrieben erhielt man aus 150 mg (0,33 mmol) (3S,4S)-4-t-Butoxycarbonyl-3-[(1S)-1-hydroxy-1-{(1S)-1-phenethylaminocarbonyl}]methyl-1-(4-methoxyphenyl)azetidin-2-on 103 mg (78% der Theorie) der Titelverbindung als farblose Kristalle, Schmp.: 186°C, $R_f$ = 0,68 (BABA).
IR(KBr) 3320, 1750, 1658, 1630, 1515, 1248 $cm^{-1}$.
$^1$H-NMR (200 MHz, DMSO); δ = 1,44 (d, J=7 Hz, 3H, CH<u>CH</u>$_3$); 3,66 (dd, J=2 Hz, 1,5 Hz, 1H, H-3); 3,74 (s, 3H, $OCH_3$); 4,34 (m, 1H, HO-<u>CH</u>-CON); 4,60 (d, J=2 Hz, 1H, H-4); 5,00 (dq, J=9 Hz, 7 Hz, 1H, <u>CH</u>$CH_3$); 6,36 (d, J=6 Hz, 1H, OH); 6,97 und 7,25 (AB, J=9 Hz, 4H, p-$H_3CO$-$C_6H_4$); 7,38 (m, 5H, Ph); 8,36 (d, J=9 Hz, 1H, CONH); 12,80 (bs, 1H, COOH).

Beispiel 20

(3R,4R)-4-Acetoxy-3-[(1S)-1-hydroxy-1-(1S)-1-phenylethylaminocarbonyl}]methyl-1-(4-methoxyphenyl)-azetidin-2-on

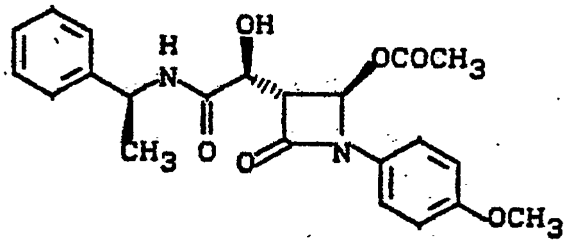

Wie für Beispiel 6 beschrieben erhielt man aus 105 mg (0,26 mmol) (3S,4S)-4-Carboxy-3-[(1S)-1-hydroxy-1-{(1S)-1-phenylethylaminocarbonyl}]methyl-1-(4-methoxyphenyl)-azetidin-2-on nach 5 min bei 60°C und Chromatographie des Rohproduktes an 6 g Kieselgel (Toluol : Ethylacetat = 3:2) 42 mg (39% der Theorie) der Titelverbindung als farblose Kristalle, Schmp.: 202°C, (Toluol), $R_f$ = 0,30 (Toluol : Ethylacetat = 1:1).
IR(KBr) 3290, 1746, 1650, 1627, 1515, 1250 $cm^{-1}$.
$^1$H-NMR (200 MHz, Aceton-$d_6$): δ = 1,51 (d, J=6,5 Hz, 3H, CH<u>CH</u>$_3$); 3,73 (dd, J=2 Hz, 1Hz, 1H, H-3); 3,78 (s, 3H,$OCH_3$); 4,48 (d, J=2 Hz, 1H, HO-<u>CH</u>-CON); 5,10 (dq, J=9 Hz, 6,5 Hz, 1H, <u>CH</u>$CH_3$); 6,63 (d, J=1 Hz, 1H, H-4); 6,95 (d, J=10 Hz, 2H, p-$C_6H_4$); 7,2-7,4 (m, 7H, Ph, p-$H_3CO$-$C_6H_4$); 7,79 (d, J=9 Hz, 1H, CONH).

Beispiel 21

(3R,4R)-4-Acetoxy-3-[(1S)-1-t-butyldimethylsilyloxy-1- (1S)-1-phenethylaminocarbonyl)]methyl-1-(4-methoxyphenyl)azetidin-2-on

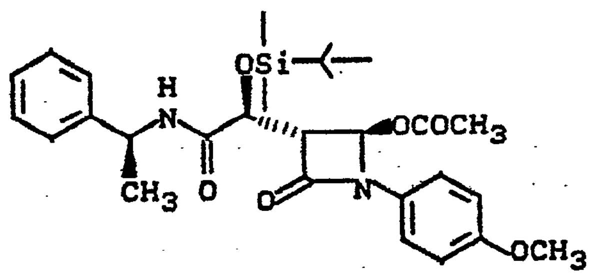

Wie für Beispiel 7 beschrieben erhielt man aus 424 mg (1,028 mmol) (3R,4R)-4-Acetoxy-3-[(1S)-1-hydroxy-1-{(1S)-1-phenylethylaminocarbonyl)]methyl-1-(4-methoxyphenyl)azetidin-2-on nach 9 Tagen bei Raumtemperatur und Chromatographie des Rohproduktes an 25 g Kieselgel (Toluol : Ethylacetat = 4:1) 409 mg (76% der Theorie) der Titelverbindung als farblose Kristalle, Schmp.: 115°C, $R_f$ = 0,60 (Toluol : Ethylacetat = 3:2).
IR($CHCl_3$) 3390, 1750, 1663, 1510, 840 $cm^{-1}$.
$^1$H-NMR (200 MHz, Aceton-$d_6$): δ = 0,03 (s, 6H, $CH_3$-Si), 0,78 (s, 9H, $CH_3$-C-Si); 1,50 (d, J=8 Hz, 3H, CH$\underline{CH_3}$); 3,70 (dd, J=2 Hz, 0,5 Hz, 1H, H-3); 3,79 (s, 3H, $OCH_3$); 4,53 (d, J=2 Hz, 1H, $\underline{CH}$-OSi); 5,05 (dq, J=9 Hz, 8 Hz, 1H, $\underline{CH}CH_3$); 6,67 (d, J=0,5 Hz, 1H, H-4); 6,95 und 7,40 (AB, J=10 Hz, 4H, p-$H_3CO$-$C_6H_4$); 7,40 (m, 5H, Ph).

Beispiel 22

(3S*,4S*)-4-t-Butoxycarbonyl-3-[(1S*)-1-{(N-Ocyclohexylaminocarbonyl}cyclohexylaminocarbonyl-1-hydroxymethyl]-1-(4-methoxyphenyl)azetidin-2-on

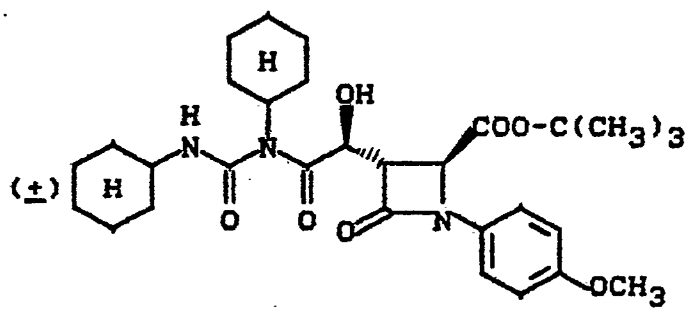

Wie für Beispiel 18 beschrieben erhielt man aus 2,23 g (4.00 mmol) cis-N-(t-Butoxycarbonylmethyl)-N-(4-methoxyphenyl)-N'-cyclohexyl-N'-cyclohexylaminocarbonyl-2,3-oxirandicarbonsäurediamid nach 2 h bei 0°C in Gegenwart von 1,7 Equivalenten Tetrabutylammoniumfluorid und Chromatographie des Rohproduktes an 150 g Kieselgel (Toluol : Ethylacetat = 4:1) die Titelverbindung, $R_f$ = 0,15 (Toluol : Ethylacetat = 9:1).
IR($CHCl_3$) 1748, 1643, 1462, 1255 $cm^{-1}$.
$^1$H-NMR (200 MHz, $CDCl_3$): δ = 1.0 - 2,5 (m, 22H, Cyclohexyl-H); 3,66 (m, 1H, H-3); 3,83 (s, 3H, $OCH_3$); 4,25 (m, 1H; H-4); 4,48 (d, J=5 Hz, 1H, HO-$\underline{CH}$-CON); 7,40 und 7,80 (AB, J=10 Hz, 4H, p-$H_3CO$-$C_6H_4$); 7,60 (d, J=9,5 Hz, 1H, CONH).

Beispiel 23

(3S*,4S*)-4-Carboxy-3-[(1S*)-1-{N-Cyclohexylaminocarbonyl}cyclohexylaminocarbonyl-1-hydroxymethyl-1-(4-methoxyphenyl)azetidin-2-on

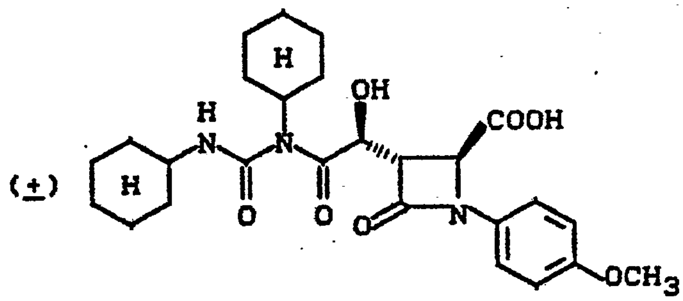

Wie für Beispiel 5 beschrieben erhielt man aus 31 mg (0,06 mmol) (3S*,4S*)-4-t-Butoxycarbonyl-3-[(1S*)-1-{N-cyclohexylaminocarbonyl}cyclohexylaminocarbonyl-1-hydroxymethyl]-1-(4-methoxyphenyl)azetidin-2-on 22 mg (79% der Theorie) der Titelverbindung als Kristalle, Schmp.: 169°C, $R_f$ = 0,08 (BABA).
IR(KBr) 3400, 1759, 1693, 1513, 1440, 1252 $cm^{-1}$.
$^1$H-NMR (200 MHz, DMSO): δ = 1,0 - 2,2 (m, 22H, Cyclohexyl-H); 3,80 (m, H-3); 3,82 (s, $OCH_3$) zusammen 4H, 4,85 (m, H-4, HO-CH-CON); 6,96 und 7,60 (AB, J=9,5 Hz, 4H, p-$H_3CO$-$C_6H_4$).

Beispiel 24

(3R,4R)-4-t-Butoxycarbonyl-3-[(1R)-1-hydroxy-1-{(1S)-1-phenylethylaminocarbonyl}]methyl-1-(4-methoxyphenyl)-azetidin-2-on

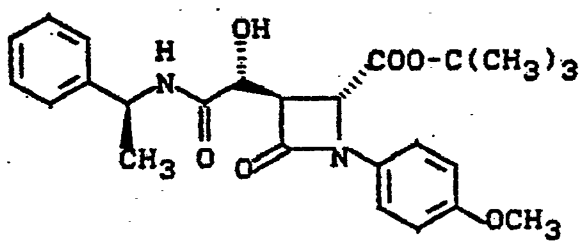

Wie für Beispiel 18 beschrieben erhielt man aus 332 mg (0,73 mmol) (2S,3R)-1-[(t-Butoxycarbonyl)-1-(4-methoxyphenyl)]methyl-4-[(1S)-1-phenylethyl]-2,3-oxiranedicarbonsäurediamid und nach Chromatographie des Rohproduktes an 28 g Kieselgel (Toluol : Ethylacetat = 3:2) 120 mg (36% der Theorie) der Titelverbindung als farblose Kristalle, Schmp.: 131°C, $R_f$ = 0,29 (Toluol : Ethylacetat = 1:1).
IR($CHCl_3$) 3340, 1750, 1665, 1510 $cm^{-1}$.
$^1$H-NMR (200 MHz, $CDCl_3$): δ = 1,45 (s, 9H, $C(CH_3)_3$), 1,59 (d, J=7 Hz, 3H, $CHCH_3$); 3,73 (dd, J=7,5 Hz, 0,5 Hz, 1H,H-3); 3,80 (s, 3H, $OCH_3$); 4,49 (m, 2H, HO-CH-CON, H-4); 5,16 (dq, J=8,5 Hz, 7 Hz, 1H, $CHCH_3$); 6,86 und 7,28 (AB, J=9,5 Hz, p-$H_3CO$-$C_6H_4$); 7,38 (m, Ph) zusammen 9H, 7,80 (d, J=8,5 Hz, 1H, CONH).

Beispiel 25

(3R,4R)-4-Carboxy-3-[(1R)-1-hydroxy-1-{(1S)-1-phenylethylaminocarbonyl}]methyl-1-(4-methoxy-phenyl)azetidin-2-on

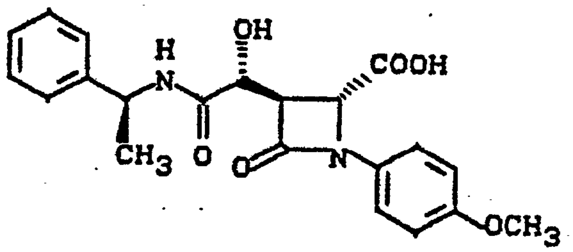

Wie für Beispiel 5 beschrieben erhielt man aus 105 mg (0,23 mmol) (3R,4R)-4-t-Butoxycarbonyl-3-[(1R)-1-hydroxy-1-{(1S)-1-phenylethylaminocarbonyl)]methyl-1-(4-methoxyphenyl)-azetidin-2-on 84 mg(91 % der Theorie) der Titelverbindung als farblosen Feststoff, Schmp.: 161°C, $R_f$ = 0,61 (BABA).
IR(KBr) 1746, 1654, 1512, 1249 $cm^{-1}$.
$^1$H-NMR (200 MHz, Aceton-$d_6$): δ = 1,56 (d, J=7 Hz, 3H, CH<u>CH$_3$</u>); 3,81 (s, 3H, $OCH_3$); 3,90 (dd, J=2 Hz, 2 Hz, 1H, H-3); 4,66 (m, 2H, HO-<u>CH</u>-CON, H-4); 5,18 (dq, J=8 Hz, 7 Hz, 1H, <u>CH</u>CH$_3$); 6,98 (d, J=9,5 Hz, 2H, p-$C_6H_4$); 7,3 - 7,5 (m, 7H, Ph, p-$H_3CO$-$C_6H_4$); 8,00 (d, J=8 Hz, 1H, CONH).

Beispiel 26

(3S,4S)-4-Acetoxy-3-[(1R)-1-hydroxy-1-{(1S)-1-phenylethylaminocarbonyl}]methyl-1-(methoxyphenyl)azetidin-2-on

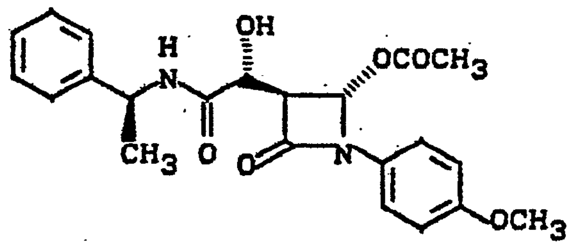

Wie für Beispiel 6 beschrieben erhielt man aus 966 mg (2,43 mmol) (3R,4R)-4-t-Butoxycarbonyl-3-[(1R)-1-hydroxy-1-{(1S)-1-phenylethylaminocarbonyl)]methyl-1-(4-methoxyphenyl)azetidin-2-on nach 5 min bei 60°C und Chromatographie des Rohproduktes an 60 g Kieselgel (Toluol : Ethylacetat = 1:1) 316 mg (32 % der Theorie) der Titelverbindung als farblosen Feststoff, Schmp.: 197°C (Aceton), $R_f$ = 0,18 (Toluol : Ethylacetat = 3:2).
IR(KBr) 3300 (OH), 1753 (C=O, β-Lactam, Ester), 1652 (C=O , Amid), 1515 $cm^{-1}$.
$^1$H-NMR (200 MHz, Aceton-$d_6$): δ = 1,49 (d, J=7,5 Hz, 3H, CH<u>CH$_3$</u>); 2,03 (s, unter Aceton, $OCOCH_3$); 3,76 (s, $OCH_3$); 3,76 (m, OH, H-3) zusammen 5H); 4,50 (d, J=2 Hz, 1H, HO-<u>CH</u>-CON); 5,05 (dq, J=9 Hz, 7,5 Hz, 1H, <u>CH</u>-CH$_3$); 6,61 (d, J=1 Hz, 1H, H-4); 6,91 (d, J=9,5 Hz, 2H, p-$C_6H_4$); 7,2 = 7,4 (m, 7H, Ph, p-$H_3CO$-$C_6H_4$); 7,79 (d, J=9 Hz, 1H, CONH).

Beispiel 27

(3S,4S)-4-Acetoxy-3-[(1R)-1-t-butyldimethylsilyloxy-1-{(1S)-1-phenylethylaminocarbonyl)]methyl-1-(4-methoxyphenyl)azetidin-2-on

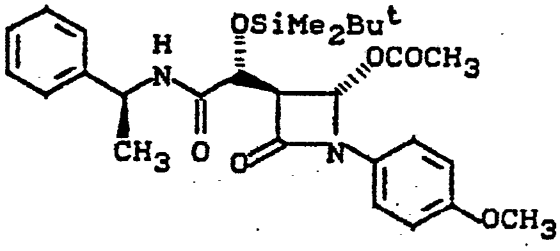

Wie für Beispiel 7 beschrieben erhielt man aus 267 mg (0,65 mmol) (3S,4S)-4-Acetoxy-3-[(1R)-1-hydroxy-1-{(1S)-1-phenylethylaminocarbonyl}]methyl-1-(4-methoxyphenyl)-azetidin-2-on nach 7 Tagen bei Raumtemperatur und Chromatographie des Rohproduktes an 15 g Kieselgel (Toluol : Ethylacetat = 4:1) 282 mg (83% der Theorie) der Titelverbindung als farblose Kristalle, Schmp.: 120°C, $R_f$ = 0,27 (Toluol : Ethylacetat = 4:1).
IR(KBr) 3426, 1767 (C=O, β-Lactam), 1748 (C=O, Ester), 1681 (C=O, Amid), 1511, 1399, 1264 $cm^{-1}$.
$^1$H-NMR (250 MHz, $CDCl_3$): δ = 0,03 (s, 3H, $CH_3Si$); 0,15 (s, 3H, $CH_3Si$); 0,83 (s, 9H, $CH_3$-C-Si); 1,53 (d, J=8 Hz, 3H, $\underline{CH_3}CH$); 1,98 (s, 3H, $OCOCH_3$); 3,78 (s, 3H, $OCH_3$); 3,81 (dd, J=2 Hz, 0,5 Hz, 1H, H-3); 4,59 (d, J=2 Hz, 1H, $\underline{CH}$-OSi); 5,08 (dq, J=8 Hz, 8 Hz, 1H, $CH_3\underline{CH}$); 6,51 (d, J=0,5 Hz, 1H, H-4); 6,84 (d, J=10 Hz, 2H, p-$C_6H_4$); 6,93 (d, J=8 Hz, 1H, CONH); 7,3-7,4 (m, 7H, Ph, p-$H_3CO$-$C_6H_4$).

Beispiel 28

(3S,4S)-4-Acetoxy-3-[(1R)-1-t-butyldimethylsilyloxy-1-{(1S)-1-phenylethylaminocarbonyl)]methyl-azetidin-2-on

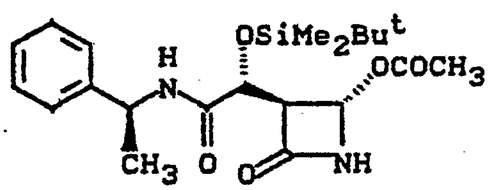

Wie für Beispiel 8 beschrieben erhielt man aus 138 mg (0,26 mmol) (3S,4S)-4-Acetoxy-3-[(1R)-1-t-butyldimethylsilyloxy-1-{(1S)-1-phenylethylaminocarbonyl)]methyl-1-(4-methoxyphenyl)azetidin-2-on nach Chromatographie des Rohprodukts an 18 g Kieselgel (Toluol : Ethylacetat = 7:3) 76 mg (69% der Theorie) der Titelverbindung als farblose Kristalle, Schmp.: 126°C (n-Heptan), $R_f$ = 0,35 (Toluol: Ethylacetat = 3:2);
$[\alpha]_D^{20}$ = -9,3° (c : 0,925, $CHCl_3$).

IR(KBr) 3420, 1786 (C=O, β-Lactam), 1750 (C=O, Ester), 1680 (C=O, Amid), 1526, 1228 $cm^{-1}$.
$^1$H-NMR (250 MHz, $CDC1_3$): δ = 0,14 und 0,16 (s, 6H, $CH_3$-Si); 0,95 (s, 9H, $CH_3$-C-Si); 1,51 (d, J=8 Hz, 3H, $\underline{CH_3}CH$); 1,96 (s, 3H, $OCOCH_3$); 3,75 (dd, J=2 Hz, 1Hz, 1H, H-3); 4,51 (d, J=2 Hz, 1H, CH-OSi); 5,12 (dq, J=9,5 Hz, 8 Hz, 1H, $CH_3\underline{CH}$); 5,63 (d, J=1 Hz, 1H, H-4); 5,43 (bs, 1H, Azetidinon NH); 6,91 (d, J=9,5 Hz, 1H, CONH, 7,2 - 7,3 (m, 5H, Ph).

Beispiel 29

(2RS,3SR)-N-(t-Butoxycarbonylmethyl)-N-(4-methoxyphenyl)-3-carboxy-2,3-epoxypropionamid

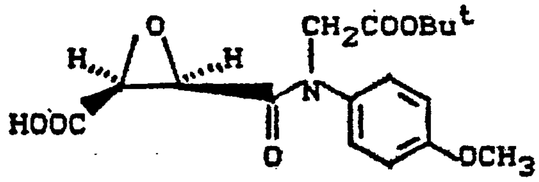

Eine Lösung von 230 mg (10,0 mgat.) Natrium in 10 ml wasserfreiem Methanol wurde bei Raumtemperatur zu einer Lösung von (2RS,3SR)-N_(t-Butoxycarbonylmethyl)-N-(4-methoxyphenyl)-3-methoxycarbonyl-2,3-epoxypropionamid gegeben. Zu dieser Lösung gab man genau 180 µl (10 mmol - 1 equiv.) Wasser und rührte 15 h bei Raumtemperatur nach. Anschließend wurde die Reaktionsmischung i.Vak. eingedampft, in 15 ml Wasser gelöst, mit Ether gewaschen, mit 10 ml Ethylacetat überschichtet und mit 2,5 N $H_2SO_4$ unter Kühlen auf pH 2,5 gestellt. Man extrahierte 4 mal mit Ethylacetat, trocknete die organische Phase über $MgSO_4$ und dampfte das Lösungsmittel i.Vak. ab. Man erhielt 3,44 g (98% der Theorie) der Titelverbindung als farblosen Feststoff, $R_f$ = 0,36 (BABA).

$^1$H-NMR (200 MHz, DMSO): δ = 1,39 (s, 9H, $C(CH_3)_3$), 3,52 und 3,59 (AB, J=5 Hz, 2H, H-2, H-3); 3,78 (s, 3H, $OCH_3$; 4,22 (bs, 2H, $NCH_2COO$); 7,02 und 7,35 (AB, J=9,5 Hz, 4H, p-$H_3CO$-$C_6H_4$); 12,87 (bs, 1H, COOH).

Beispiel 30

(3R,4R)-4-Acetoxy-3-[(1S)-1-tert-butyldimethylsilyloxy-1-{(1S)-1-phenylethylaminocarbonyl)]methyl-azetidin-2-on

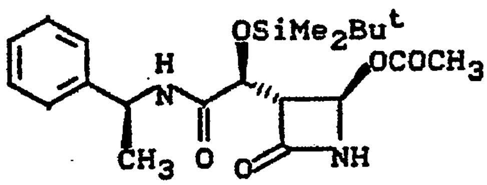

Wie für Beispel 8 beschrieben erhielt man aus 590 mg (1,12 mmol) (3R,4R)-4-Acetoxy-3-[(1S)-tert-butyldimethylsilyloxy-1-{(1S)-1-phenylethylaminocarbonyl)] methyl-1-(4-methoxyphenyl)azetidin-2-on und Chromatographie des Rohproduktes an 60 g Kieselgel (Toluol : Ethylacetat = 3:2) 342 mg (73% der Theorie) der Titelverbindung als farblosen Hartschaum, $R_f$ = 0,24 (Toluol : Ethylacetat = 3:2).

IR ($CHCl_3$): 3390, 1782 (C=O, β-Lactam, 1739 (C=O, Ester), 1662 (C=O, Amid), 1500 $cm^{-1}$.

$^1$H-NMR (250 MHz, Aceton-$d_6$): δ = 0,01 und 0,07 (s, 6H, $CH_3Si$); 0,85 (s, 9H, $CH_3$-C-Si); 1,45 (d, J=7,5 Hz, 3H, $\underline{CH_3}CH$); 2,04 (s, 3H, $OCOCH_3$); 3,52 (dd, J=2 Hz, 1Hz, 1H, H-3); 4,41 (d, J=2 Hz, 1H, CH-O-Si); 5,07 (dq, J=8 Hz, 7,5 Hz, 1H, $CH_3\underline{CH}$); 6,03 (d, J=1 Hz, 1H, H-4); 7,2 = 7,4 (m, 5H, Ph); 8,12 (bs, 1H, NH).

Beispiel 31

(3S,4R)-3-[(1S)-1-tert-Butyldimethylsilyloxy-1-{(1S)-1-phenylethylaminocarbonyl}]methyl-4-[3-p-nitrobenzyloxycarbonyl-3-diazo-2-oxopropyl]azetidin-2-on

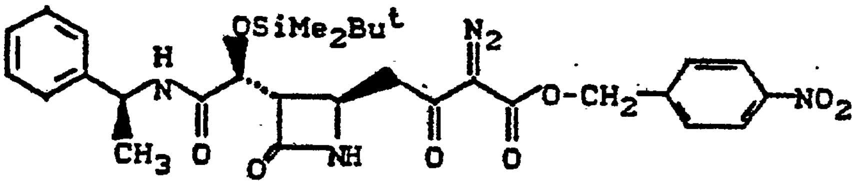

Wie für Beispiel 10 beschrieben erhielt man aus 210 mg (0,5 mmol) (3R,4R)-4-Acetoxy-3-[(1S)-1-tert-butyldimethylsilyloxy-1-{(1S)-1-phenylethylaminocarbonyl)]-methyl-azetidin-2-on nach 40 Min. bei

Raumtemperatur und Chromatographie des Rohproduktes an 15 g Kieselgel (Toluol : Ethylacetat = 1:1) 121 mg (38% der Theorie) der Titelverbindung als hellen Feststoff, $R_f$ = 0,16 (Toluol : Ethylacetat = 3:2).
IR (KBr) 2140 ($N_2$), 1758 (C=O, β-Lactam), 1717 (C=O, Ester), 1655 (C=O, Amid).
$^1$H-NMR (200 MHz, Aceton-$d_6$): δ = 0,16 und 0,21 (s, 6H, $CH_3Si$); 0,97 (s, 9H, $CH_3$-C-Si); 1,52 (d, J=7,5 Hz, 3H, <u>$CH_3$</u>CH); 2,61 (dd, J=18 Hz, 3,5 Hz, 1H, H-1); 2,91 (dd, J=18 Hz, 10 Hz, 1H, H-1); 3,20 (dd, J=2,5 Hz, 2 Hz, 1H, H-3); 4,03 (ddd, J=10 Hz, 3,5 Hz, 2 Hz, 1H, H-4); 4,47 (d, J=2,5 Hz, 1H, CH-O-Si); 5,06 (dq, J=7,5 Hz, 7,5 Hz, 1H, $CH_3$<u>CH</u>); 5,47 (s, $COOCH_2$); 5,5 (bs, NH) zusammen 3H, 7,2 - 7,5 (m, 6H, Ph, NH); 7,75 und 8,35 (AB, J=10 Hz, 4H, p-$NO_2$-$C_6H_4$).

<u>Beispiel 32</u>

(3S,4R)-3-[(1S)-1-Hydroxy-1-(1S)-1-phenylethylaminocarbonyl}]methyl-4-[3-p-nitrobenzyloxycarbonyl-3-diazo-2-oxopropyl]azetidin-2-on

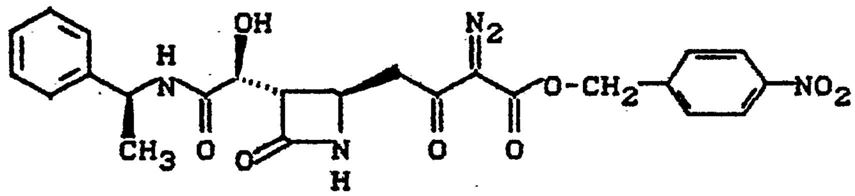

Wie für Beispiel 11 beschrieben erhielt man aus 115 mg (0,18 mmol) (3S,4R)-3-[(1S)-1-tert-Butyldimethylsilyloxy-1{(1S)-1-phenylethylaminocarbonyl)]methyl-4-[3-p-nitrobenzyloxycarbonyl-3-diazo-2-oxopropyl]azetidin-2-on 86 mg (92% der Theorie) der Titelverbindung als hellen Feststoff, $R_f$ = 0,28 (Toluol : Ethylacetat = 1:9).
IR (KBr) 3350 (OH), 2138 ($N_2$), 1758 (C=O, β-Lactam), 1725 (C=O, Ester); 1657 (C=O, Amid); 1520 ($NO_2$, asym.), 1350 $cm^{-1}$ ($NO_2$, sym.).
$^1$H-NMR (200 MHz, Aceton-$d_6$): δ = 1,49 (d, J=8 Hz, 3H, <u>$CH_3$</u>CH); 2,59 (dd, J=17,5 Hz, 3 Hz, 1H, H-1); 2,87 (dd, J=17,5 Hz, 9,5 Hz, 1H, H-1); 3,18 (dd, J=2 Hz, 2Hz, 1H, H-3); 4,0 (m, 1H, H-4); 4,44 (d, J=2 Hz, 1H, CO-CH-O); 5,10 (dq, J=8 Hz, 7,5 Hz, 1H, $CH_3$<u>CH</u>); 5,45 (s, 2H, $COOCH_2$); 5,7 (bs, 1H, NH); 7,25 - 7,45 (m, 5H, Ph), 7,78 und 8,29 (AB, J=9,5 Hz, 4H, p-$NO_2$-$C_6H_4$).

## Patentansprüche

1. Verbindungen der allgemeinen Formel (I)

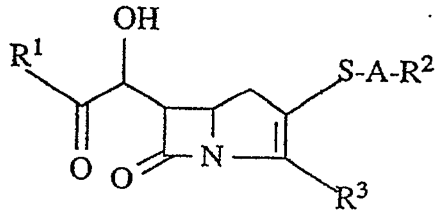

(I)

in welcher
$R^1$ für eine Gruppe der Formel $-OCH_3$, $-OC_2H_5$, $-OC_3H_7$, $-OCH(CH_3)_2$, $-OC_4H_9$, $-OC(CH_3)_3$, $-O-CH_2-CH=CH_2$, $-O-CH_2-C_6H_5$

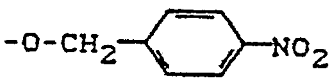, 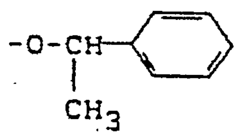 (R-Form, S-Form oder RS-Gemisch)

$-NH_2$, $-NHCH_3$, $-N(CH_3)_2$, $-NHC_2H_5$, $-N(C_2H_5)_2$, $-NHC_3H_7$, $-N(C_3H_7)_2$, $-NHCH(CH_3)_2$, 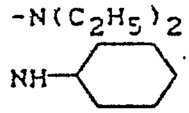,

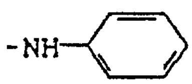, $NH-CH_2-CH=CH_2$, 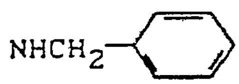,

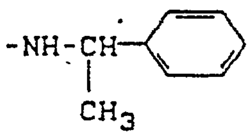 (R-Form, S-Form oder RS-Gemisch)

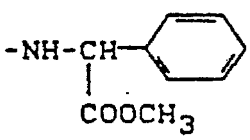 (R-Form, S-Form oder RS-Gemisch)

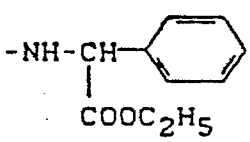 (R-Form, S-Form oder RS-Gemisch)

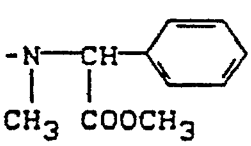 (R-Form, S-Form oder RS-Gemisch)

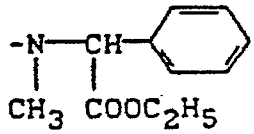 (R-Form, S-Form oder RS-Gemisch)

$-NH-CO-NH_2$, $-NH-CO-NHCH_3$, $-NH-CO-N(CH_3)_2$,

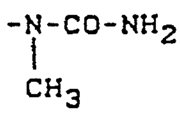, 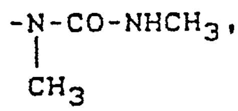, 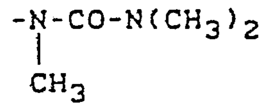,

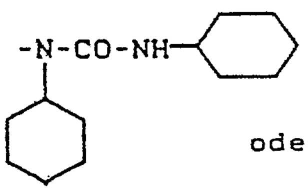 oder 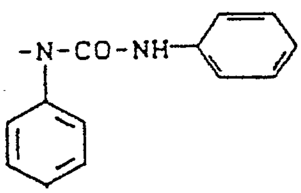 steht,

– die Gruppe AR² für einen Rest der Formel

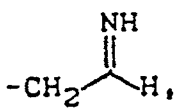, 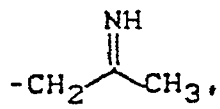, 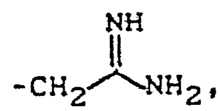, 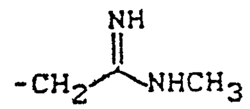,

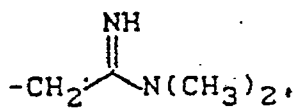, 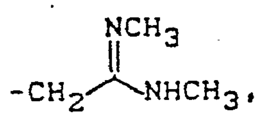, 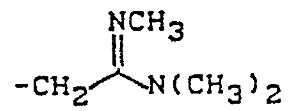,

$-CH_2-CH_2-NH_2$, $-CH_2-CH_2-NHCH_3$, $-CH_2-CH_2-N(CH_3)_2$,

$-CH_2-CH_2-NHCOCH_3$, 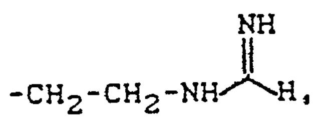,

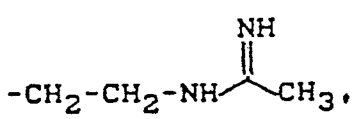, 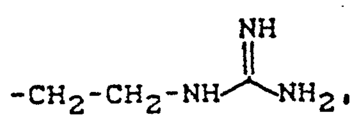, $-CH_2-COOH$,

$-CH_2-COOCH_3$, 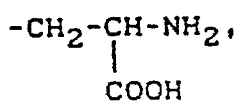, 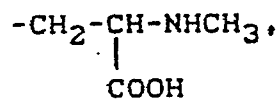

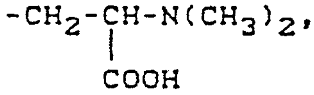 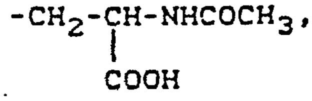 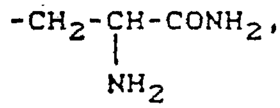

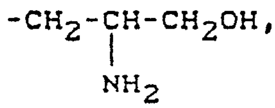 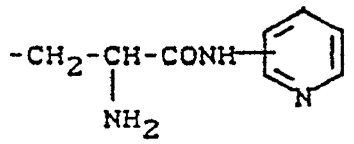 $-CH=CH-NH_2$,

$-CH-CH-NHCH_3$, $-CH=CH-N(CH_3)_2$, $-CH=CH-NHCOCH_3$,

$-CH=CH-CH_2-NH_2$, 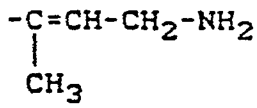

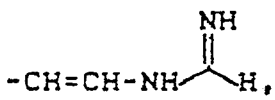 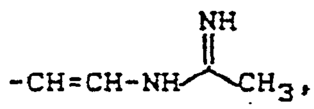 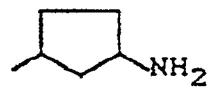

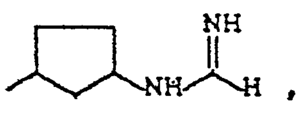 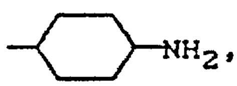 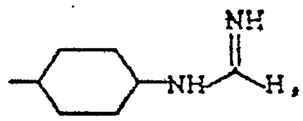

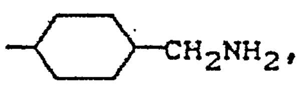 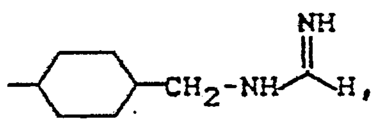 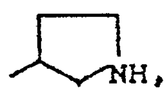

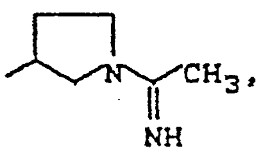 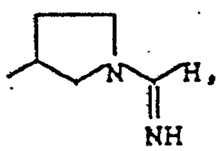 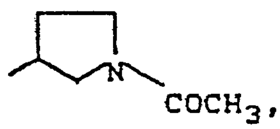 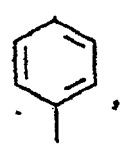

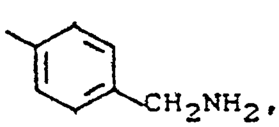 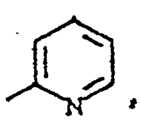 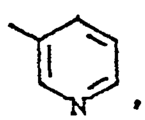 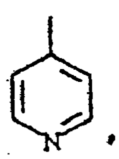 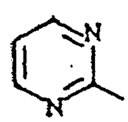

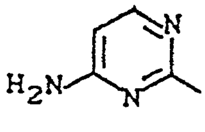 , 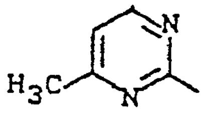 , 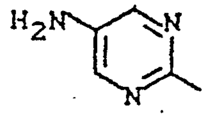 , 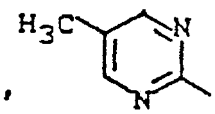 ,

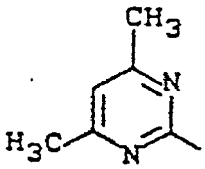 , 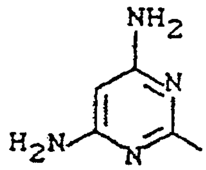 , 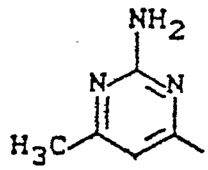 ,

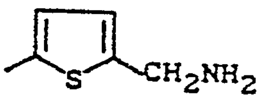 , 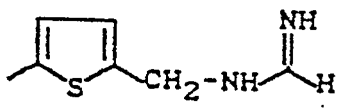 , 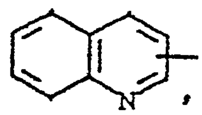 ,

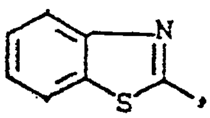 , 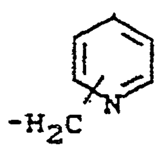 , 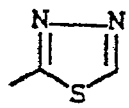 , 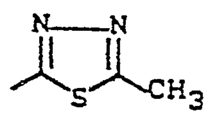 ,

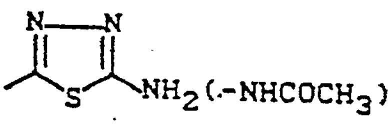 , 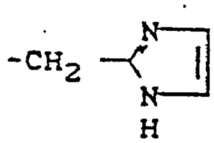 , 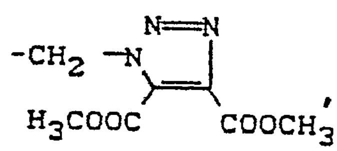 ,

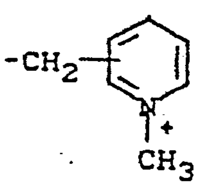 , 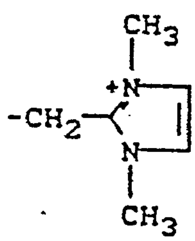 , 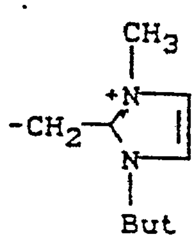 ,

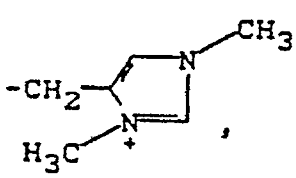 , 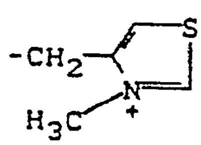 , 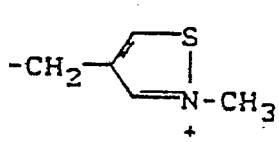

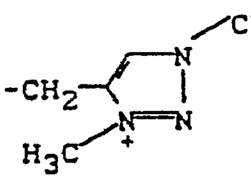 , 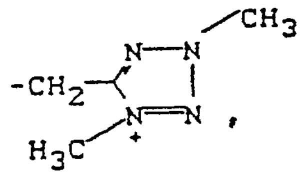 ,

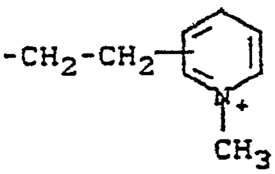 , 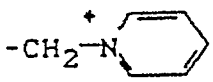 , 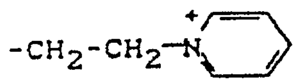

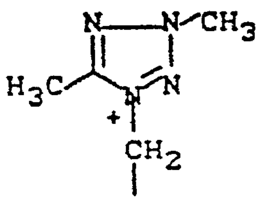 , 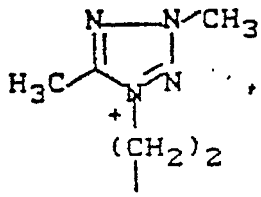 , 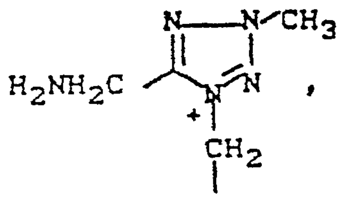 ,

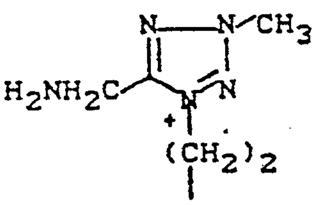 , 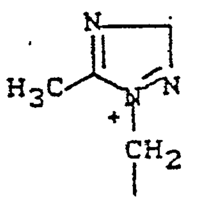 , 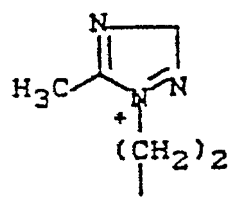

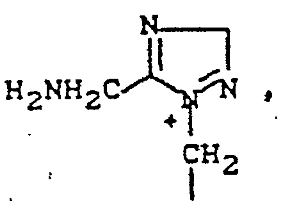 , 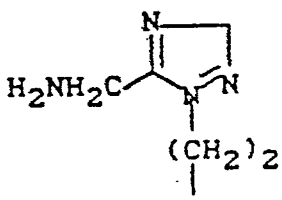 , 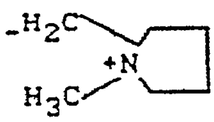 ,

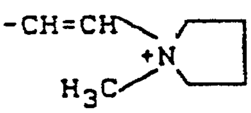 , 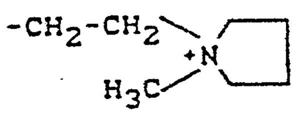 , 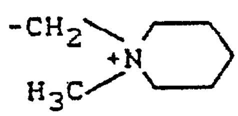 ,

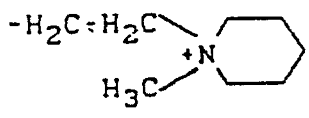 , 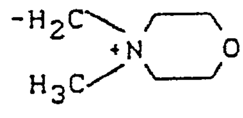 , 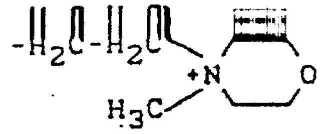 ,

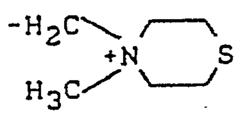 , 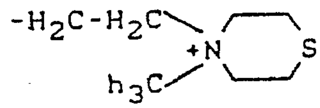 , 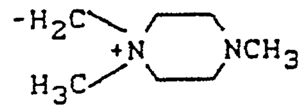 ,

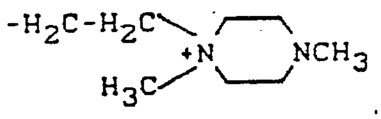 , 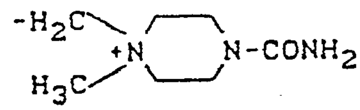 ,

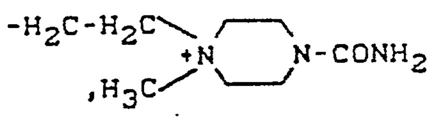 , 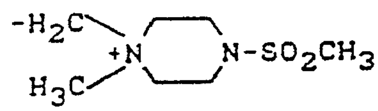 ,

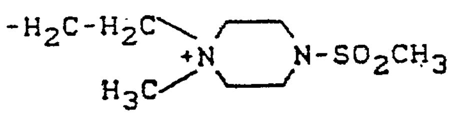 , 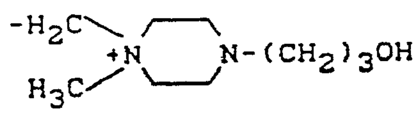 ,

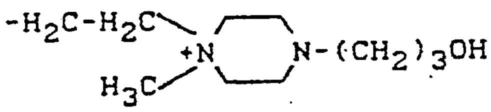 steht,

und
$R^3$ für eine Carboxygruppe $COOR^{20}$ steht, worin
$R^{20}$ Wasserstoff, Allyl, 4-Nitrobenzyl oder 4-Methoxybenzyl bedeutet oder
für $COO^-$ steht, falls die Gruppe $AR^2$ einen positiv geladenen Rest darstellt, und deren Salze.

2. Verfahren zur Herstellung von substituierten 6-Hydroxymethyl-carbapenemantibiotika der allgemeinen Formel (I)

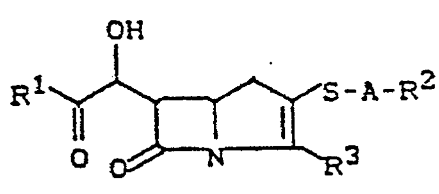 (I)

in welcher $R^1$, $R^3$ und $AR^2$ die in Anspruch 1 angegebene Bedeutung haben, dadurch gekennzeichnet, daß man Ketoester der allgemeinen Formel (II)

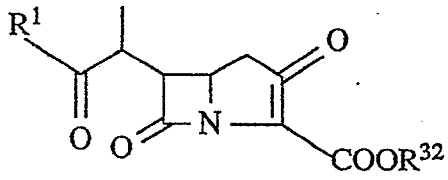

(II)

worin
$R^1$ die oben angegebene Bedeutung hat und
$R^{32}$ Wasserstoff, Carboxyschutzgruppe oder einen in vivo abspaltbaren Esterrest bedeutet, aber nicht für Wasserstoff stehen darf,
und wobei die Gruppe $COOR^{32}$ nicht für ein Carboxylatanion stehen darf,
und
Thiole der allgemeinen Formel (III) $H\text{-}S\text{-}A\text{-}R^2$
in welchen
A und $R^2$ die angegebene Bedeutung haben in inerten Lösungsmitteln, in Gegenwart von Basen mit einem Hilfsstoff umsetzt, gegebenenfalls Schutzgruppen abspaltet und gegebenenfalls die gewünschten Salze herstellt oder die Salze in die freien Verbindungen überführt.

3. Substituierte 6-Hydroxymethyl-carbapenemanitbiotika der allgemeinen Formel (I)

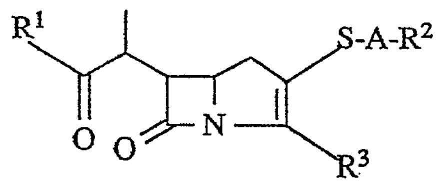

(I)

in welcher
$R^1$, $R^3$ und $AR^2$ die in Anspruch 1 angegebene Bedeutung haben, und deren Salze zur Verwendung in einem Verfahren zur therapeutischen Behandlung des menschlichen oder tierischen Körpers.

4. Arzneimittel, enthaltend substituierte 6-Hydroxymethyl-carbapenemantibiotika der allgemeinen Formel (I)

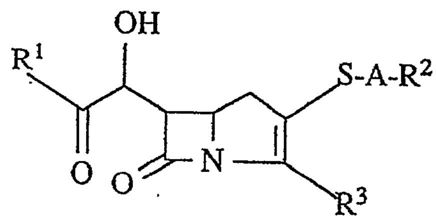

(I)

gemäß Anspruch 1.

5. Verwendung von substituierten 6-Hydroxymethylcarbapenemantibiotika der allgmeinen Formel (I) gemäß Anspruch 1 zur Herstellung von Arzneimitteln.

6. Tierfutter und Tierfutterzusätze, enthaltend substituierte 6-Hydroxymethylcarbpenemantibiotika der Formel (I) nach Anspruch 1.

## Claims

1. Compounds of the general formula (I)

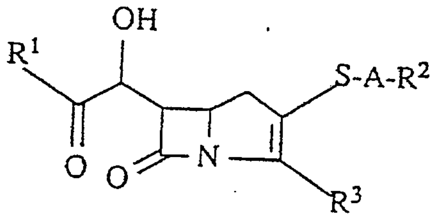

(I)

in which
$R^1$ represents a group of the formula $-OCH_3$, $-OC_2H_5$, $-OC_3H_7$, $-OCH(CH_3)_2$, $-OC_4H_9$, $-OC(CH_3)_3$, $-O-CH_2-CH=CH_2$, $-O-CH_2-C_6H_5$,

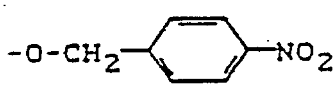, 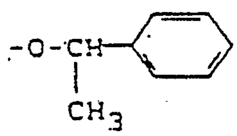 (R form, S form or RS mixture)

$-NH_2$, $-NHCH_3$, $-N(CH_3)_2$, $-NHC_2H_5$, $-N(C_2H_5)_2$, $-NHC_3H_7$, $-N(C_3H_7)_2$, $-NHCH(CH_3)_2$, 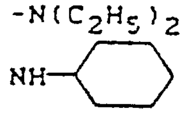,

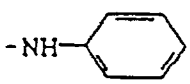, $NH-CH_2-CH=CH_2$, 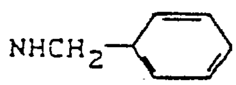,

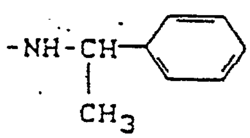 (R form, S form or RS mixture),

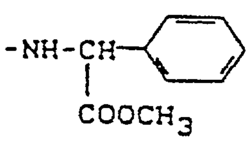 (R form, S form or RS mixture)

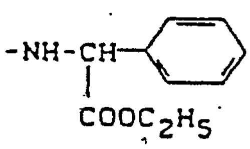 (R form, S form or RS mixture)

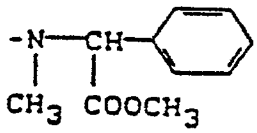 (R form, S form or RS mixture)

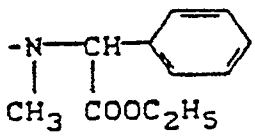 (R form, S form or RS mixture)

$-NH-CO-NH_2$, $-NH-CO-NHCH_3$, $-NH-CO-N(CH_3)_2$,

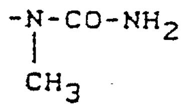, 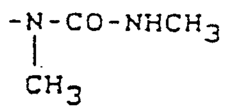, 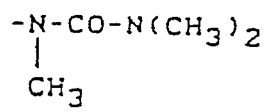,

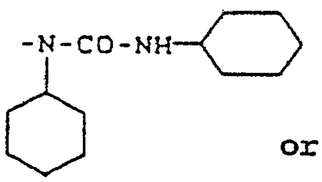 or 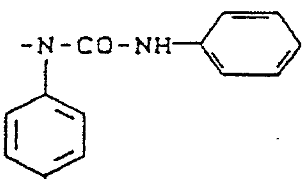

the AR² group represents a radical of the formula

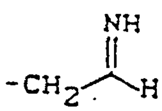, 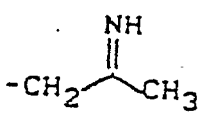, 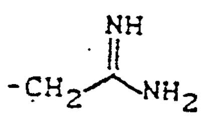, 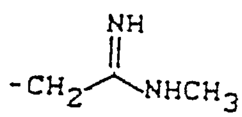,

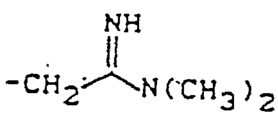, 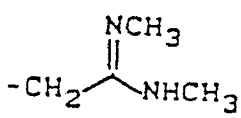, 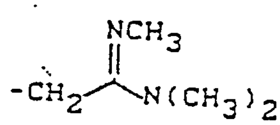,

$-CH_2-CH_2-NH_2$, $-CH_2-CH_2-NHCH_3$, $-CH_2-CH_2-N(CH_3)_2$,

$-CH_2-CH_2-NHCOCH_3$, 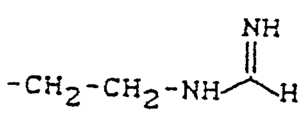,

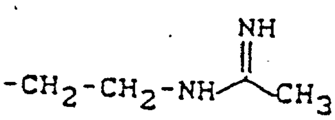, 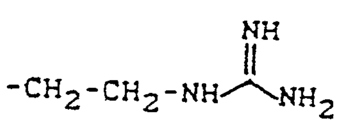, $-CH_2-COOH$,

$-CH_2-COOCH_3$, 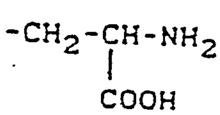, 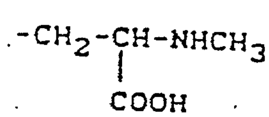

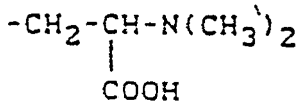, 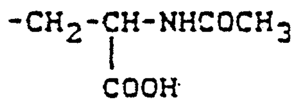, 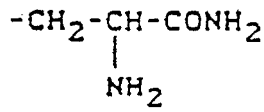,

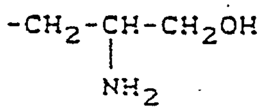, 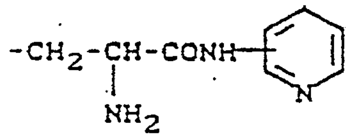, $-CH=CH-NH_2$,

$-CH-CH-NHCH_3$, $-CH=CH-N(CH_3)_2$, $-CH=CH-NHCOCH_3$,

$-CH=CH-CH_2-NH_2$, 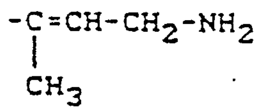

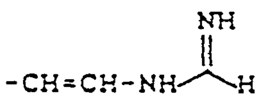, 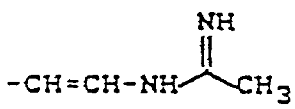, 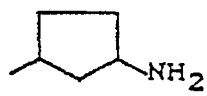,

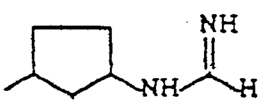, 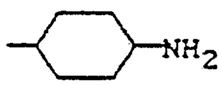, 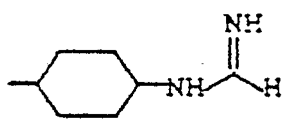,

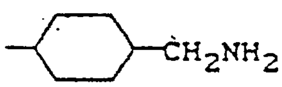, 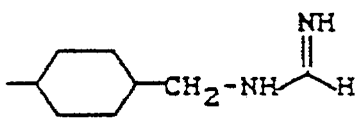, 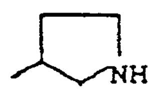,

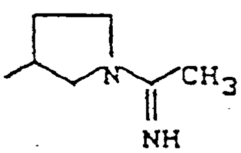, 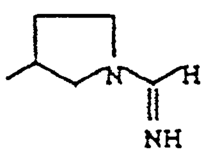, 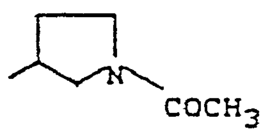, 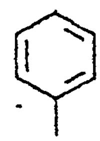,

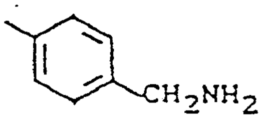, 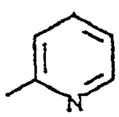, 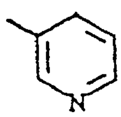, 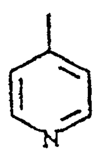, 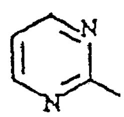

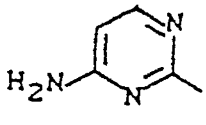 , 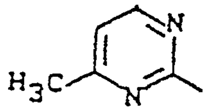 , 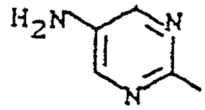 , 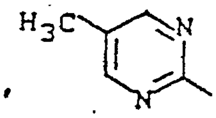 ,

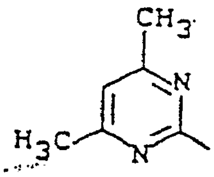 , 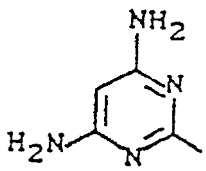 , 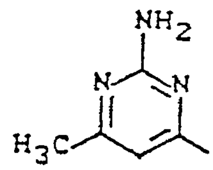 ,

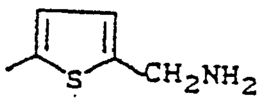 , 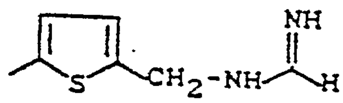 , 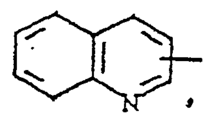 ,

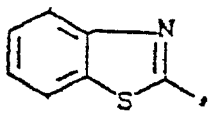 , 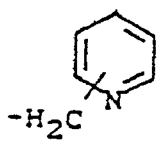 , 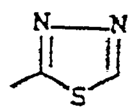 , 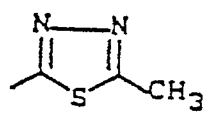 ,

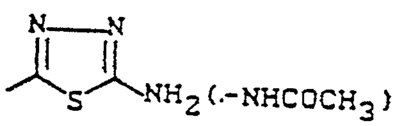 , 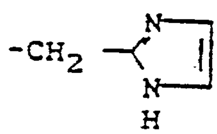 , 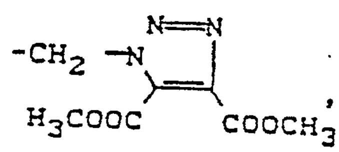 ,

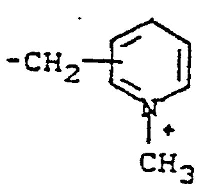 , 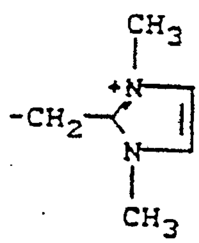 , 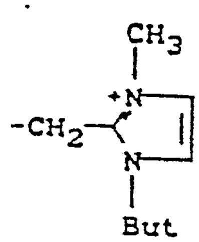 ,

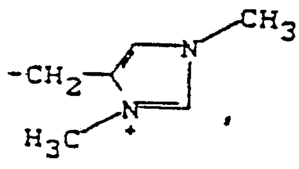 , 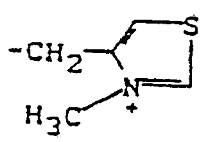 , 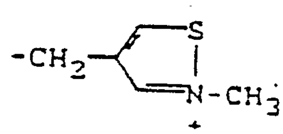

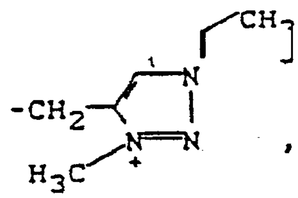 , 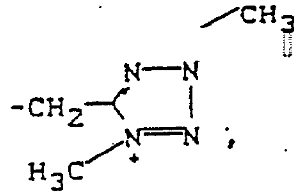 ;

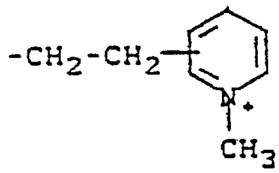 , 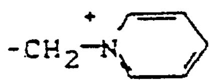 , 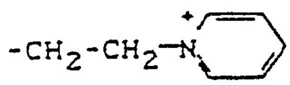 ,

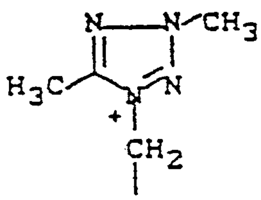 , 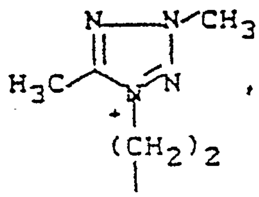 , 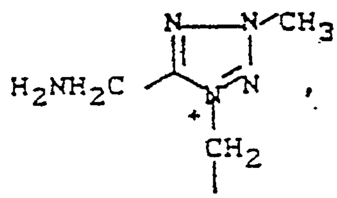 ,

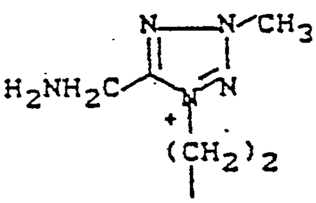 , 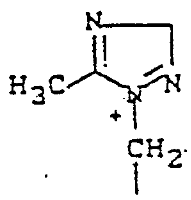 , 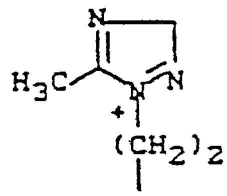

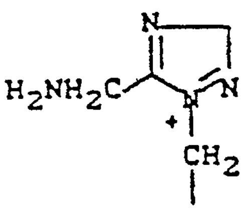 , 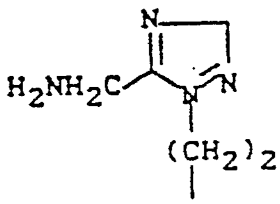 , 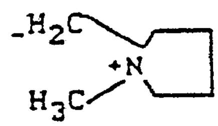 ,

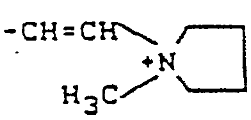 , 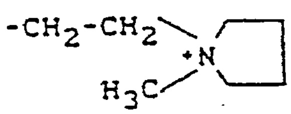 , 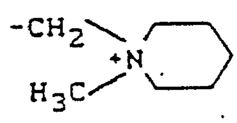 ,

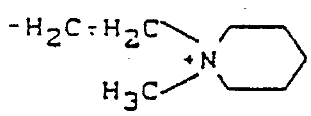 , 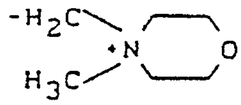 , 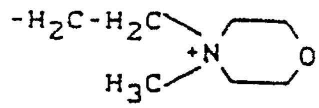 ,

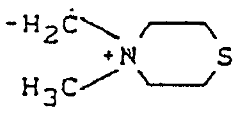 , 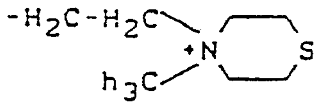 , 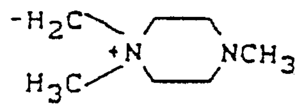 ,

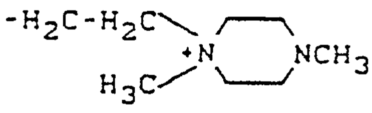 , 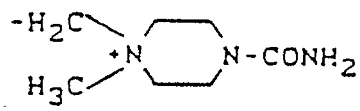 ,

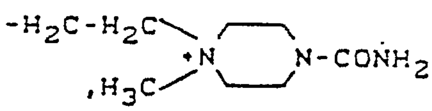 , 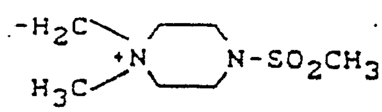 ,

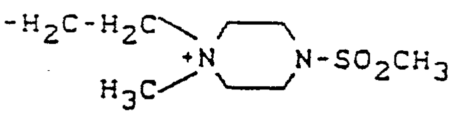 , 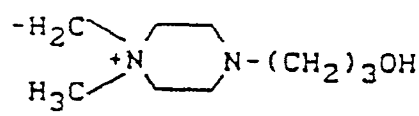 ,

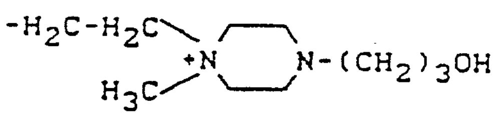

and
$R^3$ represents a carboxyl group $COOR^{20}$,
in which
$R^{20}$ denotes hydrogen, allyl, 4-nitrobenzyl or 4-methoxybenzyl, or
represents $COO^-$ if the $AR^2$ group represents a positively charged radical,
and the salts thereof.

2. Process for the preparation of substituted 6-hydroxymethyl-carbapenem antibiotics of the general formula (I)

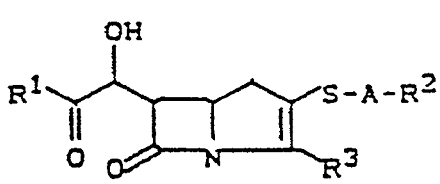 (I)

in which $R^1$, $R^3$ and $AR^2$ have the meaning indicated in Claim 1, characterized in that keto esters of the general formula (II)

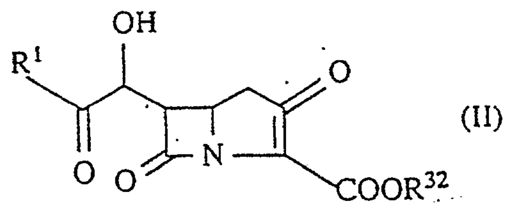

(II)

in which
$R^1$ has the abovementioned meaning
and
$R^{32}$ denotes hydrogen, a carboxyl-protecting group or an ester radical which can be cleaved off in vivo, but must not represent hydrogen,
and where the $COOR^{32}$ group may not represent a carboxylate anion,
and
thiols of the general formula (III)

$H-S-A-R^2$

in which
A and $R^2$ have the meaning indicated, are reacted in inert solvents, in the presence of bases with an auxiliary substance, protecting groups are cleaved off if appropriate, and, if appropriate, the desired salts are prepared or the salts are converted into the free compounds.

3. Substituted 6-hydroxymethyl-carbapenem antibiotics of the general formula (I)

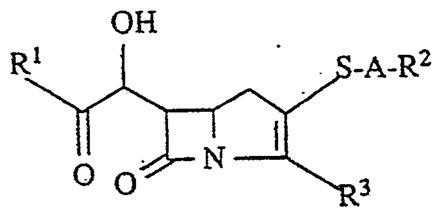

(I)

in which
$R^1$, $R^3$ and $AR^2$ have the meaning indicated in Claim 1,
and the salts thereof,
for use in a process for the therapeutic treatment of the human or animal body.

4. Medicaments containing substituted 6-hydroxy-methylcarbapenem antibiotics fo the general formula (I)

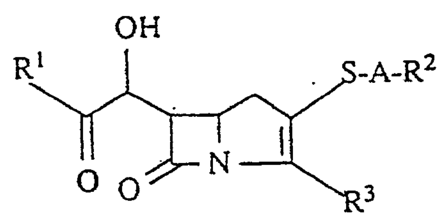

(I)

according to Claim 1.

5. Use of substituted 6-hydroxymethyl-carbapenem antibiotics of the general formula (I) according to Claim 1 for the preparation of medicaments.

6. Animal feedstuffs and animal feedstuff additives containing substituted 6-hydroxymethyl-carbapenem antibiotics of the formula (I) according to Claim 1.

## Revendications

1. Composés de formule générale (I):

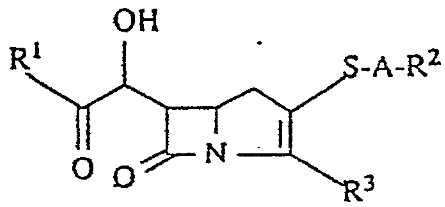

(I)

dans laquelle
$R^1$ représente un groupe de formule $-OCH_3$, $-OC_2H_5$, $-OC_3H_7$, $-OCH(CH_3)_2$, $-OC_4H_9$, $-OC(CH_3)_3$, $-O-CH_2-CH=CH_2$, $-O-CH_2-C_6H_5$,

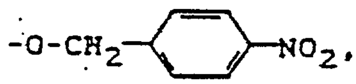

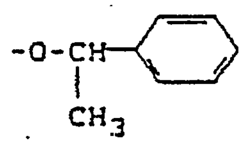

(forme R, forme S ou mélange RS)

$-NH_2$, $-NHCH_3$, $-N(CH_3)_2$, $-NHC_2H_5$, $-N(C_2H_5)_2$,

$-NHC_3H_7$, $-N(C_3H_7)_2$, $-NHCH(CH_3)_2$, 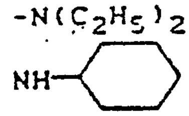,

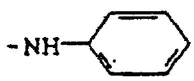, $NH-CH_2-CH=CH_2$, 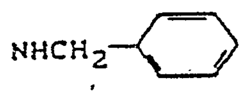,

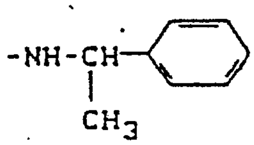

(forme R, forme S ou mélange RS)

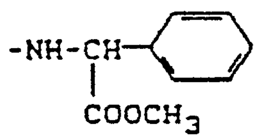

(forme R, forme S ou mélange RS)

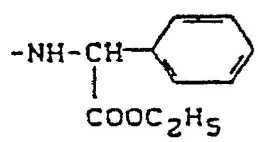

(forme R, forme S ou mélange RS)

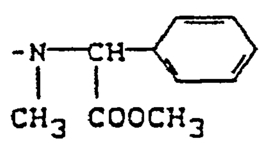

(forme R, forme S ou mélange RS)

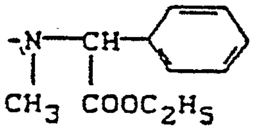 (forme R, forme S ou mélange RS)

$-NH-CO-NH_2$, $-NH-CO-NHCH_3$, $-NH-CO-N(CH_3)_2$,

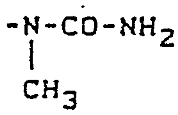 , 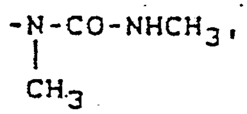 , 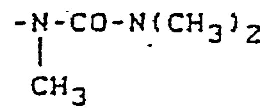 ,

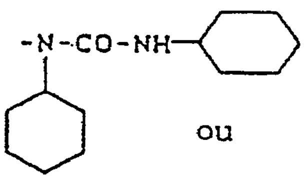 ou 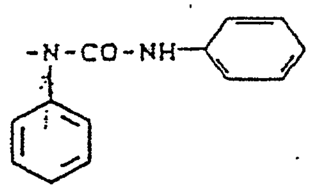

le group AR[2] représente un radical de formule:

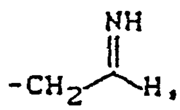 , 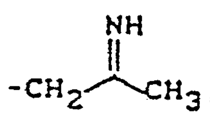 , 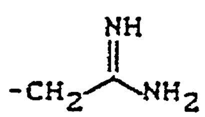 , 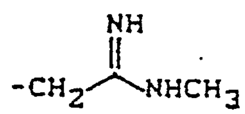 ,

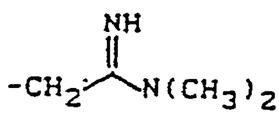 , 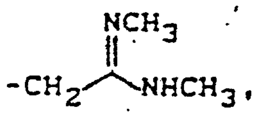 , 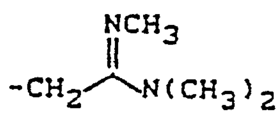 ,

$-CH_2-CH_2-NH_2$, $-CH_2-CH_2-NHCH_3$, $-CH_2-CH_2-N(CH_3)_2$,

$-CH_2-CH_2-NHCOCH_3$, 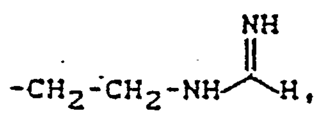 ,

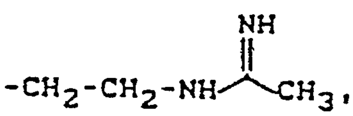 , 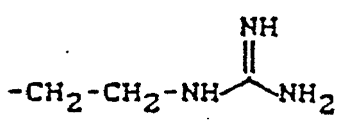 , $-CH_2-COOH$,

$-CH_2-COOCH_3$, 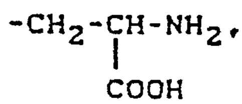 , 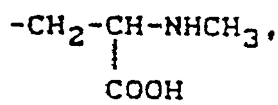 ,

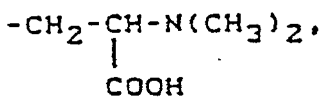, 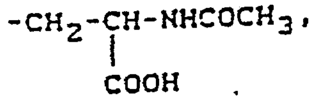, 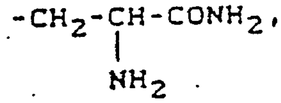,

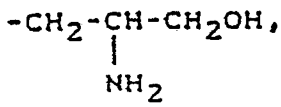, 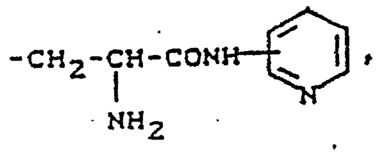, $-CH=CH-NH_2$,

$-CH-CH-NHCH_3$, $-CH=CH-N(CH_3)_2$, $-CH=CH-NHCOCH_3$,

$-CH=CH-CH_2-NH_2$, 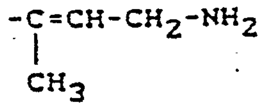

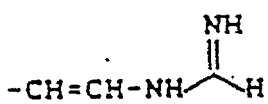, 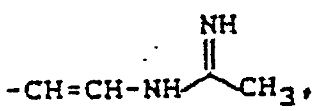, 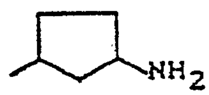,

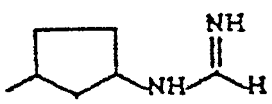, 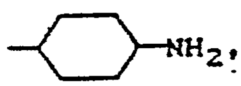, 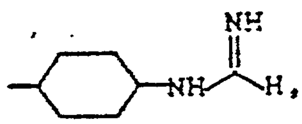,

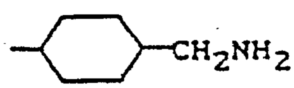, 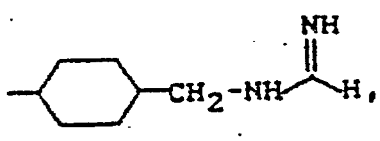, 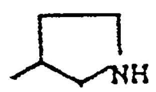,

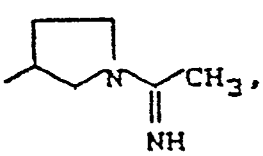, 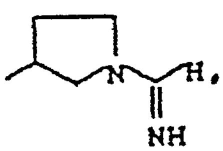, 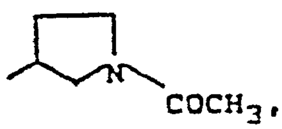, 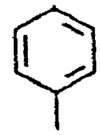,

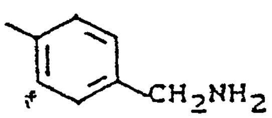, 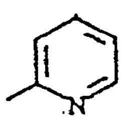, 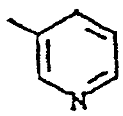, 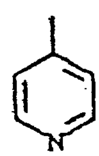, 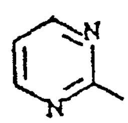

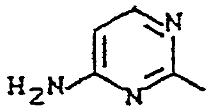 , 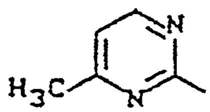 , 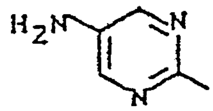 , 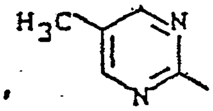 ,

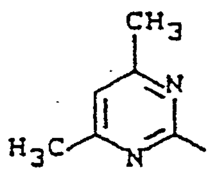 , 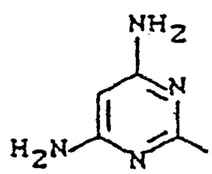 , 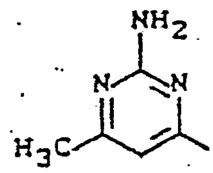 ,

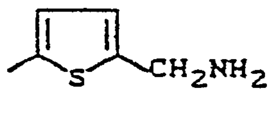 , 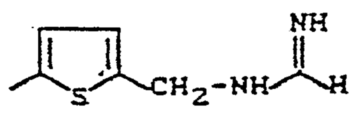 , 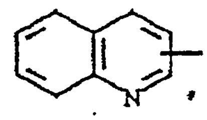 ,

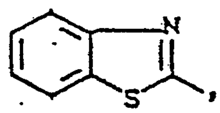 , 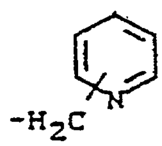 , 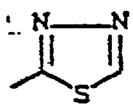 , 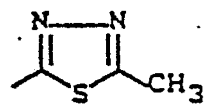 ,

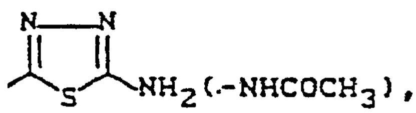 , 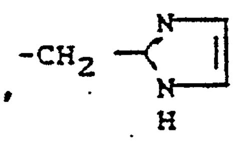 , 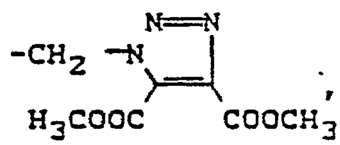 ,

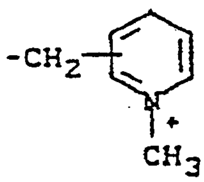 , 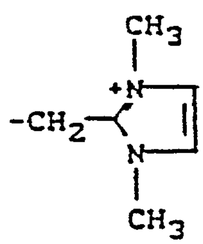 , 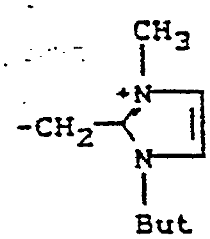 ,

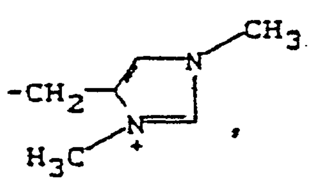 , 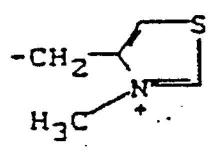 , 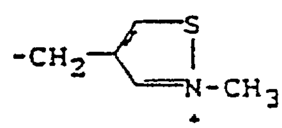

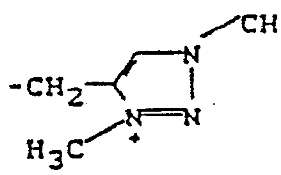 , 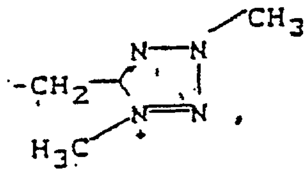 ,

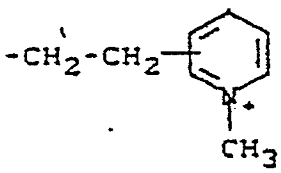 , 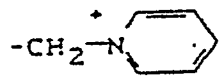 , 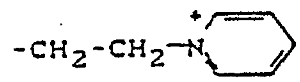 ,

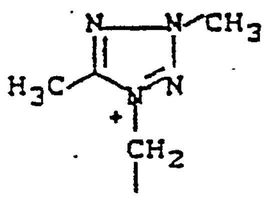 , 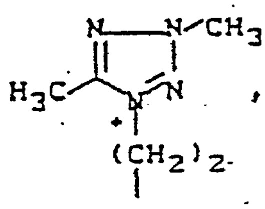 , 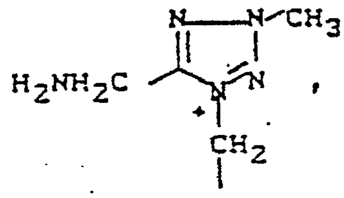 ,

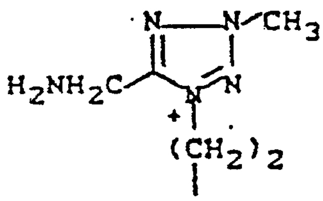 , 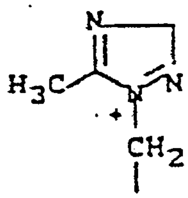 , 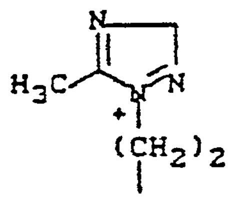

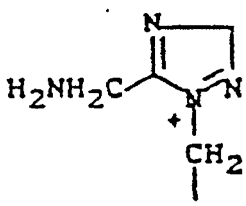 , 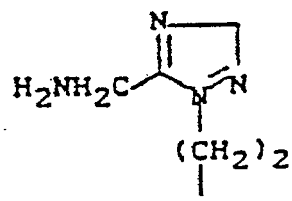 , 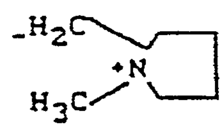 ,

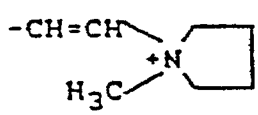 , 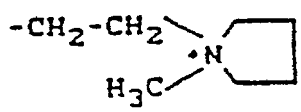 , 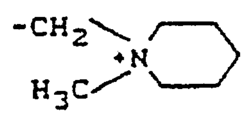 ,

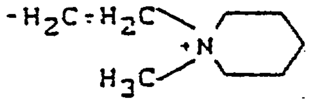 , 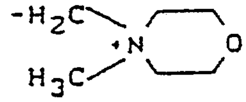 , 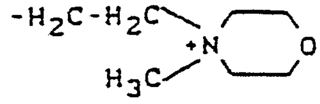 ,

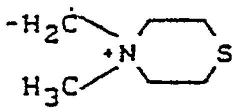 , 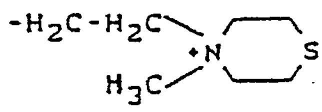 , 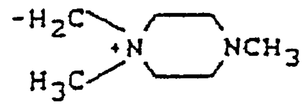 ,

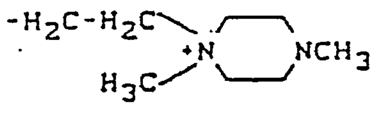 , 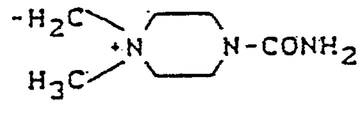 ,

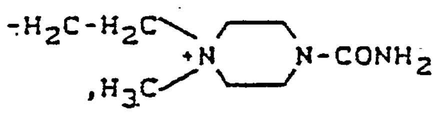 , 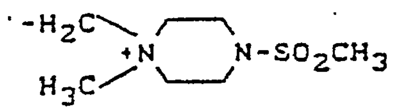 ,

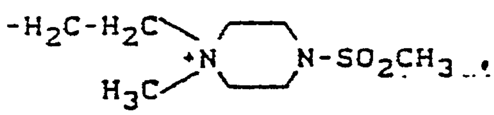 , 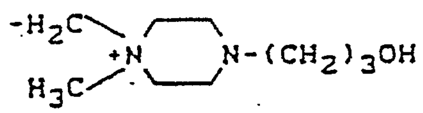 ,

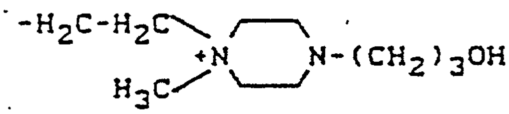 .

et
$R^3$ représente un groupe carboxyle $COOR^{20}$ où
$R^{20}$ représente un atome d'hydrogène, un groupe allyle, un groupe 4-nitrobenzyle ou un groupe 4-méthoxybenzyle, ou encore $COO^-$ si le groupe $AR^2$ représente un radical chargé positivement, ainsi que leurs sels.

2. Procédé de préparation d'antibiotiques de 6-hydroxyméthyl-carbapénem substitués de formule générale (I):

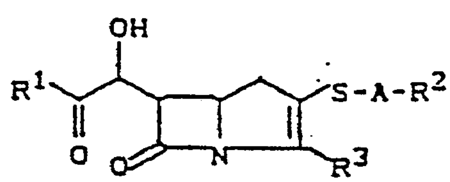 (I)

dans laquelle $R^1$, $R^3$ et $AR^2$ ont les significations indiquées dans la revendication 1, caractérisé en ce qu'on fait réagir des céto-esters de formule générale (II):

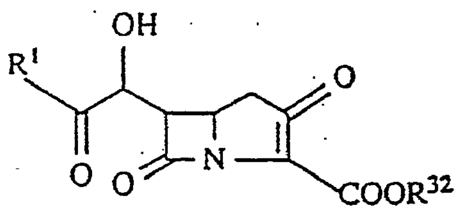

(II)

dans laquelle
$R^1$ a la signification indiquée ci-dessus, et
$R^{32}$ représente un atome d'hydrogène, un groupe protecteur du groupe carboxyle ou un radical ester dissociable in vivo, mais non un atome d'hydrogène, tandis que
le groupe $COOR^{32}$ ne peut représenter un anion carboxylate, ainsi que
des thiols de formule générale (III):

H–S–A–$R^2$

dans laquelle
A et $R^2$ ont les significations indiquées, dans des solvants inertes, en présence de bases, avec une substance auxiliaire, on dissocie éventuellement les groupes protecteurs et on prépare éventuellment les sels désirés ou on transfomre les sels en composés libres.

3. Antiobiotiques de 6-hydroxyméthylcarbapénem substitués de formule générale (I):

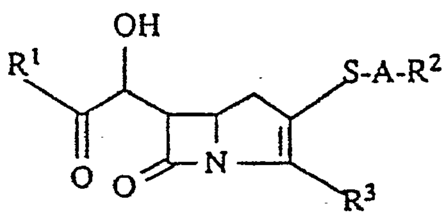

(I)

dans laquelle
$R^1$, $R^3$ et $AR^2$ ont les significations indiquées dans la revendication 1
ainsi que leurs sels en vue de les utiliser dans un procédé pour le traitement thérapeutique du corps humain ou animal.

4. Médicaments contenant des antibiotiques de 6-hydroxyméthyl-carbapénem substitués de formule générale (I):

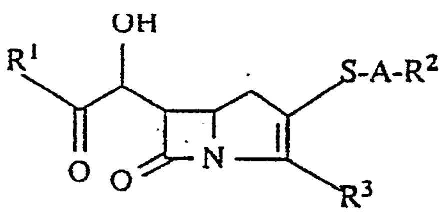

(I)

selon la revendication 1.

5. Utilisation d'antibiotiques de 6-hydroxyméthyl-carbapénem substitués de formule générale (I) selon la revendication 1 pour la préparation de médicaments.

6. Aliments pour animaux et additifs d'aliments pour animaux contenant des antibiotiques de 6-hydroxyméthyl-carbapénem substitués de formule (I) selon la revendication 1.